# FASCICULE DE BREVET EUROPEEN

(11) **EP 2 024 350 B1**
(45) Date de publication et mention de la délivrance du brevet: **18.09.2013**
(21) Numéro de dépôt: 07729287.8
(22) Date de dépôt: 18.05.2007
(51) Int. Cl.: C07D 295/135

(54) **DERIVES DE PIPERIDINE COMME INHIBITEURS DU VIRUS DU PAPILLOME HUMAIN**
PIPERIDINDERIVATE ALS INHIBITOREN DES HUMANEN PAPILLOMAVIRUS
PIPERIDINE DERIVATIVES AS HUMAN PAPILLOMA VIRUS INHIBITORS

(30) Priorité: 19.05.2006 FR 0604496
(43) Date de publication de la demande: 18.02.2009
(73) Titulaire: Anaconda Pharma, 94800 Villejuif (FR)
(72) Inventeur: BLUMENFELD, Marta, F-75013 Paris (FR); COMPERE, Delphine, F-92330 Sceaux (FR); GAUTHIER, Jean-Michel, F-78700 Conflans-Sainte-Honorine (FR)
(74) Mandataire: Regimbeau
(86) Numéro de dépôt international: PCT/EP2007/054843
(87) Numéro de publication internationale: WO 2007/135106

(56) Documents cités:
- WO-A-00/56331
- WO-A-2004/108673

## Description

La présente invention concerne des nouveaux composés antiviraux dirigés contre le virus du papillome, les compositions pharmaceutiques les contenant, leur procédé de préparation et intermédiaires de synthèse, ainsi que leur utilisation pour le traitement ou la prévention d'une infection par le virus du papillome.

Les virus du papillome sont des virus non enveloppés dont le génome est formé par un ADN double brin d'environ 8 kb. Ils sont très répandus dans la nature et provoquent des lésions épithéliales chez l'Homme ainsi que chez de nombreux animaux dont le lapin, le cheval, le chien et l'espèce bovine. Près d'une centaine de virus du papillome humains (VPH) ont été décrits. Ils sont classés en fonction de leurs sites d'infection. Environ 30 VPH ont été isolés à partir de muqueuses anogénitales (col de l'utérus, vagin, vulve, pénis, anus, rectum). Les autres VPH sont associés à des lésions cutanées. Les VPH à tropisme cutané incluent, entre autres, VPH1, VPH2, VPH3, VPH4, VPH5, VPH7, VPH8, VPH9, VPH10, VPH12, VPH14, VPH15, VPH17, VPH19, VPH20, VPH21, VPH22, VPH23, VPH24, VPH25, VPH26, VPH27, VPH28, VPH29, VPH38, VPH41, VPH47, VPH49. Ils sont associés à des lésions comme des verrues (de type vulgaire, plantaire, myrmécie, superficielle, plate...) et des maladies comme l'épidermo-dysplasie verruciforme.

Les VPH de type mucogénital sont impliqués dans des maladies laryngées et anogénitales dont certains cancers. Ils sont souvent classés en VPH à haut risque et VPH à bas risque, en se référant au type de lésions auxquels ils sont associés. Les VPH à bas risque incluent, entre autres, VPH6, VPH11, VPH13, VPH32, VPH34, VPH40, VPH42, VPH43, VPH44, VPH53, VPH54, VPH55, VPH57, VPH58, VPH74, VPH91. Les VPH à bas risque sont associés à des lésions bénignes comme les condylomes (verrues génitales telles les condylomes acuminés et condylomes plans), les papillomes laryngés, conjonctivaux ou buccaux ou d'autres lésions épithéliales comme des néoplasies intra-épithéliales de bas grade ou des papillomatoses récurrentes respiratoires, et plus rarement à des papuloses bowénoïdes ou des néoplasies intra-épithéliales de grade élevé ou des carcinomes. Les VPH à haut risque incluent, entre autres, VPH16, VPH18, VPH31, VPH33, VPH35, VPH39, VPH45, VPH51, VPH52, VPH56, VPH59, VPH61, VPH62, VPH66, VPH67, VPH68, VPH72. Ils sont impliqués dans des lésions intra-épithéliales de bas grade qui peuvent évoluer vers des lésions de plus haut grade allant jusqu'à des cancers, en particulier le cancer du col de l'utérus et autres cancers ano-génitaux.

Les infections génitales par des VPH sont les infections sexuellement transmises les plus fréquentes dans le monde, y compris dans les pays développés, avec plus de 20 millions de personnes infectées aux Etats-Unis. La prévalence des infections aux VPH varie de 3 à 42 % selon les pays et affecte 10 à 20 % de la population sexuellement active dans les pays industrialisés. Dans une partie de cette population, l'infection persiste et peut conduire à des cancers dans le cas des VPH à haut risque.

On estime à 1 à 2 % la prévalence des verrues génitales (condylomes) dans la population sexuellement active des pays industrialisés, soit environ 3 500 000 nouveaux cas par an dans ces pays et 28 000 000 dans le monde. Les verrues génitales peuvent être trouvées sur des parties du corps comprenant ou périphériques de l'anus, la vulve, le vagin, le col de l'utérus et le pénis.

Les traitements des verrues génitales reposent sur plusieurs stratégies, depuis la destruction physique (cryothérapie, laser CO₂, électro-chirurgie, excision chirurgicale), l'application d'agents cytotoxiques (TCA, podophylline, podofilox) jusqu'à l'application d'agents immuno-modulateurs (interféron, imiquimod). Cependant, aucune de ces méthodes n'élimine complètement toutes les particules virales et des taux de récurrence importants, accompagnés d'effets secondaires sérieux sont observés avec les stratégies thérapeutiques actuelles. Ceci renforce un besoin en nouvelles stratégies pour contrôler ou éliminer les infections par les virus du papillome.

Contrairement à ce qui existe dans le traitement d'autres maladies virales, comme celles causées par VIH, les virus de l'Herpès ou influenza, il n'y a pas, à ce jour, de traitement antiviral ciblant spécifiquement les pathogènes viraux que sont les virus du papillome.

Les virus du papillome infectent les épithéliums pluristratifiés et leur cycle viral est étroitement lié à l'organogenèse de ces organes et à la différentiation des kératinocytes. Après infection, le génome viral est présent et répliqué en faible nombre dans les cellules basales de l'épithélium. A mesure que les cellules se différencient, l'expression des gènes viraux et le nombre de copies du génome viral augmentent, jusqu'à l'expression des gènes de la capside virale et la formation de virions infectieux dans les kératinocytes totalement différenciés.

Le génome des VPH code potentiellement pour une dizaine de protéines. Les protéines les plus précocement exprimées, E1 et E2, sont impliquées dans la réplication du génome viral et la régulation de l'expression des gènes viraux. Les autres protéines précoces de ces virus (E4, E5, E6, E7) ont des fonctions en relation avec la prolifération cellulaire ou des rôles non encore complètement élucidés. L'existence des protéines E3 et E8 est encore incertaine. Les protéines tardives L1 et L2 sont celles qui forment la capside virale.

Les 2 seules protéines virales nécessaires et suffisantes pour la réplication des VPH sont E1 et E2. Elles sont capables de former un complexe E1/E2 et de se fixer sur l'origine de réplication (Ori) des VPH, une séquence contenue dans le génome viral et portant des sites proches reconnus par E1 et par E2. E2 est capable de se fixer avec une très grande affinité sur les sites E2 alors que E1, seule, ne possède pas une très grande affinité pour les sites E1. L'interaction entre E1 et E2 augmente la fixation de E1 sur l'Ori par coopérativité de fixation à l'ADN. Une fois fixée à l'ADN, E1 n'interagit plus avec E2 puis forme un hexamère. Les activités hélicase et ATPase de E1 permettent le déroulement de l'ADN viral qui est ensuite répliqué par la machinerie cellulaire de réplication.

Les inventeurs ont cherché à mettre au point des petites molécules qui inhibent la réplication des VPH, de préférence à bas risque, en interférant notamment avec la formation du complexe entre les protéines E1 et E2.

Autres composés avec la même activité ont été décrits dans WO 00/56331 et WO 2004/108673.

Une solution a été trouvée par l'élaboration de nouveaux dérivés.

La présente invention a pour objet ces nouveaux dérivés, leur synthèse, ainsi que leur utilisation dans des compositions pharmaceutiques aptes à être utilisées dans la prévention et le traitement des pathologies liées à une inhibition de la réplication des VPH, comme, à titre d'exemples, VPH1, VPH2, VPH3, VPH4, VPH5, VPH7, VPH8, VPH9, VPH10, VPH12, VPH14, VPH15, VPH17, VPH19, VPH20, VPH21, VPH22, VPH23, VPH24, VPH25, VPH26, VPH27, VPH28, VPH29, VPH38, VPH41, VPH47, VPH49, VPH6, VPH11, VPH13, VPH32, VPH34, VPH40, VPH42, VPH43, VPH44, VPH53, VPH54, VPH55, VPH57, VPH58, VPH74, VPH91, VPH16, VPH18, VPH31, VPH33, VPH35, VPH39, VPH45, VPH51, VPH52, VPH56, VPH59, VPH61, VPH62, VPH66, VPH67, VPH68, VPH72 de préférence les VPH à bas risque tels que VPH6, VPH11, VPH13, VPH32, VPH34, VPH40, VPH42, VPH43, VPH44, VPH53, VPH54, VPH55, VPH57, VPH58, VPH74, VPH91.

Les nouveaux dérivés objets de la présente invention sont actifs contre le virus du papillome. Ils sont aussi capables d'inhiber l'interaction E1/E2.

Dans le cadre de la présente invention, on donne les définitions suivantes :
"Alkyle" ou "Alk" signifie une chaîne hydrocarbonée saturée, linéaire ou ramifiée, monovalente ou divalente, comprenant de 1 à 6 atomes de carbone tel que le groupement méthyle, éthyle, propyle, isopropyle, tert-butyle, méthylène, éthylène, propylène, ...
"Acyle" signifie un groupement -COR où R est un groupement alkyle tel que défini précédemment ou phényle, par exemple acétyle, éthylcarbonyle, benzoyle, ...
"Acylamino" signifie un groupement -NHC(O)R où R est un groupement alkyle tel que défini précédemment.

"Acylaminoalkyle" signifie un groupement -AlkNHC(O)R où Alk et R sont des groupements alkyle tel que défini précédemment.

"Alkoxy" signifie un groupement -OAlk où Alk est un groupement alkyle tel que défini précédemment. Alkoxy comprend par exemple méthoxy, éthoxy, *n*-propyloxy, *tert*-butyloxy, ...

"Aryle" signifie un système monocyclique ou bicyclique aromatique comprenant de 4 à 10 atomes de carbone, étant compris que dans le cas d'un système bicyclique, l'un des cycles présente un caractère aromatique et l'autre cycle est aromatique ou insaturé. Aryle comprend par exemple les groupements phényle, naphthyle, indényle, benzocyclobutényle, ...

"Hétérocycle" signifie un système monocyclique ou bicyclique fusionné, spiro-fusionné ou ponté, de 3 à 12 chaînons, saturé, insaturé ou aromatique, comprenant de 1 à 4 hétéroatomes, identiques ou différents, choisis parmi oxygène, soufre et azote, et contenant éventuellement 1 ou 2 groupements oxo ou thioxo, étant compris que dans le cas d'un système bicyclique, l'un des cycles peut présenter un caractère aromatique et l'autre cycle est aromatique ou insaturé. Hétérocycle comprend par exemple les groupements pipéridyle, pipérazyle, furyle, thiényle, pyrrolyle, pyrazolyle, imidazolyle, pyridyle, pyrimydile, pyrazynile, pyradizinyle, benzofuryle, benzothiényle, indolyle, quinolyle, isoquinolyle, benzodioxolyle, benzodioxinyle, benzo[1,2,5]thiadiazolyle, benzo[1,2,5]oxadiazolyle, [1,2,3]triazolyle, [1,2,4]triazolyle, ...

"Alkylthio" signifie un groupement -SAlk où Alk est un groupement alkyle tel que défini précédemment. Alkylthio comprend par exemple méthylthio, éthylthio, isopropylthio, heptylthio, ...

"Arylalkyle" signifie un groupement -Alk-Ar où Alk représente un groupement alkyle tel que défini précédemment et Ar représente un groupement aryle tel que défini précédemment.

"Atome d'halogène" signifie un atome de fluor, de brome, de chlore ou d'iode.

"Cycloalkyle" signifie un système monocyclique ou bicyclique fusionné ou ponté, saturé, comprenant de 3 à 12 atomes de carbone tel que le groupement cyclopropyle, cyclobutyle, cyclopentyle, cyclohexyle, cycloheptyle, cyclooctyle, adamantyle, décalinyle, norbornyle, ...

"Cycloalkényle" signifie un système monocyclique ou bicyclique fusionné ou ponté, insaturé, comprenant de 3 à 12 atomes de carbone tel que le groupement cyclopropényle, cyclobutényle, cyclopentényle, cyclohexényle, ...

"Monoalkylamino" signifie un groupement -NHAlk où Alk est un groupement alkyle tel que défini précédemment.

"Dialkylamino" signifie un groupement -NAlkAlk' où Alk et Alk' représentent chacun indépendamment l'un de l'autre un groupement alkyle tel que défini précédemment.

"Monoalkylamide" signifie un groupement -C(O)NHAlk où Alk est un groupement alkyle tel que défini précédemment.

"Dialkylamide" signifie un groupement -C(O)NAlkAlk' où Alk et Alk' représentent chacun l'un indépendamment de l'autre un groupement alkyle tel que défini précédemment.

"N-cycloalkyle" signifie un radical cycloalkyle tel que défini précédemment, comprenant un atome d'azote, relié au restant de la molécule par cet atome. N-cycloalkyle comprend par exemple le groupement pipérid-1-yle ou pyrrolid-1-yle.

"N-cycloalkényle" signifie un radical cycloalkényle tel que défini précédemment, comprenant un atome d'azote, relié au restant de la molécule par cet atome. N-cycloalkényle comprend par exemple le groupement tétrahydropyridin-1-yle.

"Halogénoalkyle" signifie une chaîne hydrocarbonée saturée, linéaire ou ramifiée, comprenant de 1 à 6 atomes de carbone et substituée par 1 à 6 atomes d'halogène tel que le groupement trifluorométhyle, 2,2,2-trifluoroéthyle, ...

"Halogénoalkoxy" signifie une chaîne hydrocarbonée saturée, linéaire ou ramifiée, comprenant de 1 à 6 atomes de carbone et substituée par 1 à 6 atomes d'halogène, ladite chaîne étant reliée au composé par un atome d'oxygène tel que le groupement trifluorométhoxy, 2,2,2-trifluoroéthoxy, ...

"Halogénoalkylthio" signifie une chaîne hydrocarbonée saturée, linéaire ou ramifiée, comprenant de 1 à 6 atomes de carbone et substituée par 1 à 6 atomes d'halogène, ladite chaîne étant rattachée par un atome de soufre tel que le groupement trifluorométhylthio, ...

« Groupe protecteur » ou « groupe de protection » signifie le groupe qui bloque sélectivement le site réactif dans un composé multifonctionnel de telle sorte qu'une réaction chimique peut être effectuée sélectivement au niveau d'un autre site réactif non protégé dans la signification classiquement associée à celui-ci en chimie de synthèse.

« Isomérisme » signifie des composés qui ont des formules moléculaires identiques mais qui diffèrent par nature ou dans la séquence de liaison de leurs atomes ou dans l'agencement de leurs atomes dans l'espace. Les isomères qui diffèrent dans l'agencement de leurs atomes dans l'espace sont désignés par « stéréoisomères ». Les stéréoisomères qui ne sont pas des images dans un miroir l'un de l'autre sont désignés par « diastéréoisomères », et les stéréoisomères qui sont des images dans un miroir non superposables sont désignés par « énantiomères » ou isomères optiques. Les « stéréoisomères » se réfèrent aux racémates, énantiomères et diastéréoisomères.

« Pharmaceutiquement acceptable » signifie celui qui est de manière générale sûr, non toxique, et qui n'est pas indésirable biologiquement, aussi bien pour une utilisation vétérinaire que pour une utilisation pharmaceutique humaine.

« Sels pharmaceutiquement acceptables » d'un composé signifie des sels qui sont pharmaceutiquement acceptables, tels que définis ici, et qui possèdent l'activité pharmacologique souhaitée du composé parent. Il devrait être compris que toutes les références aux sels pharmaceutiquement acceptables comprennent les formes d'addition de solvants (solvates) ou les formes cristallines (polymorphes) tels que définis ici, du même sel d'addition d'acide ou de base. Une revue des sels pharmaceutiquement acceptables est notamment décrite dans J. Pharm. Sci., 1977, 66, 1-19.

Les « acides pharmaceutiquement acceptables » signifient les sels d'acides non toxiques issus d'acides organiques ou minéraux. Parmi les acides pharmaceutiquement acceptables, on peut citer à titre non limitatif les acides chlorhydrique, bromhydrique, sulfurique, phosphonique, nitrique, acétique, trifluoroacétique, lactique, pyruvique, malonique, succinique, glutarique, fumarique, tartrique, maléïque, citrique, ascorbique, oxalique, méthane sulfonique, camphorique, benzoïque, toluènesulfonique, ...

Les « bases pharmaceutiquement acceptables » signifient les sels basiques non toxiques issues de bases organiques ou minérales, formés quand un proton acide présent dans le composé parent est remplacé par un ion métallique ou est coordiné à une base organique. Parmi les bases pharmaceutiquement acceptables, on peut citer à titre non limitatif l'hydroxyde de sodium, l'hydroxyde de potassium, l'hydroxyde de lithium, l'hydroxyde de calcium, la triétylamine, la tertbutylamine, la 2-diéthylaminoéthanol, l'éthanolamine, l'éthylènediamine, la dibenzyléthylènediamine, la pipéridine, la pyrrolidine, la morpholine, la pipérazine, la benzylamine, l'arginine, la lysine, l'histidine, la glucosamine, les hydroxydes d'ammonium quaternaire, ...

On entend par « pro-drogue » un dérivé chimique du composé objet de la présente invention qui génère in vivo ledit composé par réaction chimique spontanée avec le milieu physiologique, notamment par réaction enzymatique, photolyse et/ou réaction métabolique.

On entend par « radical pro-drogue de la fonction acide» un groupe fonctionnel labile qui va générer in vivo une fonction acide en se séparant du composé objet de la présente invention par réaction chimique spontanée avec le milieu physiologique, notamment par réaction enzymatique, photolyse et/ou réaction métabolique. Les radicaux pro-drogue de fonction acide comprennent notamment les groupements ester, pivoyloxyméthyle, acétoxyméthyle, phthalidyle, indanyle, méthoxyméthyle ou 5-R-2-oxo-1,3-dioxolen-4-yl)méthyle. D'autres exemples sont décrits dans T. Higuchi and V. Stella, "Pro-drugs as Novel Delivery Systems", Vol. 14, A.C.S. Symposium Series, American Chemical Society (1975) et "Bioreversible Carriers in Drug Design: Theory and Application", édité par E. B. Roche, Pergamon Press: New York, 14-21 (1987).

Dans la présente demande de brevet, les composés chimiques sont nommés selon la nomenclature IUPAC (The International Union of Pure and Applied Chemistry).

La présente invention a pour objet les composés de formule (I) : 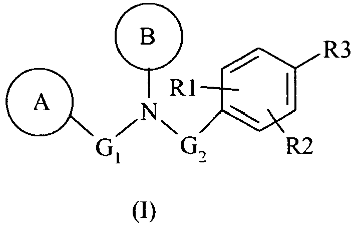 ainsi que leurs stéréoisomères,
dans laquelle :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
**G₂** représente un groupement 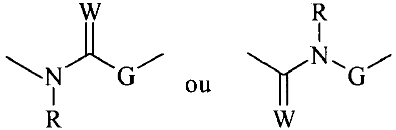 dans lequel :
   - **R** représente un atome d'hydrogène, un groupement alkyle, halogénoalkyle, ou un radical pro-drogue tel que carbamate, acétyle, dialkylaminométhyle ou -CH₂-O-CO-Alk,
   - **G** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
   - **W** représente un atome d'oxygène, de soufre ou NH,
**R₁** et **R_{2.}** identiques ou différents, représentent chacun un groupement choisi parmi un atome d'hydrogène, un atome d'halogène, un groupement hydroxyle, thio, alkoxy, halogénoalkoxy, alkylthio, halogénoalkylthio, amino, monoalkylamino, dialkylamino, cycloalkyle, alkyle ou halogénoalkyle,
**R₃** représente un groupement acide ou un radical pro-drogue de la fonction acide tel qu'ester, ou bien un bioisostère de la fonction acide tel que tétrazole, phosphonate, phosphonamide, sulfonate ou sulfonamide,
**A** représente un groupement aryle, cycloalkyle, cycloalkényle ou un hétérocycle, chacun éventuellement substitué, et
**B** représente un groupement aryle ou un hétérocycle à 6 chaînons, chacun éventuellement substitué,
ainsi que leurs sels pharmaceutiquement acceptables.

**A** tel que défini précédemment peut être substitué par un ou deux groupements, identiques ou différents, choisis parmi :
- un atome d'hydrogène, un atome d'halogène,
- un groupement alkoxy, alkylthio, halogénoalkoxy, halogénoalkylthio, hydroxyle, thio, cyano, amino, monoalkylamino ou dialkylamino,
- un groupement -SOnR', -COR', -CO₂R', -OCOR', -CONR'R", -NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
- un groupement alkyle ou halogénoalkyle, le groupement alkyle étant éventuellement substitué par un groupement cyano, amino, monoalkylamino, dialkylamino ou acylamino,
- un groupement aryle, arylalkyle, -X-aryle, -X-arylalkyle ou -Alk-X-aryle, où X représente -O-, -NH-, -N(Alk)-, -N(COCH₃)-, -S-, -SO-, -SO₂-, -CO- ou - CONH-, chacun substitué sur la partie aryle par un ou deux substituants, identiques ou différents, choisis parmi :
   ✔ un atome d'hydrogène ou un atome d'halogène,
   ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide,
   ✔ un groupement -SOnR', -COR', -CO₂R', -OCOR', -CONR'R", - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
- un groupement hétérocycle, -Alk-hétérocycle, -X-hétérocycle, -X-Alk-hétérocycle ou -Alk-X-hétérocycle, où X représente -O-, -NH-, -N(Alk)-, -N(COCH₃)-, -S-, -SO-, -SO₂-, -CO- ou -CONH-, chacun éventuellement substitué sur la partie hétérocycle par un ou deux substituants, identiques ou différents, choisis parmi :
   ✔ un atome d'hydrogène ou un atome d'halogène,
   ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, ou
   ✔ un groupement -SOnR', -COR', -CO₂R', -OCOR', -CONR'R", - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
- un groupement cycloalkyle, -Alk-cycloalkyle, cycloalkényle, -Alk-cycloalkényle, -X-cycloalkyle, -X-Alk-cycloalkyle, -X-cycloalkényle, -X-Alk-cycloalkényle, -Alk-X-cycloalkyle, -Alk-X-cycloalkényle, où X représente -O-, - NH-, -N(Alk)-, -N(COCH₃)-, -S-, -SO-, -SO₂-, -CO- ou -CONH-, chacun éventuellement substitué sur la partie cyclique par un ou deux substituants, identiques ou différents, choisis parmi :
   ✔ un atome d'hydrogène ou un atome d'halogène,
   ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide ou oxo, ou
   ✔ un groupement -SOnR', -COR', -CO₂R', -OCOR', -CONR'R", - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,

**B** tel que défini précédemment peut être un aryle ou hétérocycle à 6 chaînons, substitué en position ortho par un groupement **R₄** et éventuellement substitué par un groupement **R₅,** dans lequel :
- **R₄** représente :
   ✔ un groupement alkyle, -NHAlk, -NAlkAlk', -NHcycloalkyle ou -NAlkcycloalkyle, Alk et Alk' étant identiques ou différents,
   ✔ un groupement cycloalkyle, cycloalkényle, N-cycloalkyle ou N-cycloalkényle, chacun éventuellement substitué par un ou deux substituants, identiques ou différents, choisis parmi un atome d'hydrogène, un atome d'halogène, un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, oxo ou -X-Aryle et où X représente -O-, -NH-, -N(Alk)-, -N(COCH₃)-, -S-, - SO-, -SO₂-, -CO- ou -CONH-, ou
   ✔ un groupement aryle éventuellement substitué par un ou deux groupements substituants, identiques ou différents, un atome d'hydrogène, un atome d'halogène, un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide ou -X-Aryle et où X représente -O-, -NH-, -N(Alk)-, -N(COCH₃)-, -S-, -SO-, - SO₂-, -CO- ou -CONH-,
- **R₅** représente :
   ✔ un atome d'hydrogène ou un atome d'halogène,
   ✔ un groupement hydroxyle, alkoxy, halogénoalkoxy, thio, alkylthio, halogénoalkylthio, amino, monoalkylamino, dialkylamino, -NHAcyle, cyano, acyle, acide, ester, amide, monoalkylamide ou dialkylamide,
   ✔ un groupement alkyle ou halogénoalkyle, le groupement alkyle pouvant être substitué un groupement cyano, hydroxyle, alkoxy, acide ou ester,
   ✔ un groupement -SOₙAlk, -SOₙNH₂, -SOₙNHAlk ou -SOₙNAlkAlk', où n vaut 1 ou 2 et Alk et Alk' sont identiques ou différents, ou
   ✔ un groupement pipéridine, oxopipéridine, morpholine ou bien un groupement pipérazine éventuellement substitué par un groupement alkyle ou acyle,

Les composés préférés sont les composés de formule (I) dans laquelle :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
**G₂** représente un groupement 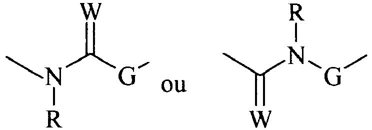 dans lequel :
   - **R** représente un atome d'hydrogène, un groupement alkyle, halogénoalkyle, ou un radical pro-drogue tel que carbamate, acétyle, dialkylaminométhyle, ou -CH₂-O-CO-Alk,
   - G représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques, et
   - W représente un atome d'oxygène, de soufre ou NH,
**R₁** et **R_{2.}** identiques ou différents, représentent chacun un groupement choisi parmi un atome d'hydrogène, un atome d'halogène, un groupement hydroxyle, thio, alkoxy, halogénoalkoxy, alkylthio, halogénoalkylthio, amino, monoalkylamino, dialkylamino, cycloalkyle, alkyle ou halogénoalkyle,
**R₃** représente un groupement acide ou un radical pro-drogue de la fonction acide tel qu'ester, ou bien un bioisostère de la fonction acide tel que tétrazole, phosphonate, phosphonamide, sulfonate ou sulfonamide,
**A** représente un groupement aryle ou un hétérocycle, chacun éventuellement substitué par un ou deux groupements, identiques ou différents, choisis parmi :
   - un atome d'hydrogène, un atome d'halogène,
   - un groupement alkoxy, alkylthio, halogénoalkoxy, halogénoalkylthio, hydroxyle, thio, cyano, amino, monoalkylamino ou dialkylamino,
   - un groupement -SOnR', -COR', -CO₂R', -OCOR', -CONR'R", -NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
   - un groupement alkyle ou halogénoalkyle, le groupement alkyle étant éventuellement substitué par un groupement cyano, amino, monoalkylamino, dialkylamino ou acylamino,
   - un groupement aryle, arylalkyle, -X-aryle, où X représente -O-, -NH-, -N(Alk)-, -N(COCH₃)-, -S-, -SO-, -SO₂-, -CO- ou -CONH-, chacun substitué sur la partie aryle par un ou deux substituants, identiques ou différents, choisis parmi :
      ✔ un atome d'hydrogène ou un atome d'halogène,
      ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, ou
      ✔ un groupement -SOnR', -COR', -CO₂R', -OCOR', -CONR'R", - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
   - un groupement hétérocycle, -X-hétérocycle, où X représente -O-, -NH-, -N(Alk)-, -N(COCH₃)-, -S-, -SO-, -SO₂-, -CO- ou -CONH-, chacun éventuellement substitué sur la partie hétérocycle par un ou deux substituants, identiques ou différents, choisis parmi :
      ✔ un atome d'hydrogène ou un atome d'halogène,
      ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, ou
      ✔ un groupement -SOnR', -COR', -CO₂R', -OCOR', -CONR'R", - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2, ou
   - un groupement cycloalkyle, cycloalkényle, -X-cycloalkyle, -X-cycloalkényle, où X représente -O-, -NH-, -N(Alk)-, -N(COCH₃)-, -S-, -SO-, -SO₂-, -CO- ou - CONH-, chacun éventuellement substitué sur la partie cyclique par un ou deux substituants, identiques ou différents, choisis parmi :
      ✔ un atome d'hydrogène ou un atome d'halogène,
      ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide ou oxo, ou
      ✔ un groupement -SOnR', -COR', -CO₂R', -OCOR', -CONR'R", - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2, et
**B** représente un groupement phényle ou pyridine :
   - substitué en position ortho par un groupement N-cycloalkyle tel que pipéridine ou par un cyclohexyle, chacun éventuellement substitué par un ou deux substituant, identiques ou différents, choisis parmi un atome d'hydrogène, un groupement alkyle, halogénoalkyle, alkoxy, halogénoalkoxy, -X-Aryle où X représente -O-, - NH-, -N(Alk)-, -N(COCH₃)-, -S-, -SO-, -SO₂-, -CO- ou -CONH-, et/ou
   - éventuellement substitué par un atome d'halogène ou par un groupement alkyle ou halogénoalkyle.

Les composés qui sont encore plus préférés sont les composés de formule (I) dans laquelle :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
**G₂** représente un groupement 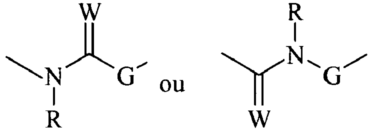 dans lequel :
   - R représente un atome d'hydrogène, un groupement alkyle, halogénoalkyle, ou un radical pro-drogue tel que carbamate, acétyle, dialkylaminométhyle, ou -CH₂-O-CO-Alk,
   - G représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques, et
   - W représente un atome d'oxygène, de soufre ou NH,
**R₁** et **R_{2.}** identiques ou différents, représentent chacun un groupement choisi parmi un atome d'hydrogène, un atome d'halogène, un groupement hydroxyle, thio, alkoxy, halogénoalkoxy, alkylthio, halogénoalkylthio, amino, monoalkylamino, dialkylamino, cycloalkyle, alkyle ou halogénoalkyle,
**R₃** représente un groupement acide ou un radical pro-drogue de la fonction acide tel qu'ester, ou bien un bioisostère de la fonction acide tel que tétrazole, phosphonate, phosphonamide, sulfonate ou sulfonamide,
**A** représente un groupement aryle éventuellement substitué par un ou deux groupements, identiques ou différents, choisis parmi :
   - un atome d'hydrogène, un atome d'halogène,
   - un groupement alkoxy, alkylthio, halogénoalkoxy, halogénoalkylthio, hydroxyle, thio, cyano, amino, monoalkylamino ou dialkylamino,
   - un groupement -SOnR', -COR', -CO₂R', -OCOR', -CONR'R", -NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
   - un groupement alkyle ou halogénoalkyle, le groupement alkyle étant éventuellement substitué par un groupement cyano, amino, monoalkylamino, dialkylamino ou acylamino,
   - un groupement aryle, arylalkyle, -X-aryle, où X représente -O-, -NH-, -N(Alk)-, -N(COCH₃)-, -S-, -SO-, -SO₂-, -CO- ou -CONH-, chacun substitué sur la partie aryle par un ou deux substituants, identiques ou différents, choisis parmi :
      ✔ un atome d'hydrogène ou un atome d'halogène,
      ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, ou
      ✔ un groupement -SOnR', -COR', -CO₂R', -OCOR', -CONR'R", - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
   - un groupement hétérocycle, -X-hétérocycle, où X représente -O-, -NH-, -N(Alk)-, -N(COCH₃)-, -S-, -SO-, -SO₂-, -CO- ou -CONH-, chacun éventuellement substitué sur la partie hétérocycle par un ou deux substituants, identiques ou différents, choisis parmi :
      ✔ un atome d'hydrogène ou un atome d'halogène,
      ✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, ou
      ✔ un groupement -SOnR', -COR', -CO₂R', -OCOR', -CONR'R", -NR'COR" ou -NR'SO₂R'', dans lequel R' et R" représentent chacun indépendamment l'un de l'autre un atome d'hydrogène, un groupement alkyle, halogénoalkyle, et n vaut 1 ou 2, ou
   - un groupement cycloalkyle ou -X-cycloalkyle, où X représente -O-, -NH-, - N(Alk)-, -N(COCH₃)-, -S-, -SO-, -SO₂-, -CO- ou -CONH-, chacun éventuellement substitué sur la partie cyclique par un ou deux substituants, identiques ou différents, choisis parmi un atome d'hydrogène, un atome d'halogène, un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide ou oxo, et
**B** représente un groupement phényle ou pyridine :
   - substitué en position ortho par un groupement N-cycloalkyle tel que pipéridine ou par un cyclohexyle, chacun éventuellement substitué par un ou deux substituants, identiques ou différents, choisis parmi un atome d'hydrogène, un groupement alkyle, halogénoalkyle, alkoxy, halogénoalkoxy, -X-Aryle où X représente -O-, - NH-, -N(Alk)-, -N(COCH₃)-, -S-, -SO-, -SO₂-, -CO- ou -CONH-, et/ou
   - éventuellement substitué par un atome d'halogène ou par un groupement alkyle ou halogénoalkyle.

Les composés particulièrement préférés sont les composés de formule (I) dans laquelle :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
de préférence une liaison ou une chaîne hydrocarbonée comprenant 1 ou 2 atomes de carbone,
**G₂** représente un groupement 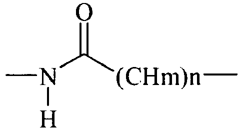 où n est un entier compris entre 1 et 4 et m est un entier valant 1 ou 2, de préférence n vaut 1 ou 2,
**R₁** représente un groupement alkoxy, tel que méthoxy, de préférence en position ortho par rapport à R₃,
**R₂** représente un atome d'hydrogène ou d'halogène, tel que chlore ou brome, ou un groupement alkyle, tel que méthyle, de préférence en position méta par rapport à R₃,
**R₃** représente un groupement acide ou ester,
A représente un groupement aryle, tel que phényle, substitué de préférence :
   - en position méta ou para par :
      - Un atome d'halogène ou un groupement alkyle, halogénoalkyle, cyano, alkoxy, halogénoalkoxy, acylaminoalkyle ou un groupement -XR où X représente -O-, - NH-, -N(Alk)-, -N(COCH₃)-, -S-, -SO-, -SO₂-, -CO- ou -CONH- et R représente un groupement arylalkyle, cycloalkyle ou aryle, chacun éventuellement substitué par un ou deux substituants, identiques ou différents, tels que atome d'halogène, groupement alkoxy, alkyle, halogénoalkyle, cyano, acyle, amino, monoalkylamino ou dialkylamino, acide, ester, amide, mono ou dialkylamide, ou un groupement -SOnR', -OCOR', -NR'COR" ou -NR'SO₂R'', dans lequel R' et R" représentent chacun indépendamment l'un de l'autre un atome d'hydrogène, un groupement alkyle, halogénoalkyle, et n vaut 1 ou 2, ou
      - Un groupement cycloalkyle, aryle, arylalkyle ou hétérocycle, de préférence N-cycloalkyle, chacun étant éventuellement substitué par un ou deux substituants, identiques ou différents, tels que atome d'halogène, groupement alkoxy, alkyle, halogénoalkyle, cyano, acyle, amino, monoalkylamino ou dialkylamino, acide, ester, amide, mono ou dialkylamide, ou un groupement -SOnR', -OCOR', - NR'COR" ou -NR'SO₂R'', dans lequel R' et R" représentent chacun indépendamment l'un de l'autre un atome d'hydrogène, un groupement alkyle, halogénoalkyle, et n vaut 1 ou 2,
   - et/ou en position ortho ou méta par un groupement alkyle, et
**B** représente un groupement aryle, de préférence un phényle,
   - substitué en position ortho par un hétérocycle, de préférence un N-cycloalkyle, tel qu'un groupement pipéridine, et/ou
   - substitué en position ortho' par un groupement alkyle, tel qu'un méthyle.

Les composés particulièrement préférés sont les composés de formule (I) dans laquelle :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
de préférence une liaison ou une chaîne hydrocarbonée comprenant 1 ou 2 atomes de carbone,
**G₂** représente un groupement 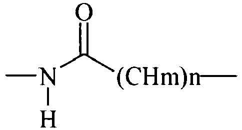 où n est un entier compris entre 1 et 4 et m est un entier valant 1 ou 2, de préférence n vaut 1 ou 2,
**R₁** représente un groupement alkoxy, tel que méthoxy, de préférence en position ortho par rapport à R₃,
**R₂** représente un atome d'hydrogène ou d'halogène, tel que chlore ou brome, ou un groupement alkyle, tel que méthyle, de préférence en position méta par rapport à R₃,
**R₃** représente un groupement acide ou ester,
**A** représente un groupement aryle, tel que phényle, substitué de préférence :
   - en position méta ou para par :
      - Un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, tels que atome d'halogène, groupement alkoxy ou acyle, ou
      - Un groupement cycloalkyle, aryle ou arylalkyle, chacun éventuellement substitué par un ou deux substituants, identiques ou différents, tels qu'un groupement acyle ou alkoxy, et
   - et/ou en position ortho ou méta par un groupement alkyle, et
**B** représente un groupement aryle, de préférence un phényle,
   - substitué en position ortho par un hétérocycle, de préférence un N-cycloalkyle, tel qu'un groupement pipéridine, et/ou
   - substitué en position ortho' par un groupement alkyle, tel qu'un méthyle.

Les composés particulièrement préférés sont les composés de formule (I) dans laquelle :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
de préférence une liaison ou une chaîne hydrocarbonée comprenant 1 ou 2 atomes de carbone,
**G₂** représente un groupement 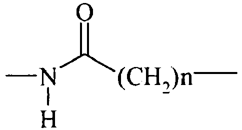 où n est un entier compris entre 1 et 4, de préférence n vaut 1,
**R₁** représente un groupement alkoxy, tel que méthoxy, de préférence en position ortho par rapport à R₃,
**R₂** représente un atome d'hydrogène ou d'halogène, tel que chlore ou brome, ou un groupement alkyl, tel que méthyle, de préférence en position méta par rapport à R₃,
**R₃** représente un groupement acide ou ester,
**A** représente un groupement aryle, tel que phényle, substitué de préférence en position méta ou para par :
   - Un atome d'halogène ou un groupement alkoxy, halogénoalkoxy ou -XR où X représente -O- et R représente un groupement arylalkyle, cycloalkyle ou aryle, chacun éventuellement substitué par un ou deux substituants, identiques ou différents, tels que atome d'halogène, groupement alkoxy ou acyle, ou
   - Un groupement cycloalkyle, aryle ou arylalkyle, chacun éventuellement substitué par un ou deux substituants, identiques ou différents, tels qu'un groupement acyle, et
**B** représente un groupement aryle, de préférence un phényle,
   - substitué en position ortho par un hétérocycle, de préférence un N-cycloalkyle, tel qu'un groupement pipéridine, et/ou
   - substitué en position ortho' par un groupement alkyle, tel qu'un méthyle.

Les composés encore plus particulièrement préférés sont rassemblés dans le tableau I :

**Tableau 1**

| | |
|---|---|
| **1** | Chlorhydrate d'acide 5-bromo-2-méthoxy-4-[*N'*-(4-méthoxy-phényl)-*N'*-(2-pipéridin-1-yl-phényl)-hydrazinocarbonylméthyl]-benzoïque |
| **2** | Chlorhydrate d'acide 5-bromo-2-méthoxy-4-[*N'*-(2-pipéridin-1-yl-phényl)-*N'*-(4-trifluorométhoxy-phényl)-hydrazinocarbonylméthyl]-benzoïque |
| **3** | Chlorhydrate d'acide 5-bromo-2-méthoxy-4-[*N'*-(3-méthoxy-benzyl)-*N'*-(2-pipéridin-1-yl-phényl)-hydrazinocarbonylméthyl]-benzoïque |
| **4** | Chlorhydrate d'acide 4-[*N'*-(4-benzyloxy-phényl)-*N'*-(2-pipéridin-1-yl-phényl)-hydrazinocarbonylméthyl]-5-bromo-2-méthoxy-benzoïque |
| **5** | Chlorhydrate d'acide 5-bromo-4-{*N'*-[4-(4-fluoro-phénoxy)-phényl]-*N'*-(2-pipéridin-1-yl-phényl)-hydrazinocarbonylméthyl}-2-méthoxy-benzoïque |
| **6** | Chlorhydrate d'acide 5-bromo-2-méthoxy-4-{*N'*-[2-(4-méthoxy-phényl)-éthyl]-*N'-*(2-pipéridin-1-yl-phényl)-hydrazinocarbonylméthyl} -benzoïque |
| **7** | Chlorhydrate d'acide 5-bromo-2-méthoxy-4-[*N'*-(4-méthoxy-phényl)-*N'*-(2-méthyl-6-pipéridin-1-yl-phényl)-hydrazinocarbonylméthyl]-benzoïque |
| **7a** | 5-Bromo-2-méthoxy-4-[*N'*-(4-méthoxy-phényl)-*N'*-(2-méthyl-6-pipéridin-1-yl-phényl)-hydrazinocarbonylméthyl]-benzoate de méthyle |
| **8** | Chlorhydrate d'acide 5-bromo-2-méthoxy-4-[*N'*-(4-méthoxy-benzyl)-*N'*-(2-pipéridin-1-yl-phényl)-hydrazinocarbonylméthyl]-benzoïque |
| **9** | Chlorhydrate d'acide 5-bromo-4-[*N'*-(4-cyclohexyl-phényl)-*N'*-(2-pipéridin-1-yl-phényl)-hydrazinocarbonylméthyl]-2-méthoxy-benzoïque |
| **10** | Chlorhydrate d'acide 5-bromo-2-méthoxy-4-[*N'*-(2-méthyl-6-pipéridin-1-yl-phényl)-*N'*-(4-trifluorométhoxy-phényl)-hydrazinocarbonylméthyl]-benzoïque |
| **11** | Chlorhydrate d'acide 5-bromo-2-méthoxy-4-[*N'*-(4'-méthoxy-biphényl-4-yl)-*N'*-(2-pipéridin-1-yl-phényl)-hydrazinocarbonylméthyl]-benzoïque |
| **12** | Chlorhydrate d'acide 5-bromo-4-[*N'*-(4-cyclohexyloxy-phényl)-*N'*-(2-pipéridin-1-yl-phényl)-hydrazinocarbonylméthyl]-2-méthoxy-benzoïque |
| **13** | Chlorhydrate d'acide 5-bromo-2-méthoxy-4-[*N'*-(4-phénoxy-phényl)-*N'*-(2-pipéridin-1-yl-phényl)-hydrazinocarbonylméthyl]-benzoïque |
| **14** | Chlorhydrate d'acide 5-bromo-4-{*N'*-[4-(4-chloro-phénoxy)-phényl]-*N'*-(2-pipéridin-1-yl-phényl)-hydrazinocarbonylméthyl}-2-méthoxy-benzoïque |
| **15** | Chlorhydrate d'acide 4-{*N'*-[4-(4-fluoro-phénoxy)-phényl]-*N'*-(2-méthyl-6-pipéridin-1-yl-phényl)-hydrazinocarbonylméthyl}-2-méthoxy-benzoïque |
| **16** | Chlorhydrate d'acide 5-bromo-4-{*N'*-[4-(4-fluoro-phénoxy)-phényl]-*N'*-(2-méthyl-6-pipéridin-1-yl-phényl)-hydrazinocarbonylméthyl}-2-méthoxy-benzoïque |
| **16a** | 5-Bromo-4-{*N'*-[4-(4-fluoro-phénoxy)-phényl]-*N'*-(2-méthyl-6-pipéridin-1-yl-phényl)-hydrazinocarbonylméthyl}-2-méthoxy-benzoate de méthyle |
| **17** | Chlorhydrate d'acide 4-[*N'*-(4-benzyl-phényl)-*N'*-(2-pipéridin-1-yl-phényl)-hydrazinocarbonylméthyl]-5-bromo-2-méthoxy-benzoïque |
| **18** | Chlorhydrate d'acide 4-[*N'*-(4-bromo-phényl)-*N'*-(2-pipéridin-1-yl-phényl)-hydrazinocarbonylméthyl]-5-chloro-2-méthoxy-benzoïque |
| **19** | Chlorhydrate d'acide 4-[*N'*-(3'-acétyl-biphényl-4-yl)-*N'*-(2-pipéridin-1-yl-phényl)-hydrazinocarbonylméthyl]-5-chloro-2-méthoxy-benzoïque |
| **20** | Chlorhydrate d'acide 4-[*N'*-(4'-acétyl-biphényl-4-yl)-*N'*-(2-pipéridin-1-yl-phényl)-hydrazinocarbonylméthyl]-5-chloro-2-méthoxy-benzoïque |
| **21** | Chlorhydrate d'acide 5-bromo-2-méthoxy-4-[*N'*-(3-phénoxy)-phényl]-*N'*-(2-pipéridin-1-yl-phényl)-hydrazinocarbonylméthyl]-benzoïque |
| **22** | Chlorhydrate d'acide 5-bromo-2-méthoxy-4-[*N'*-(4-phénylsulfanyl)-phényl]-*N'*-(2-pipéridin-1-yl-phényl)-hydrazinocarbonylméthyl]-benzoïque |
| **23** | Chlorhydrate d'acide 4-[*N'*-(4-benzènesulfonyl-phényl)-*N'*-(2-pipéridin-1-yl-phényl)-hydrazinocarbonylméthyl]-5-bromo-2-méthoxy-benzoïque |
| **24** | Chlorhydrate d'acide 4-[*N'*-(4-benzènesulfinyl-phényl)-*N'*-(2-pipéridin-1-yl-phényl)-hydrazinocarbonylméthyl]-5-bromo-2-méthoxy-benzoïque |
| **25** | Chlorhydrate d'acide 2-méthoxy-4-{(*E*)-2-[*N'*-(4-méthoxy-phényl)-*N'*-(2-méthyl-6-pipéridin-1-yl-phényl)-hydrazinocarbonyl]-vinyl}-benzoïque |
| **26** | Chlorhydrate d'acide 5-bromo-2-méthoxy-4-{(*E*)-2-[*N'*-(4-méthoxy-phényl)-*N'*-(2-méthyl-6-pipéridin-1-yl-phényl)-hydrazinocarbonyl]-vinyl}-benzoïque |
| **27** | Chlorhydrate d'acide 4-[*N'*-(4-benzyl-phényl)-*N'*-(2-méthyl-6-pipéridin-1-yl-phényl)-hydrazinocarbonylméthyl]-5-bromo-2-méthoxy-benzoïque |
| **28** | Chlorhydrate d'acide 5-bromo-2-méthoxy-4-[*N'*-(4'-méthoxy-biphényl-4-yl)-*N'*-(2-méthyl-6-pipéridin-1-yl-phényl)-hydrazinocarbonylméthyl]-benzoïque |
| **29** | Chlorhydrate d'acide 4-[*N'*-(acétylamino-méthyl)-phényl]-*N'*-(2-méthyl-6-pipéridin-1-yl-phényl)-hydrazinocarbonylméthyl]-5-bromo-2-méthoxy-benzoïque |
| **30** | Chlorhydrate d'acide 4-[*N'*-(4-benzoyl-phényl]-*N'*-(2-pipéridin-1-yl-phényl)-hydrazinocarbonylméthyl]-5-bromo-2-méthoxy-benzoïque |
| **31** | Chlorhydrate d'acide 5-bromo-4-[*N'*-(4-cyano-phényl)-*N'*-(2-méthyl-6-pipéridin-1-yl-phényl)-hydrazinocarbonylméthyl]-2-méthoxy-benzoïque |
| **32** | Chlorhydrate d'acide 4-[*N'*-(4'-acétyl-biphényl-4-yl)-*N'*-(2-méthyl-6-pipéridin-1-yl-phényl)-hydrazinocarbonylméthyl]-5-bromo-2-méthoxy-benzoïque |
| **33** | Chlorhydrate d'acide 5-bromo-2-méthoxy-4-[*N'*-(4'-méthoxy-2-méthyl-biphényl-4-yl)-*N'*-(2-méthyl-6-pipéridin-1-yl-phényl)-hydrazinocarbonylméthyl]-benzoïque |

La présente invention a également pour objet les compositions pharmaceutiques comprenant au moins un composé de formule (I), en association avec un excipient pharmaceutiquement acceptable.

Les compositions pharmaceutiques selon l'invention peuvent être des compositions administrables dans l'organisme par toute voie d'administration. De manière non-exhaustive, la voie d'administration des compositions pharmaceutiques selon l'invention peut être topique, entérale ou parentérale, de préférence une administration buccale, conjonctivale, cutanée, endotrachéale, intradermale, intraépidermale, intramusculaire, intravasculaire, laryngale, nasale, ophtalmique, orale, rectale, respiratoire, sous-cutanée, transcutanée ou vaginale. Il est généralement avantageux de formuler de telles compositions pharmaceutiques sous forme de dose unitaire. Chaque dose comprend alors une quantité prédéterminée du principe actif, associée au véhicule, excipients et/ou adjuvants appropriés, calculée pour obtenir un effet thérapeutique donné. A titre d'exemple de forme de dose unitaire administrable par voie orale on peut citer les comprimés, les gélules, les granules, les poudres et les solutions ou suspensions orales. A titre d'exemple de forme de dose unitaire administrable par voie topique (notamment pour le traitement local des verrues génitales et péri-anales externes), on peut citer les ovules, gels, crèmes, lotions, solutions et patchs.

Les formulations appropriées pour la forme d'administration choisie sont connues et décrites, par exemple dans le Remington, The Science and Practice of Pharmacy, 19ième édition, 1995, Mack Publishing Company et peuvent donc être facilement préparées par l'homme de l'art.

Il est connu que la posologie varie d'un individu à l'autre, selon la nature et l'intensité de l'affection, la voie d'administration choisie, le poids, l'âge et le sexe du malade en conséquence les doses efficaces devront être déterminées en fonction de ces paramètres par le spécialiste en la matière. A titre indicatif, les doses efficaces pourraient s'échelonner entre 1 et 500 mg par jour.
La présente invention a également pour objet l'utilisation des composés de formule (I) pour le traitement ou la prévention d'une infection par le virus du papillome, chez l'Homme de préférence.

La présente invention a également pour objet l'utilisation des composés de formule (I) pour inhiber la réplication du virus du papillome par inhibition de la formation du complexe protéinique E1/E2.

La présente invention a en outre pour objet l'utilisation des composés de formule (I) pour la préparation d'un médicament destiné au traitement ou à la prévention d'une infection par le virus du papillome, chez l'Homme de préférence.

La présente invention a en particulier pour objet l'utilisation des composés de formule (I) pour la préparation d'un médicament destiné au traitement ou à la prévention d'une infection par un virus du papillome à bas risque, tel que VPH6, VPH7, VPH11, VPH13, VPH32, VPH34, VPH40, VPH42, VPH43, VPH44, VPH53, VPH54, VPH55, VPH57, VPH58, VPH74, VPH91.

La présente invention a en particulier pour objet l'utilisation des composés de formule (I) pour la préparation d'un médicament destiné au traitement ou à la prévention d'une infection par VPH6 et/ou VPH11.

Ainsi, la présente invention a également pour objet l'utilisation des composés de formule (I) pour la préparation d'un médicament destiné au traitement ou à la prévention des lésions et maladies associées aux infections par le virus de papillome.

La présente invention a en particulier pour objet l'utilisation des composés de formule (I) pour la préparation d'un médicament destiné au traitement ou à la prévention des verrues ano-génitales, comme les condylomes acuminés et condylomes plans, les papillomes laryngés, conjonctivaux ou buccaux et d'autres lésions épithéliales, comme des papillomatoses récurrentes respiratoires et des néoplasies intra-épithéliales de bas grade et de haut grade, des papuloses bowénoïdes, des verrues (vulgaires, plantaires, myrmécies, superficielles, plates...), des épidermodysplasies verruciformes, des carcinomes, en particulier ano-génitaux, et toutes les lésions qui sont associées au virus du papillome.

La présente invention a en particulier pour objet l'utilisation des composés de formule (I) pour la préparation d'un médicament destiné au traitement ou à la prévention des verrues ano-génitales, comme les condylomes acuminés et condylomes plans, les papillomes laryngés, conjonctivaux ou buccaux et d'autres lésions épithéliales, comme des papillomatoses récurrentes respiratoires et des néoplasies intra-épithéliales de bas grade et toutes les lésions qui sont associées au virus du papillome.

Les composés objets de la présente invention peuvent être préparés selon les diverses voies de synthèse décrites ci-après.

Les composés où G₂ représente le radical 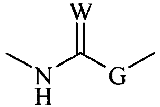 tel que défini précédemment peuvent être préparés de la façon suivante.

Lorsque W représente un atome d'oxygène, les précurseurs ester des composés de formule (I) sont obtenus à partir des intermédiaires de formule (II) et (III) suivants :

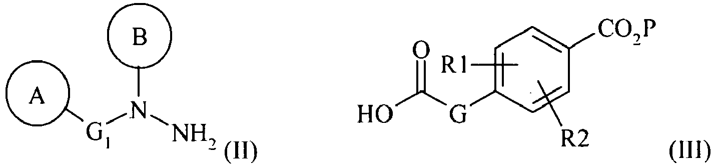

Dans la formule (II), A, B et G₁, sont tels que définis précédemment. Dans la formule (III), R₁, R₂ et G sont tels que définis précédemment et P représente un groupe protecteur de fonction acide, tel que (C₁-C₄)alkyle linéaire ou ramifié.

Dans ce cas, on effectue un couplage peptidique entre les composés (II) et (III) en présence par exemple d'EDCI dans un milieu basique et apolaire pour conduire au composé de formule (IV) :

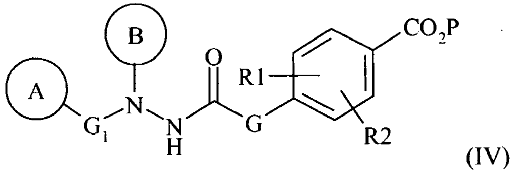

On peut aussi obtenir le composé (IV) de la façon suivante. On transforme le composé (III) en chlorure d'acide correspondant de formule (V) par action du chlorure de thionyle par exemple, 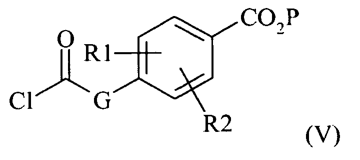 dans lequel R₁, R₂, G et P sont tels que définis précédemment et que l'on fait réagir sur le composé (II).

Puis on déprotège le groupement -CO₂P du composé de formule (IV) par hydrolyse, pour obtenir le composé de formule (I) 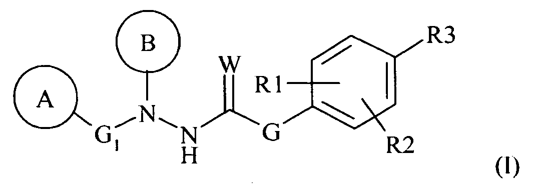 dans laquelle W représente un atome d'oxygène et R₃ est tel que défini précédemment. Le cas échéant, on fait réagir le composé de formule (I) obtenu à l'issue de l'étape de déprotection ou bien le composé de formule (IV) avec le réactif de Lawesson, ce qui permet d'obtenir le composé de formule (VI) : 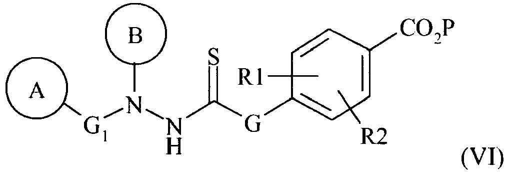 qui, après hydrolyse, correspond au cas où W représente un atome de soufre dans la formule (I). Le réactif de Lawesson peut par exemple être du [2,4-bis(4-méthoxyphényl)1,3-dithia-2,4-diphosphétane-2,4-disulfide (Lawesson et al. Bull. Soc. Chim. Belg. 1978, 87, 229).

Dans le cas où G₁ représente une liaison, les composés de formule (II) peuvent être obtenus à partir des composés de formule (VII) : 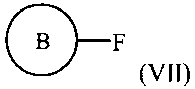 dans laquelle B est tel que défini précédemment.

On soumet les composés de formule (VII) à une substitution nucléophile aromatique en milieu basique et polaire en présence de composés (VIII) de formule : 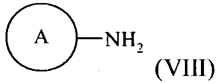 dans laquelle A est tel que défini précédemment et G₁ représente une liaison, et on obtient les composés de formule (IX) : 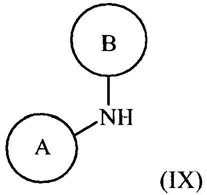 dans laquelle A et B sont tels que définis précédemment.

Dans le cas où G₁ représente une chaîne hydrocarbonée telle que définie dans la formule (I), on fait réagir le composé de formule (X) : 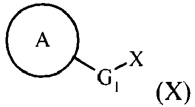 dans laquelle A et G₁ sont tels que définis précédemment et X représente un atome d'halogène, dans des conditions basiques et polaires, avec un composé de formule (XI) : 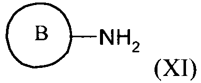 dans laquelle B est tel que défini précédemment, et on obtient les composés de formule (XII) : 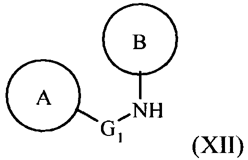 dans laquelle A, B et G₁ sont tels que définis précédemment.

Les composés de formule (IX) et (XII) sont mis en présence de nitrite de sodium en milieu acide puis réduit par un hydrure, par exemple l'hydrure de lithium et d'aluminium (J. Org. Chem. 1953, 18, 971, J. (Org. Chem. 1954, 19, 1157 et J. Am. Chem. Soc. 1952, 74, 3192) pour donner les composés de formule (II) tels que définis précédemment.
Dans le cas où R₂ représente un atome d'hydrogène ou de brome, les composés de formule (III) peuvent être obtenus selon les méthodes de la littérature (J. Med. Chem. 1998, 41, 5219 ou WO 0135900).

Dans le cas où R₂ représente un atome de chlore, les composés de formule (III) peuvent être obtenus en faisant réagir du chlorure de sulfuryle en milieu acide avec un précurseur de formule (XIII) : 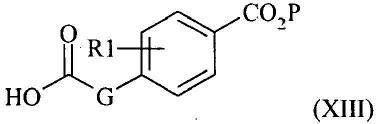 dans laquelle R₁, G et P sont tels que définis précédemment.

Les composés de formule (VII), (VIII), (X) et (XI) sont soit des composés commerciaux, soit des composés obtenus selon des méthodes connues de la synthèse organique aisément accessibles et compréhensibles à l'homme de métier.

Dans le cas préféré où B est un phényle substitué par une pipéridine, on peut préparer les composés de formule (I) selon la voie de synthèse suivante.

Dans le cas où G₁ représente une liaison, les composés de formule (II) peuvent être obtenus à partir des composés de formule (XIV) : 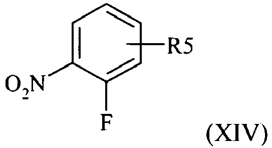 dans laquelle R₅ est tel que défini précédemment.

On soumet les composés de formule (XIV) à une substitution nucléophile aromatique en milieu basique et polaire en présence de composés (VIII), et on obtient les composés de formule (XV) : 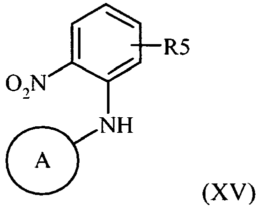 dans laquelle A et R₅ sont tels que définis précédemment.

Les composés de formule (XV) sont réduits par du chlorure d'étain en milieu polaire (Tet. Lett. 1984, 25 (8), 839) puis mis à réagir avec un dibromoalkane, par exemple du dibromopentane en milieu basique et apolaire (Bioorg. Med. Chem. Lett. 1996, 6 (5), 563) pour conduire aux composés de formule (XVI) : 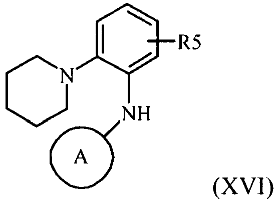 dans laquelle A et R₅ sont tels que définis précédemment,

Dans le cas où G₁ représente une chaîne hydrocarbonée telle que définie dans la formule (I), on fait réagir le composé de formule (X), dans des conditions basiques et polaires, avec un composé de formule (XVII) : 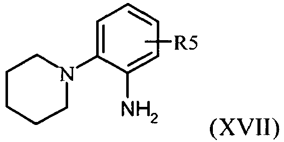 dans laquelle R₅ est tel que défini précédemment, et on obtient les composés de formule (XVIII) : 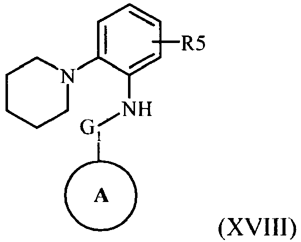 dans laquelle A, G₁ et R₅ sont tels que définis précédemment.

Les composés de formule (XVI) et (XVIII) sont mis en présence de nitrite de sodium en milieu acide puis réduit par un hydrure, par exemple l'hydrure de lithium et d'aluminium pour donner les composés de formule (II) tels que définis précédemment.

Dans le cas particulier où A représente un phényle substitué et G1 est une liaison, on peut préparer les composés de formule (I) selon la voie de synthèse suivante.

On fait réagir le composé de formule (XIX) : 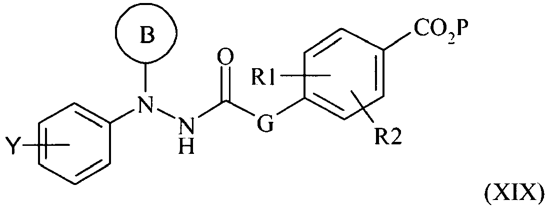 dans laquelle B, G, R₁, R₂ et P sont tels que définis précédemment et Y représente un halogène tel que brome ou iode ou un groupement triflate, dans des conditions basiques de couplage au palladium, avec un composé de formule (XX) : 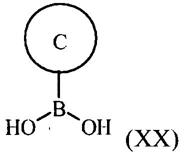 dans laquelle C représente un aryle ou un hétérocycle diversement substitué, pour conduire au composé (XXI) de formule : 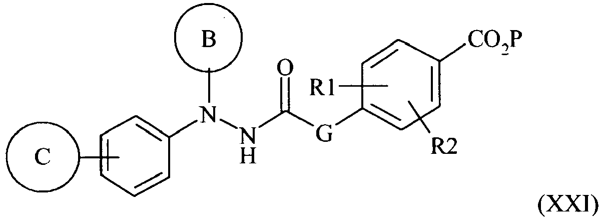 dans laquelle B, C, G, R₁, R₂ et P sont tels que définis précédemment.

Puis on fait réagir le composé (XXI) dans des conditions basiques de saponification pour conduire au composé de formule générale (I).

Les composés où G₂ représente le radical 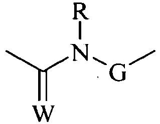 tel que défini précédemment peuvent être préparés de la façon suivante.

On fait réagir les composés de formule (IX) ou (XII) tels que décrits précédemment avec un composé de formule (XXII): 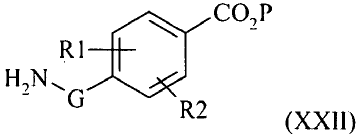 dans laquelle R₁, R₂, G et P sont tels que définis précédemment,

soit directement en présence de triphosgène par exemple ou par transformation préalable d'un des précurseurs en chlorure de carbamoyle. Puis on effectue une hydrolyse finale.

Le composé (XXII) peut être obtenu à partir du composé (XXIII) de formule : 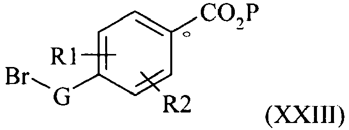 dans laquelle R₁, R₂, G et P sont tels que définis précédemment,
en faisant réagir le composé (XXIII) en présence d'hexaméthylènetétramine (HMTA) dans un solvant apolaire suivi d'un traitement acide.

Les composés où G₂ représente le radical 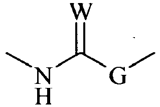 tel que défini précédemment peuvent être aussi préparés de la façon suivante.

Lorsque W représente un atome d'oxygène, les précurseurs ester des composés de formule (I) sont obtenus à partir des intermédiaires de formule (XXIV) et (V) suivants :

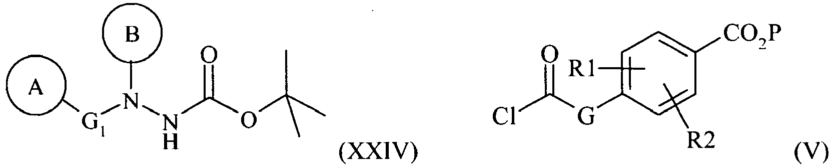

Dans la formule (XXIV), A, B et G₁, sont tels que définis précédemment. Dans la formule (V), R₁, R₂, et G sont tels que définis précédemment et P représente un groupe protecteur de fonction acide, tel que (C₁-C₄)alkyle linéaire ou ramifié.

Dans ce cas, on fait réagir le composé (V) sur le composé (XXIV) dans un milieu acide pour conduire au composé de formule (IV) :

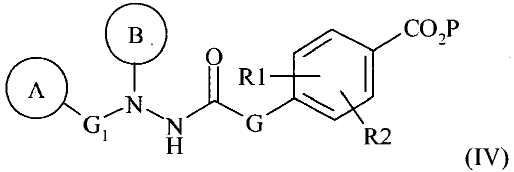

Puis on déprotège le groupement -CO₂P du composé de formule (IV) par hydrolyse, pour obtenir le composé de formule (I) : 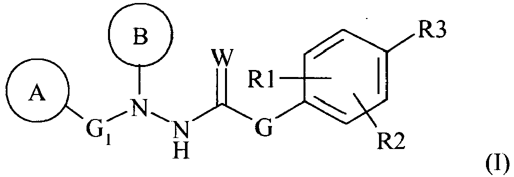 dans laquelle W représente un atome d'oxygène et R₃ est tel que défini précédemment.

Dans le cas préféré où B est un phényle substitué par une pipéridine et où G₁ représente une liaison, les composés de formule (XXIV) peuvent être obtenus à partir des composés de formule (XIV) : 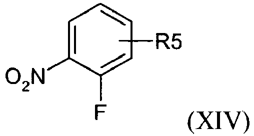 dans laquelle R₅ est tel que défini précédemment.

On soumet les composés de formule (XIV) à une substitution nucléophile aromatique en milieu polaire en présence d'hydrazine bocquée commerciale, et on obtient les composés de formule (XXV) : 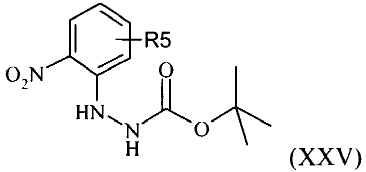 dans laquelle R₅ est tel que défini précédemment:

Le composé de formule (XXV) est mis en présence d'oxyde de manganèse (Org. Letters, 2006, 8, 1, 43) puis soumis à une réaction de N-arylation pour conduire au composés de formule (XXVI) : 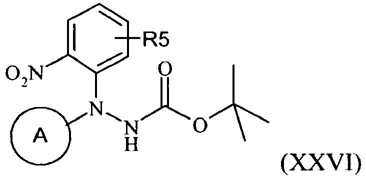 dans laquelle A et R₅ sont tels que définis précédemment.

Les composés de formule (XXVI) sont réduits par hydrogénation catalytique puis mis à réagir avec un dibromoalkane ou un chlorure d'acide, par exemple du chlorure de 5-bromovaléryle en milieu basique et apolaire, puis cyclisé en milieu basique et polaire comme par de l'hydrure de sodium dans la diméthylformamide et enfin réduit par du borane, pour conduire au composé de formule (XXVII) : 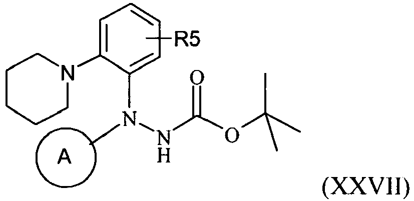 dans laquelle A et R₅ sont tels que définis précédemment.

La présente invention a également pour objet les intermédiaires de synthèse correspondant aux composés (II) rassemblés dans le tableau (II).

**Tableau II**

| | |
|---|---|
| **a** | *N*-(4-Méthoxy-phényl)-*N*-(2-pipéridin-1-yl-phényl)-hydrazine |
| **b** | *N*-(2-Pipéridin-1-yl-phényl)-*N*-(4-ttifluorométhoxy-phényl)-hydrazinc |
| **c** | *N*-(3-Méthoxy-benzyl)-*N*-(2-pipéridin-1-yl-phényl)-hydrazine |
| **d** | *N*-(4-Benzyloxy-phényl)-*N*-(2-pipéridin-1-yl-phényl)-hydrazine |
| **e** | *N*-[4-(4-Fluoro-phénoxy)-phényl]-*N*-(2-pipéridin-1-yl-phényl)-hydrazine |
| **f** | *N*-[2-(4-Méthoxy-phényl)-éthyl]-*N*-(2-pipéridin-1-yl-phényl)-hydrazine |
| **g** | *N*-(4-Méthoxy-phényl)-*N*-(2-méthyl-6-pipéridin-1-yl-phényl)-hydrazine |
| **h** | *N*-(4-Méthoxy-benryl)-*N*-(2-pipéridin-1-yl-phényl)-hydrazine |
| **i** | *N*-(4-Cyclohexyl-phényl)-*N*-(2-pipéridin-1-yl-phényl)-hydrazine |
| **j** | *N*-(2-Méthyl-6-pipéridin-1-yl-phényl)-*N*-(4-trifluorométhoxy-phényl)-hydrazine |
| **k** | *N*-(4'-Méthoxy-biphényl-4-yl)-*N*-(2-pipéridine-1-yl-phényl)-hydrazine |
| **l** | *N*-(4-Cyclohexyloxy-phényl)-*N*-(2-pipéridin-1-yl-phényl)-hydrazine |
| **m** | *N*-(4-Phénoxy-phényl)-*N*-(2-pipéridin-1-yl-phényl)-hydrazine |
| **n** | *N*-[4-(4-Chloro-phénoxy)-phényl]-*N*-(2-pipéridin-1-yl-phényl)-hydrazine |
| **o** | *N*-[4-(4-Fluoro-phénoxy)-phényl]-*N*-(2-méthyl-6-pipéridin-1-yl-phényl)-hydrazine |
| **p** | *N*-(4-Benzyl-phényl]-*N*-(2-pipéridin-1-yl-phényl)-hydrazine |
| **q** | *N*-(4-Bromo-phényl)-*N*-(2-pipéridin-1-yl-phényl)-hydrazine |
| **r** | *N*-(3-Phénoxy-phényl)-*N*-(2-pipéridin-1-yl-phényl)-hydrazine |
| **s** | *N*-(4-Phénylsulfanyl-phényl)-*N*-(2-pipéridin-1-yl-phényl)-hydrazine |
| **t** | *N*-(4-Benzyl-phényl)-*N-*(2-méthyl-6-pipéridin-1-yl-phényl)-hydrazine |
| **u** | *N*-(4'-Méthoxy-biphényl-4-yl)-*N*-(2-méthyl-6-pipéridin-1-yl-phényl)-hydrazine |
| **v** | *N*-(4-Benzoyl-phényl)-*N*-(2-pipéridin-1-yl-phényl)-hydrazine |
| **w** | *N*-[4'-(2-Méthyl-[1,3]dithian-2-yl)-biphenyl-4-yl]-*N*-(2-méthyl-6-pipéridin-1-yl-phényl)-hydrazine |
| **x** | *N*- (4'-Méthoxy-2-méthyl-biphényl-4-yl)-*N*- (2-méthyl-6-pipéridin-1-yl-phényl)-hydrazine |

Les exemples suivants illustrent l'invention mais ne la limitent en aucune façon.

Les produits de départ utilisés sont des produits commerciaux ou des produits préparés selon des modes opératoires connus à partir de composés commerciaux ou connus par l'homme du métier. Les différentes préparations conduisent à des intermédiaires de synthèse utiles pour la préparation des composés de l'invention.

Les structures des composés décrits dans les exemples et dans les préparations ont été déterminées selon les techniques spéctrophotométriques usuelles (résonance magnétique nucléaire (RMN), spectrométrie de masse (SM) dont électrospray (ES), point de fusion (PF)...) et la pureté a été déterminée par chromatographie liquide à haute performance (HPLC) et confirmée par l'analyse élémentaire.

Abréviations utilisées dans les modes opératoires :
- AIBN : 2,2'-azobis(2-méthylpropionitrile)
- CCM : chromatographie sur couche mince
- EDCI : chlorhydrate de 1-(3-diméthylaminopropyl)-3-éthylcarbodiimide
- DMAP : 4-diméthylaminopyridine
- DMSO : diméthylsulfoxyde
- DIPEA : *N,N*-diisopropyléthylamine
- HOBt : 1-hydroxybenzotriazole
- TFA : acide trifluoroacétique

### Préparation 1 : 4-Carboxyméthyl-2-méthoxy-benzoate de méthyle

Le 4-carboxyméthyl-2-méthoxy benzoate de méthyle peut être préparé selon la méthode décrite dans J. Med. Chem. 1998, 41, 5219 ou le brevet WO 0135900.

### Préparation 2 : 5-Bromo-4-carboxyméthyl-2-méthoxy-benzoate de méthyle

Le 5-bromo-4-carboxyméthyl-2-méthoxy benzoate de méthyle est obtenu à partir de la préparation 1 suivant le protocole décrit dans le brevet WO 0135900.

### Préparation 3 : 5-Chloro-4-carboxyméthyl-2-méthoxy-benzoate de méthyle

A 300 mg de 4-carboxyméthyl-2-méthoxy benzoate de méthyle issu de la préparation 1 placés dans 6 mL d'acide acétique sont ajoutés 110 µL de chlorure de sulfuryle (1 équivalent). L'ensemble est porté à reflux pendant 6 heures. Après évaporation du solvant, le brut réactionnel est purifié sur gel de silice (éther de pétrole/acétate d'éthyle : 80/20 puis 60/40) pour conduire à 165 mg du composé désiré.
Rendement : 47 %
HPLC : 96 %
SM : MH⁺ 259/261

### Préparation 4 : 4-((E)-2-Carboxy-vinyl)-2-méthoxy-benzoate de méthyle

### Stade 1 : 4-Bromométhyl-2-méthoxy-benzoate de méthyle

A 5,38 g de 2-méthoxy-4-méthyl benzoate de méthyle dans 20 mL de tétrachlorure de carbone sont ajoutés 5,3 g de *N*-bromosuccinimide (1 équivalent) et 490 mg de AIBN (0,1 équivalent) à l'abri de la lumière. L'ensemble est chauffé à reflux pendant une nuit. Le milieu réactionnel est évaporé sous pression réduite puis purifié sur gel de silice (éther de pétrole/acétate d'éthyle : 90/10) pour conduire à 3,73 g du produit désiré.
Rendement : 48 %
RMN ¹H (CDCl3, 300 MHz) δ (ppm) : 7,76 (d, 1H), 6,99 (m, 2H), 4,46 (s, 2H), 3,92 (s, 3H), 3,88 (s, 3H)
HPLC : 96 %
SM : MH⁺ 259/261

### Stade 2 : 4-Hydroxyméthyl-2-méthoxy-benzoate de méthyle

A 1,5 g de produit obtenu au stade précédent dans 25 mL de dioxane est ajoutée une suspension de 2,55 g (4,4 équivalents) de carbonate de calcium dans 25 mL d'eau. L'ensemble est chauffé 6 heures à 100 °C. Après évaporation du milieu réactionnel, le brut repris dans du dichlorométhane, est acidifié avec une solution d'acide chlorhydrique 1 N. Le milieu réactionnel est extrait plusieurs fois au dichlorométhane, les phases organiques réunies sont ensuite séchées sur sulfate de magnésium, filtrées et évaporées sous pression réduite pour conduire à 1,10 g du produit souhaité.
Rendement : 96 %
RMN ¹H (CDCl3, 300 MHz) δ (ppm) : 7,78 (d, 1H), 7,01 (m, 1H), 6,93 (d, 1H), 4,73 (s, 2H), 3,91 (s, 3H), 3,88 (s, 3H)

### Stade 3 : 4-Formyl-2-méthoxy-benzoate de méthyle

Au 1,10 g de produit obtenu au stade précédent dans 20 mL de dioxane sont ajoutés 4,87 g d'oxyde de manganèse activé (10 équivalents). L'ensemble est agité à température ambiante pendant 24 heures, puis filtré sur célite. Le filtrat est évaporé sous pression réduite et le résidu obtenu purifié par chromatographie sur gel de silice (éther de pétrole/acétate d'éthyle : 90/10 puis 80/20) pour conduire à 540 mg du produit désiré.
Rendement : 50 %
RMN ¹H (CDCl3, 300 MHz) δ (ppm) : 10,02 (s, 1H), 7,89 (d, 1H), 7,48 (m, 2H), 3,97 (s, 3H), 3,92 (s, 3H)

### Stade 4 : 4-((E)-2-tert-Butoxycarbonyl-vinyl)-2-méthoxy-benzoate de méthyle

A 720 µL de diéthylphosphonoacétate de tert-butyle (1,1 équivalents) dans 3 mL de tétrahydrofurane refroidis à 0 °C sous atmosphère inerte sont ajoutés 307 mg de tert-butanolate de sodium (1,15 équivalents). L'ensemble est agité 30 minutes à 0 °C puis 1 heure à température ambiante. Une solution refroidie à 0-4 °C de 540 mg d'aldéhyde obtenus au stade 3 dans 1 mL de tétrahydrofurane est ajoutée goutte à goutte à l'ensemble précédent également refroidi à 0 °C. L'agitation est maintenue à cette température pendant 30 minutes avant de laisser la température remonter à l'ambiante pendant 2 heures. Le milieu est hydrolysé avec une solution saturée de chlorure d'ammonium et extrait à l'acétate d'éthyle plusieurs fois. Les phases organiques réunies sont lavées à l'eau, puis séchées sur sulfate de magnésium, filtrées et évaporées sous pression réduite. Le brut réactionnel est purifié par chromatographie sur gel de silice (éther de pétrole/acétate d'éthyle : 95/5) pour conduire à 500 mg du produit attendu.
Rendement : 61 %
RMN ¹H (DMSO, 300 MHz) δ (ppm) : 7,48-7,66 (massif aromatique, 3H), 6,70 (d, 1H), 3,87 (s, 3H), 3,79 (s, 3H), 1,49 (s, 9H)
HPLC : 93 %
SM : MH⁺ 293

### Stade 5 : 4-((E)-2-Carboxy-vinyl)-2-méthoxy-benzoate de méthyle,

A 100 mg de diester obtenu au stade précédent dans 1,6 mL de dichlorométhane sont ajoutés 0,8 mL d'acide trifluoroacétique. L'ensemble est agité une heure à température ambiante puis évaporé sous pression réduite avec un mélange de toluène et dichlorométhane comme co-solvants.
Rendement : 99 %
RMN ¹H (DMSO, 300 MHz) δ (ppm) : 7,57-7,67 (m, 2H), 7,47 (s, 1H), 7,32 (d, 1H), 6,67 (d, 1 H), 3,87 (s, 3H), 3,79 (s, 3H)
HPLC : 90 %

### Préparation 5 : 5-Bromo-4-((E)-2-Carboxy-vinyl)-2-méthoxy-benzoate de méthyle

### Stade 1 : 5-Bromo-4-bromométhyl-2-méthoxy-benzoate de méthyle

A 2,5 g de 2-méthoxy-4-méthyl benzoate de méthyle dans 15 mL d'acide acétique sont ajoutés goutte à goutte 550 µL de brome (1,1 équivalents) à température ambiante. L'ensemble est agité une nuit, jusqu'à disparition complète du produit de départ (suivi CCM). Le milieu réactionnel, hydrolysé par une solution de soude 1 N, est extrait à l'acétate d'éthyle. Les phases organiques sont séchées sur sulfate de magnésium puis concentrées sous pression réduite pour conduire à 2,72 g d'une huile jaune pâle qui cristallise.
Rendement : 83 %
RMN ¹H (CDCl3, 300 MHz) δ (ppm) : 7,98 (s, 1H), 7,06 (s, 1H), 4,55 (s, 2H), 3,92 (s, 3H), 3,89 (s, 3H)
HPLC : 85 %
SM : MH⁺ 336/338/340

### Stade 2 : 5-Bromo-4-hydroxyméthyl-2-méthoxy-benzoate de méthyle

Le produit (880 mg) est obtenu selon le procédé du stade 2 de la préparation 4 à partir de 1,3 g de produit du stade précédent en présence de 1,7 g de carbonate de calcium.
Rendement : 83 %
RMN ¹H (CDCl3, 300 MHz) δ (ppm) : 7,96 (s, 1H), 7,20 (s, 1H), 4,75 (s, 2H), 3,92 (s, 3H), 3,88 (s, 3H)
HPLC : 96 %
SM : MH⁺ 275/277

### Stade 3 : 5-Bromo-4-formyl-2-méthoxy-benzoate de méthyle

Le produit (725 mg) est obtenu selon le procédé du stade 3 de la préparation 4, en utilisant 875 mg de l'alcool précédent en présence de 2,8 g d'oxyde de manganèse activé.
Rendement : 83 %
RMN ¹H (CDCl3, 300 MHz) δ (ppm) : 10,33 (s, 1H), 8,01 (s, 1H), 7,49 (s, 1H), 3,95 (s, 3H), 3,92 (s, 3H)
HPLC : 96 %
SM : MH⁺ 273/275

### Stade 4: 5-Bromo-4-((E)-2-tert-Batoxycarbonyl-vinyl)-2-méthoxy-benzoate de méthyle

Le produit (860 mg) est obtenu selon le procédé du stade 4 de la préparation 4, en utilisant 725 mg de l'aldéhyde précédent en présence de 688 µL de diéthylphosphonoacétate de tert-butyle et 293 mg de tert-butanolate de sodium.
Rendement estimé : 87 %
RMN ¹H (CDC13, 300 MHz) δ (ppm) : 8,00 (s, 1 H), 7,89 (d, 1H), 7,13 (s, 1H), 6,34 (d, 1H), 3,92 (s, 3H), 3,89 (s, 3H), 1,55 (s, 9H)
HPLC : 63 %
SM : MH⁺ 371/373

### Stade 5 : 5-Bromo-4-((E)-2-Carboxy-vinyl)-2-méthoxy-benzoate de méthyle

Le produit (489 mg) est obtenu selon le procédé du stade 5 de la préparation 4, en utilisant 860 mg de l'ester cinnamique précédent en présence de 5 mL d'acide trifluoroacétique.
Rendement : 67 %
RMN ¹H (DMSO, 300 MHz) δ (ppm) : 7,88 (s, 1H), 7,75 (d, 1H), 7,57 (s, 1H), 6,83 (d, 1H), 3,90 (s, 3H), 3,80 (s, 3H)
HPLC : 89 %
SM : MH⁺ 315/317

### Exemple 1 : Chlorhydrate d'acide 5-bromo-2-méthoxy-4-[N'-(4-méthoxy-phényl)-N'-(2-pipéridin-1-yl-phényl)-hydrazinocarbonylméthyl]-benzoïque (1)

### Stade 1 : (4-Méthoxy-phényl)-(2-nitro-phényl)-amine

A 3,7 mL de 2-fluoro-nitrobenzène sont ajoutés 8,73 g de 4-méthoxyaniline (2 équivalents). L'ensemble est chauffé à 110 °C pendant une nuit. Le milieu est repris dans l'acétate d'éthyle, lavé successivement avec de l'eau, une solution saturée de bicarbonate de sodium puis une solution saturée de chlorure de sodium. La phase organique est séchée sur sulfate de magnésium, filtrée puis concentrée sous pression réduite. Une chromatographie sur gel de silice du résidu (éther de pétrole puis éther de pétrole/acétate d'éthyle : 90/10) permet d'isoler 8,63 g du produit désiré.
Rendement : 99 %
RMN 1H (DMSO, 300 MHz) δ (ppm) : 9,35 (s, 1H), 8,10 (d, 1H), 7,45 (t, 1H), 7,25 (d, 2H), 6,98 (m, 3H), 6,79 (t, 1H), 3,78 (s, 3H)
HPLC : 100 %

### Stade 2 : N-(4-Méthoxy-phényl)-benzène-1,2-diamine

A une solution de 4 g du produit obtenu au stade précédent dans 80 mL d'éthanol sont additionnés 18,5 g de chlorure d'étain hydraté (5 équivalents). L'ensemble est porté à reflux pendant 5 h puis agité à température ambiante pendant une nuit. Le milieu est hydrolysé à froid, ajusté à pH8 à l'aide d'une solution de bicarbonate de sodium et extrait plusieurs fois à l'acétate d'éthyle. Les phases organiques réunies sont séchées sur sulfate de magnésium, filtrées et évaporées sous vide. Le résidu obtenu est purifié par chromatographie sur gel de silice (toluène puis toluène/acétate d'éthyle : 95/5) pour conduire à 2,9 g du produit attendu.
Rendement : 82 %
RMN 1H (DMSO, 300 MHz) δ (ppm) : 6,89 (d, 1H), 6,75 (m, 7H), 6,50 (t, 1H), 4,67 (s, 2H), 3,67 (s, 3H)
HPLC : 100 %

### Stade 3 : (4-Méthoxy-phényl)-(2-pipéridin-1-yl-phényl)-amine

A une solution de 2,5 g d'aniline obtenue précédemment dans 15 mL de toluène, sont ajoutés successivement 2,47 g de carbonate de sodium (2 équivalents) et 1,6 mL de dibromopentane (1 équivalent). L'ensemble est porté à reflux pendant 24 h. Après retour à température ambiante, le carbonate de sodium est éliminé par filtration et rincé au dichlorométhane. Le filtrat est évaporé sous pression réduite, le résidu obtenu est purifié par chromatographie sur gel de silice (éther de pétrole, éther de pétrole/acétate d'éthyle : 98/2) pour conduire à 1,72 g du produit désiré.
Rendement : 52 %
RMN 1H (CDCl3, 300 MHz) δ (ppm) : 7,10 (m, 4H), 6,92 (m, 3H), 6,78 (m, 1H), 6,52 (s, 1H), 3,81 (s, 3H), 2,86 (m, 4H), 1,71 (m, 4H), 1,60 (m, 2H)
HPLC: 100 %

### Stade 4 : N-(4-Méthoxy-phényl)-N-(2-pipéridin-1-yl-phényl)-hydrazine (a)

806 mg de produit obtenu au stade précédent sont solubilisés dans 10 mL d'acide acétique. A cette solution refroidie entre 5 et 15 °C est additionnée goutte à goutte une solution de 1,14 g de nitrite de sodium dans 3 mL d'eau (5,8 équivalents). Après 10 minutes d'agitation, de la glace est ajoutée dans le milieu réactionnel, le précipité formé est filtré, lavé à l'eau puis séché sous vide. Une poudre marron correspondant à l'intermédiaire nitroso est obtenue (suivi par CCM et RMN).
Cet intermédiaire non purifié, repris dans 8 mL de diéthyl éther, est directement refroidi à 10 °C. A cette solution est ajoutée une suspension de 119 mg d'hydrure de lithium et d'aluminium (1,1 équivalents) dans 2 mL de diéthyl éther. L'ensemble est agité pendant 1 h jusqu'à disparition complète du produit de départ (suivi CCM). Le milieu réactionnel est versé sur une solution de soude 1 N et extrait plusieurs fois au diéthyl éther. La phase organique est séchée, filtrée et évaporée sous pression réduite pour conduire à un résidu qui est purifié par chromatographie (éther de pétrole/acétate d'éthyle : 95/5). 382 mg de produit correspondant à l'hydrazine sont obtenus sous forme d'huile rose.
Rendement : 45 %
RMN ¹H (CDCl3, 300 MHz) δ (ppm) : 7,08 (m, 5H), 6,95 (m, 1H), 6,81 (m, 2H), 3,78 (s, 3H), 3,04 (m, 4H), 1,73 (m, 4H), 1,57 (m, 2H)

### Stade 5 : 5-Bromo-2-méthoxy-4-[N'-(4-méthoxy-phényl)-N'-(2-pipéridin-1-yl-phényl)-hydrazinocarbonylméthyl]-benzoate de méthyle

A une solution de 367 mg du composé précédent dans 10 mL de dichlorométhane sont ajoutés 412 mg de l'acide de la préparation 2 (1,1 équivalents), 260 mg d'EDCI (1,1 équivalents) et 45 mg de DMAP (0,3 équivalent). Le milieu réactionnel est agité à température ambiante, et si besoin chauffé, jusqu'à complète disparition de l'hydrazine de départ (temps > 15h), puis hydrolysé et extrait au dichlorométhane plusieurs fois. Les phases organiques sont lavées avec une solution de soude 1 N puis une solution saturée de chlorure de sodium, séchées sur sulfate de magnésium, filtrées et évaporées sous pression réduite. Le résidu obtenu est purifié par chromatographie sur gel de silice (éther de pétrole/acétate d'éthyle : 72/25 puis 70/30) pour conduire à 546 mg du produit attendu.
Rendement : 76 %
RMN ¹H (DMSO, 300 MHz) δ (ppm) : 10,65 (s, 1H), 7,81 (s, 1H), 7,30 (m, 2H), 6,99 (m, 4H), 6,72 (m, 4H), 3,79 (2s élargis, 8H), 3,69 (s, 3H), 2,50 (s élargi, 4H), 1,12 (m, 6H)
HPLC : 96 %

### Stade 6 : Chlorhydrate d'acide 5-bromo-2-méthoxy-4-[N'-(4-méthoxy-phényl)-N'-(2-pipéridin-1-yl-phényl)-hydrazinocarbonylméthyl]-benzoïque (1)

A une solution de 100 mg du produit obtenu au stade 5 dans 2 mL de dioxane sont ajoutés 343 µL d'une solution de soude 1 N (2 équivalents). Le milieu réactionnel est agité à température ambiante pendant 4 h puis concentré sous pression réduite. Le solide obtenu est repris dans un minimum d'eau et acidifié avec une solution d'acide chlorhydrique 1 N jusqu'à pH1. Dans le cas présent, une extraction au dichlorométhane a permis d'isoler le brut réactionnel. Après évaporation, le résidu est repris dans l'éther. Il se forme un précipité qui est filtré et lavé pour conduire à 52 mg du produit attendu sous forme de chlorhydrate.
Rendement : 50 %
PF : 109 °C (décomposition)
Analyse élémentaire calculée pour C28H30BrN3O5. 1HCl. 1,5H2O : C, 53,22 ; H, 5,42 ; N, 6,65. Trouvée : C, 52,84 ; H, 5,08 ; N, 6,24.
HPLC : 97 %
SM : MH⁺ 568/570

### Exemple 2 : Chlorhydrate d'acide 5-bromo-2-méthoxy-4[N'-(2-pipéridin-1-yl-phényl)-N'-(4-trifluorométhoxy-phényl)-hydrazinocarbonylméthyl]-benzoïque (2)

### Stade 1 : (2-Nitro-phényl)-(4-trifluorométhoxy-phényl)-amine

A 374 µL de 2-fluoro-nitrobenzène dans 2 mL de DMSO sont ajoutées 962 µL de 4-trifluorométhoxyaniline (2 équivalents) et 636 mg de tert-butanolate de potassium (1,6 équivalents). L'ensemble est chauffé à 110 °C pendant 3 heures. Une fois la réaction terminée, le milieu repris dans un minimum de dichlorométhane, est hydrolysé puis extrait. La phase organique est lavée successivement avec de l'eau puis une solution saturée de chlorure de sodium, séchée sur sulfate de magnésium, filtrée et concentrée sous pression réduite. Une chromatographie sur gel de silice du résidu (cyclohexane puis cyclohexane/acétate d'éthyle : 99/1) permet d'isoler 575 mg du produit désiré.
Rendement : 54 %
RMN 1H (CDCl3, 300 MHz) δ (ppm) : 9,43 (s, 1H), 8,22 (d, 1H), 7,43 (t, 1H), 7,25-7,38 (m, 4H), 7,19 (d, 1H), 6,83 (t, 3H)
HPLC : 97 %
SM : MH⁺ 299

### Stade 2 : N-(4-Trifluorométhoxy-phényl)-benzène-1,2-diamine

Le produit (600 mg) est obtenu selon le procédé du stade 2 de l'exemple 1, en utilisant 570 mg du dérivé précédent comme produit de départ et 2,16 g de chlorure d'étain hydraté dans 10 mL d'éthanol.
Rendement : quantitatif
RMN 1H (CDCl3, 300 MHz) δ (ppm) : 7,01-7,12 (m, 4H), 6,73-6,86 (m, 2H), 6,70 (m, 2H), 5,30 (s élargi, 1H)
HPLC : 96 %
SM : MH⁺ 269

### Stade 3 : (2-Pipéridin-1-yl-phényl)-(4-trifluorométhoxy-phényl)-amine

Le produit (350 mg) est obtenu selon le procédé du stade 3 de l'exemple 1, en utilisant 513 mg de l'aniline précédente comme substrat et 261 µL de dibromopentane en présence de 405 mg de carbonate de sodium dans 5 mL de toluène.
Rendement : 54 %
RMN 1H (CDCl3, 300 MHz) δ (ppm) : 7,28 (m, 2H), 7,13 (s, 3H), 7,09 (d, 1H), 7,01 (t, 1H), 6,88 (t, 1H), 6,70 (s, 1H), 2,83 (m, 4H), 1,71 (m, 4H), 1,59 (m, 2H)

### Stade 4 : N-(2-Pipéridin-1-yl-phényl)-N-(4-trifluorométhoxy-phényl)-hydrazine (b)

Le produit (180 mg) est obtenu selon le procédé du stade 4 de l'exemple 1, en utilisant 345 mg du dérivé précédent comme produit de départ et 410 mg de nitrite de sodium dans 4 mL d'acide acétique conduisant à l'intermédiaire nitroso qui est réduit par 28 mg d'hydrure de lithium et d'aluminium dans 4 mL de diéthyl éther.
Rendement : 50 %
RMN ¹H (CDCl3, 300 MHz) δ (ppm) : 7,04-7,22 (massif aromatique, 8H), 3,09 (m, 4H), 1,83 (m, 4H), 1,59 (m, 2H)
SM : MH⁺ 352

### Stade 5 : 5-Bromo-2-méthoxy-4[N'-(2-pipéridin-1-yl-phényl)-N'-(4-trifluorométhoxy-phényl)-hydrazinocarbonylméthyl]-benzoate de méthyle

Le produit (250 mg) est obtenu selon le procédé du stade 5 de l'exemple 1, en utilisant 176 mg de l'hydrazine précédente comme substrat et 167 mg de l'acide de la préparation 2 comme co-substrat en présence de 106 mg d'EDCI et 18 mg de DMAP.
Rendement : 78 %
RMN ¹H (CDCl3, 300 MHz) δ (ppm) : 9,32 et 9,35 (2s dans les proportions 70/30, 1H), 7,93 et 7,99 (2s dans les proportions 30/70, 1H), 6,74-7,50 (m, 9H), 3,70-4,07 (3s, 8H), 2,67 (m, 4H), 1,43-1,56 (m, 6H)
HPLC : 98 %
SM : MH⁺ 636/638

### Stade 6 : Chlorhydrate d'acide 5-bromo-2-méthoxy-4[N'-(2-pipéridin-1-yl-phényl)-N'-(4-trifluorométhoxy-phényl)-hydrazinocarbonylméthyl]-benzoïque (2)

A une solution de 100 mg du produit obtenu au stade 5 dans 2 mL de dioxane sont ajoutés 1,6 mL d'une solution de soude 1 N (10 équivalents). Le milieu réactionnel est agité à température ambiante pendant 4 h puis concentré sous pression réduite. Le solide obtenu est repris dans un minimum d'eau et acidifié avec une solution d'acide chlorhydrique 1 N jusqu'à pH1. Il se forme un précipité qui est filtré, repris dans l'éther et lavé pour conduire à 52 mg du produit attendu sous forme de chlorhydrate.
Rendement : 69 %
PF : 157 °C (décomposition)
Analyse élémentaire calculée pour C28H27BrF3N3O5.1HCl.1H2O: C, 49,68; H, 4,47 ; N, 6,21. Trouvée : C, 50,01 ; H, 4,62 ; N, 5,91.
HPLC : 98 %
SM : MH⁺ 622/624

### Exemple 3 : Chlorhydrate d'acide 5-bromo-2-méthoxy-4-[N'-(3-méthoxy-benzyl)-N'-(2-pipéridin-1-yl-phényl)-hydrazinocarbonylméthyl]-benzoïque (3)

### Stade 1 : (3-Méthoxy-benzyl)-(2-pipéridin-1-yl-phényl)-amine

A 1 g de 2-pipéridinoaniline dans 10 mL de DMF sont ajoutées 795 µL de bromure de 3-méthoxy benzyle (1 équivalent) et 1,57 g de carbonate de potassium (2 équivalents). L'ensemble est chauffé à 100 °C pendant 2 à 3 heures, jusqu'à disparition de l'aniline de départ. Le milieu est versé sur de la glace puis extrait à l'acétate d'éthyle. La phase organique lavée à l'eau est séchée sur sulfate de magnésium, filtrée et évaporée sous pression réduite. Une chromatographie sur gel de silice du résidu (cyclohexane/acétate d'éthyle : 99/1) permet d'isoler 1,46 g du produit désiré.
Rendement : 87 %
RMN 1H (CDCl3, 300 MHz) δ (ppm) : 7,26 (m, 1H), 6,99 (m, 4H), 6,81 (d, 1H), 6,68 (t, 1H), 6,57 (d, 1 H), 4,35 (s, 2H), 3,80 (s, 3H), 2,86 (s élargi, 4H), 1,69 (s élargi, 6 H) HPLC : 97 %
SM : MH⁺ 297

### Stade 2 : N-(3-Méthoxy-benzyl)-N-(2-pipéridin-1-yl-phényl)-hydrazine (c)

Selon le procédé du stade 4 de l'exemple 1, l'intermédiaire nitroso (376 mg) est obtenu par extraction à l'acétate d'éthyle du milieu réactionnel tamponné à pH7 en utilisant 353 mg du dérivé précédent comme produit de départ et 477 mg de nitrite de sodium dans 3 mL d'acide acétique. Une suspension de 180 mg d'hydrure de lithium et d'aluminium (4 équivalents) dans 5 mL de diéthyl éther appliquée à cet intermédiaire pendant 2h30 à reflux permet de conduire à 138 mg d'hydrazine après purification.
Rendement : 37 %
RMN ¹H (CDC13, 300 MHz) δ (ppm) : 7,26 (m, 1H), 6,91-7,11 (massif aromatique, 6H), 6,80 (dd, 1H), 4,56 (s, 2H), 3,80 (s, 3H), 3,12 (m, 4H), 1,59-1,79 (m, 6H)

### Stade 3 : 5-Bromo-2-méthoxy-4-[N'-(3-méthoxy-benzyl)-N'-(2-pipéridin-1-yl-phényl)-hydrazinocarbonylméthylJ-benzoate de méthyle

Le produit (183 mg) est obtenu selon le procédé du stade 5 de l'exemple 1, en utilisant 137 mg de l'hydrazine précédente comme substrat et 160 mg de l'acide de la préparation 2 comme co-substrat en présence de 101 mg d'EDCI et 16 mg de DMAP Rendement : 70 %
HPLC: 100 %
SM : MH⁺ 596/598

### Stade 4 : Chlorhydrate d'acide 5-bromo-2-méthoxy-4-[N'-(3-méthoxy-benzyl)-N'-(2-pipéridin-1-yl-phényl)-hydrazinocarbonylméthyl]-benzoïque (3)

Le produit (152 mg) est obtenu sous forme de chlorhydrate selon le procédé du stade 6 de l'exemple 2, en utilisant comme substrat le produit obtenu au stade précédent.
Rendement : 82 %
PF : 109-112 °C
Analyse élémentaire calculée pour C29H32BrN3O5.1HCl.1,5H2O : C, 53,92 ; H, 5,62 ; N, 6,50. Trouvée : C, 54,08 ; H, 5,67 ; N, 5,79.
HPLC : 100 %
SM : MH⁺ 582/584

### Exemple 4 : Chlorhydrate d'acide 4-[N'-(4-benzyloxy-phényl)-N'-(2-pipéridin-1-yl-phényl)-hydrazinocarbonylméthyl]-5-bromo-2-méthoxy-benzoïque (4)

### Stade 1 : (4-Benzyloxy-phényl)-(2-nitro-phényl)-amine

A 600 µL de 2-fluoro-nitrobenzène dans 6 mL de DMSO sont ajoutées 1,7 g de 4-(benzyloxy)aniline (1,5 équivalents) et 1,02 g de tert-butanolate de potassium (1,6 équivalents). L'ensemble est chauffé à 110 °C pendant 1 heure 30. Le milieu est ensuite hydrolysé et extrait plusieurs fois à l'acétate d'éthyle. Les phases organiques sont lavées avec une solution saturée de chlorure de sodium, séchées sur sulfate de magnésium, filtrées puis concentrées sous pression réduite. Une chromatographie sur gel de silice du résidu (cyclohexane/acétate d'éthyle : 99/1) permet d'isoler 754 mg du produit désiré.
Rendement : 41 %
RMN 1H (CDCl3, 300 MHz) δ (ppm) : 9,40 (s élargi, 1H), 8,18 (d, 1H), 7,23-7,45 (m, 6H), 7,17-7,21 (m, 2H), 7,05 (m, 3H), 6,71 (t, 1H), 5,09 (s, 2H)
SM : MH⁺ 321

### Stade 2 : N-(4-Benzyloxy-phényl)-benzène-1,2-diamine

Le produit (653 mg) est obtenu selon le procédé du stade 2 de l'exemple 1, en utilisant 754 mg du dérivé précédent comme produit de départ et 2,66 g de chlorure d'étain hydraté dans 12 mL d'éthanol.
Rendement : 95 %
RMN 1H (CDC13, 300 MHz) δ (ppm) : 6,68-7,37 (massif aromatique, 13H), 5,01 (s, 2H)
HPLC : 82 %
SM : MH⁺ 291

### Stade 3: (4-Benzyloxy-phényl)-(2-pipéridin-1-yl-phényl)-amine

A une solution de 653 mg d'aniline obtenue précédemment dans 4 mL de toluène, sont ajoutés successivement 940 µL de DIPEA (2,4 équivalents) et 305 µL de dibromopentane (1 équivalent). L'ensemble est porté à reflux pendant 2 h jusqu'à complète disparition de l'aniline de départ. Après retour à température ambiante, le milieu réactionnel est hydrolysé et extrait plusieurs fois à l'acétate d'éthyle. Les phases organiques sont lavées à l'eau, séchées sur sulfate de magnésium, filtrées et évaporées sous pression réduite. Le résidu obtenu est purifié par chromatographie sur gel de silice (cyclohexane puis cyclohexane/acétate d'éthyle : 99,5/0,5) pour conduire à 480 mg du produit désiré.
Rendement : 60 %
RMN 1H (CDCl3, 300 MHz) δ (ppm) : 7,31-7,41 (m, 5H), 7,05-7,15 (m, 4H), 6,95 (m, 3H), 6,78 (t, 3H), 6,53 (s élargi, 1H), 5,06 (s, 2H)
HPLC : 94 %
SM : MH⁺ 359

### Stade 4: N-(4-Benzyloxy-phényl)-N-(2-pipéridin-1-yl-phényl)-hydrazine (d)

Le produit (202 mg) est obtenu selon le procédé du stade 4 de l'exemple 1, en utilisant 488 mg du dérivé précédent comme produit de départ et 545 mg de nitrite de sodium dans 5 mL d'acide acétique conduisant à l'intermédiaire nitroso qui est réduit par 74 mg d'hydrure de lithium et d'aluminium (2 équivalents) dans 5 mL de diéthyl éther.
Rendement : 40 %
RMN ¹H (CDCl3, 300 MHz) δ (ppm) : 6,73-7,40 (massif aromatique, 13H), 4,94 (s, 2H), 2,90 (m, 4H), 1,61 (m, 4H), 1,43 et 1,50 (m, 2H)

### Stade 5 : 4-[N'-(4-Benzyloxy-phényl)-N'-(2-pipéridin-1-yl-phényl)-hydrazinocarbonyl méthyl]-5-bromo-2-méthoxy-benzoate de méthyle

Le produit (193 mg) est obtenu selon le procédé du stade 5 de l'exemple 1, en utilisant 202 mg de l'hydrazine précédente comme substrat et 180 mg de l'acide de la préparation 2 comme co-substrat en présence de 114 mg d'EDCI et 20 mg de DMAP.
Rendement : 54 %
RMN ¹H (CDCl3, 300 MHz) δ (ppm) : 9,37 (s élargi, 1H), 7,98 (d, 1H), 7,30-7,37 (m, 5H), 7,05-7,30 (m, 2H), 6,70-6,84 (m, 5H), 4,99 (d, 2H), 3,68-4,08 (3s, 8H), 2,66 (m, 4H), 1,43-1,63 (m, 6H)

### Stade 6 : Chlorhydrate d'acide 4-[N'-(4-benzyloxy-phényl)-N'-(2-pipéridin-1-yl-phényl)-hydrazinocarbonylméthyl]-5-bromo-2-methoxy-benzoïque (4)

Le produit est obtenu selon le procédé du stade 6 de l'exemple 2, en utilisant comme substrat le produit obtenu au stade précédent. Une purification par chromatographie en phase inverse (conditions: colonne C18, 21,2x150 mm, mode isochratique 30% acétonitrile/H2O + 0,05% TFA, débit : 15 mL/min, longueurs d'onde : 220 et 254 nm) suivie d'un traitement par une solution 1 N d'acide chlorhydrique a permis d'isoler le produit attendu sous forme de chlorhydrate (40 mg).
Rendement : 21 %
PF : 114 °C
HPLC : 98 %
SM : MH⁺ 644/646

### Exemple 5 : Chlorhydrate d'acide 5-bromo-4-{N'-[4-(4-fluoro-phénoxy)-phényl]-N'-(2-pipéridin-1-yl-phényl)-hydrazinocarbonylméthyl}-2-méthoxy-benzoïque (5)

### Stade 1 : 1-Nitro-4-(4-fluoro-phénoxy)-benzène

A une solution de 1,5 mL de 4-fluoronitrobenzène dans 28 mL de diméthylformamide sont ajoutés successivement 1,75 g de 4-fluoronitrobenzène (1,1 équivalents) et 2,15 g de carbonate de potassium (1,1 équivalents). L'ensemble est chauffé à 150 °C pendant 4h30. Après retour à température ambiante, le milieu est versé sur de la glace et mis sous agitation pendant 30 min. Il se forme un précipité qui est filtré, rincé à l'eau puis séché (3,02 g).
Rendement : 91 %
RMN ¹H (CDC13, 300 MHz) δ (ppm) : 8,20 (d, 2H), 6,97-7,16 (massif aromatique, 6H)

### Stade 2 : 4-(4-Fluoro-phénoxy)phénylamine

A une solution de 3,02 g du produit obtenu au stade précédent dans 50 mL d'éthanol sont additionnés 14,6 g de chlorure d'étain hydraté (5 équivalents). L'ensemble est porté à reflux pendant 1 h. Après retour à température ambiante, le milieu est versé sur de la glace, basifié à pH10 à l'aide d'une solution de soude 4 N et extrait plusieurs fois à l'acétate d'éthyle. Les phases organiques réunies sont séchées sur sulfate de magnésium, filtrées et évaporées sous pression réduite pour conduire à 2,6 g du produit attendu.
Rendement : quantitatif
RMN 1H (CDCl3, 300 MHz) δ (ppni) : 6,82-7,03 (massif aromatique, 6H), 6,71-6,79 (m, 2H)
SM : MH⁺ 204

### Stade 3 : [4-(4-Fluoro-phénoxy)-phényl]-(2-nitro-phényl)-amine

Le produit (790 mg) est obtenu selon le procédé du stade 1 de l'exemple 4, en utilisant 900 µL de 2-fluoro-nitrobenzène et 2,6 g de produit obtenu au stade précédent en présence de 1,53 g de tert-butanolate de potassium dans 9 mL de DMSO.
Rendement : 28 %
RMN 1H (CDCl3, 300 MHz) δ (ppm) : 9,46 (s élargi, 1H), 8,25 (d, 1H), 7,42 (t, 1H), 7,28 (m, 2H), 7,05-7,19 (massif aromatique, 7H), 6,77 (t, 1H)
HPLC : 92 %
SM : MH⁺ 325

### Stade 4 : N-[4-(4-Fluoro-phénoxy)-phényl]-benzène-1,2-diamine

Le produit (637 mg) est obtenu selon le procédé du stade 2 de l'exemple 1, en utilisant 750 mg du dérivé précédent comme produit de départ et 2,61 g de chlorure d'étain hydraté dans 12 mL d'éthanol.
Rendement : 93 %
RMN 1H (CDCl3, 300 MHz) δ (ppm) : 6,17-7,13 (massif aromatique, 12H)
HPLC : 92 %
SM : MH⁺ 295

### Stade 5 : [4-(4-Fluoro-phénoxy)-phényl]-(2-pipéridin-1-yl-phényl)-amine

Le produit (715 mg) est obtenu selon le procédé du stade 3 de l'exemple 4, en utilisant 635 mg du dérivé précédent comme produit de départ, 294 µL de dibromopentane et 905 µL de DIPEA dans 4 mL de toluène.
Rendement : 91 %
RMN 1H (CDCl3, 300 MHz) δ (ppm): 6,79-7,26 (massif aromatique, 12H), 6,63 (s élargi, 1H), 2,84 (m, 4H), 1,74 (m, 4H), 1,60 (m, 2H)
HPLC : 98 %
SM : MH⁺ 363

### Stade 6 : N-[4-(4-Fluoro-phénoxy)-phényl]-N-(2-pipéridin-1-yl-phényl)-hydrazine (e)

Le produit (275 mg) est obtenu selon le procédé du stade 4 de l'exemple 1, en utilisant 715 mg du dérivé précédent comme produit de départ et 791 mg de nitrite de sodium dans 7 mL d'acide acétique conduisant à l'intermédiaire nitroso qui est réduit par 150 mg d'hydrure de lithium et d'aluminium (2 équivalents) dans 7 mL de diéthyl éther.
Rendement : 37 %
RMN ¹H (CDCl3, 300 MHz) δ (ppm) : 6,83-7,19 (massif aromatique, 12H), 3,00 (m, 4H), 1,73 (m, 4H), 1,58 (m, 2H)

### Stade 7 : 5-Bromo-4-{N'-[4-(4-fluoro-phénoxy)-phényl]-N'-(2-pipéridin-1-yl-phényl)-hydrazinocarbonylméthyl}-2-méthoxy-benzoate de méthyle

Le produit (336 mg) est obtenu selon le procédé du stade 5 de l'exemple 1, en utilisant 275 mg de l'hydrazine précédente comme substrat et 243 mg de l'acide de la préparation 2 comme co-substrat en présence de 154 mg d'EDCI et 27 mg de DMAP Rendement : 69 %
RMN ¹H (CDCl3, 300 MHz) δ (ppm): 9,35 (s élargi, 1H), 7,98 (d, 1H), 6,73-7,50 (massif aromatique, 13H), 3,69-4,13 (3s, 8H), 2,72 (m, 4H), 1,46-1,60 (m, 6H)
HPLC : 95 %
SM : MH⁺ 662/664

### Stade 8 : Chlorhydrate d'acide 5-bromo-4-{N'-[4-(4-fluoro-phénoxy)-phényl]-N'-(2-pipéridin-1-yl-phényl)-hydrazinocarbonylméthyl}-2-méthoxy-benzoïque (5)

Le produit est obtenu selon le procédé du stade 6 de l'exemple 2, en utilisant comme substrat le produit obtenu au stade précédent. Une purification par chromatographie en phase inverse d'une fraction (conditions : colonne C18, 21,2x150 mm, mode isochratique 30% acétonitrile/H2O + 0,05% TFA, débit : 15 mL/min, longueurs d'onde : 220 et 254 nm) suivie d'un traitement par une solution 1 N d'acide chlorhydrique a permis d'isoler ie produit attendu sous forme de chlorhydrate (43 mg).
Rendement estimé : 44 %
PF : 224-231 °C
Analyse élémentaire calculée pour C33H31BrFN3O5.1HCl.1H2O : C, 56,38 ; H, 4,88 ; N, 5,98. Trouvée : C, 56,28 ; H, 4,91 ; N, 5,78.
HPLC : 98 %
SM : MH⁺ 648/650

### Exemple 6: Chlorhydrate d'acide 5-bromo-2-méthoxy-4-{N'-[2-(4-méthoxy-phényl)-éthyl]-N'-(2-pipéridin-1-yl-phényl)-hydrazinocarbonylméthyl}-benzoïque (6)

### Stade 1 : [2-(4-Méthoxy-phényl)-éthyl]-(2-pipéridin-1-yl-phényl)-amine

A 1 g de 2-pipéridinoaniline dans 10 mL de DMF sont ajoutées 1,72 mL de chlorure de 4-méthoxyphenéthyle (2 équivalents) et 2,35 g de carbonate de potassium (3 équivalents). L'ensemble est chauffé à 100 °C pendant 60 heures puis versé sur de la glace et extrait à l'acétate d'éthyle. La phase organique lavée avec une solution saturée de chlorure de sodium est séchée sur sulfate de magnésium, filtrée et évaporée sous pression réduite. Plusieurs chromatographies sur gel de silice du résidu (éther de pétrole, éther de pétrole/acétate d'éthyle : 98/2) permettent d'isoler 255 mg du produit désiré.
Rendement : 14 %
RMN 1H (CDCl3, 300 MHz) δ (ppm) : 7,17 (d, 2H), 6,99 (m, 2H), 6,86 (m, 2H), 6,66 (m, 2H), 4,77 (s élargi, 1H), 3,80 (s, 3H), 3,36 (t, 2H), 2,90 (t, 2H), 2,72 (s élargi, 4H), 1,57 (m, 6H)

### Stade 2 : N-[2-(4-Méthoxy-phényl)-éthyl]-N-(2-pipéridin-1-yl-phényl)-hydrazine (f)

Le produit (160 mg) est obtenu selon le procédé du stade 4 de l'exemple 1, en utilisant 250 mg du dérivé précédent comme produit de départ et 322 mg de nitrite de sodium dans 3 mL d'acide acétique conduisant à l'intermédiaire nitroso qui est réduit par 103 mg d'hydrure de lithium et d'aluminium (4 équivalents) dans 3 mL de tétrahydrofurane à reflux.
Rendement : 61 %
RMN ¹H (CDC13, 300 MHz) δ (ppm) : 6,80-7,38 (massif aromatique, 8H), 3,77 (s, 3H), 3,42 (t, 2H), 3,08 (t, 2H), 2,93 (m, 4H), 1,55-1,70 (m, 6H)
HPLC : 88 %
SM : MH⁺ 326

### Stade 3 : 5-Bromo-2-méthoxy-4-{N'-[2-(4-méthoxy-phényl)-éthyl]-N'-(2-pipéridin-1-yl-phényl)-hydrazinocarbonylméthyl}-benzoate de méthyle

A une solution de 160 mg du composé précédent dans 3 mL de diméthylformamide sont ajoutés 164 mg de l'acide de la préparation 2 (1,1 équivalents), 104 mg d'EDCI (1,1 équivalents) et 73 mg de HOBt (1,1 équivalents). Le milieu réactionnel est agité à température ambiante pendant 30 min puis chauffé à 40°C pendant 1h30. Le brut de réaction est versé sur de la glace et extrait à l'acétate d'éthyle plusieurs fois. Les phases organiques réunies sont séchées sur sulfate de magnésium, filtrées et évaporées sous pression réduite. Une première chromatographie sur gel de silice du résidu (éther de pétrole/acétate d'éthyle : 95/5, 80/20 puis 50/50) permet d'isoler le produit qui est ensuite purifié par chromatographie en phase inverse (conditions : colonne C18, 21,2x150 mm, mode isochratique 30% acétonitrile/H2O + 0,05% TFA, débit : 15 mL/min, longueurs d'onde : 220 et 254 nm) pour conduire à 90 mg du produit désiré sous forme de sel de TFA.
Rendement : 25 %
RMN ¹H (DMSO, 300 MHz) δ (ppm) : 12,15 (s élargi, 1H), 11,53 (s, 1H), 8,06 (d, 1H), 7,92 (s, 1H), 7,54 (t, 1H), 7,42 (m, 1 H), 7,32 (d, 1H), 7,03 (d, 3H); 6,76 (d, 2H), 3,86 et 3,83 (2s, 10H), 3,73 (s, 2H), 3,47 (s, 3H), 2,98 (m, 2H), 2,85 (t, 2H), 1,79 (m, 4H), 1,34 (m, 2H)
HPLC : 99 %
SM : MH⁺ 610/612

### Stade 4 : Chlorhydrate d'acide 5-bromo-2-méthoxy-4-{N'-[2-(4-méthoxy-phényl)-éthyl]-N'-(2-pipéridin-1-yl-phényl)-hydrazinocarbonylméthyl}-benzoïque (6)

Le produit (63 mg) est obtenu sous forme de chlorhydrate selon le procédé du stade 6 de l'exemple 2, en utilisant comme substrat le produit obtenu au stade précédent.
Rendement : 68 %
PF : 162-176 °C
Analyse élémentaire calculée pour C30H34BrN3O5.1HCl.1,5H2O : C, 54,59 ; H, 5,80 ; N, 6, 37. Trouvée : C, 54,65 ; H, 5,31 ; N, 5,85.
HPLC : 90 %
SM : MH⁺ 596/598

### Exemple 7: Chlorhydrate d'acide 5-bromo-2-méthoxy-4-[N'-(4-méthoxy-phényl)-N'-(2-méthyl-6-pipéridin-1-yl-phényl)-hydrazinocarbonylméthyl]-benzoïque (7)

### Stade 1 : (4-Méthoxy-phényl)-(2-méthyl-6-nitro-phényl)-amine

Le produit (487 mg) est obtenu selon le procédé du stade 1 de l'exemple 4, en utilisant 1 g de 2-fluoro-3-méthyl-nitrobenzène et 1,19 g de 4-méthoxy-aniline en présence de 1,16 g de tert-butanolate de potassium dans 9 mL de DMSO.
Rendement : 29 %
RMN 1H (CDCl3, 300 MHz) δ (ppm) : 7,99 (d, 1H), 7,37 (m, 1H), 6,96 (t, 1H), 6,78 (m, 4H), 3,78 (s, 3H), 1,99 (s, 3H)

### Stade 2 : N²-(4-Méthoxy-phényl)-3-méthyl-benzène-1,2-diamine

Le produit (428 mg) est obtenu selon le procédé du stade 2 de l'exemple 1, en utilisant 487 mg du dérivé précédent comme produit de départ et 2,13 g de chlorure d'étain hydraté dans 6 mL d'éthanol.
Rendement : 99 %
RMN 1H (CDC13, 300 MHz) δ (ppm) : 6,99 (t, 1H), 6,75 (m, 2H), 6,65 (d, 2H), 6,52 (m, 2H), 3,74 (s, 3H), 2,17 (s, 3H)

### Stade 3 : (4-Méthoxy-phényl)-(2-méthyl-6-pipéridin-1-yl-phényl)-amine

Le produit (250 mg) est obtenu selon le procédé du stade 3 de l'exemple 4, en utilisant 425 mg du dérivé précédent comme produit de départ, 253 µL de dibromopentane et 778 µL de DIPEA dans 8 mL de toluène.
Rendement : 45 %
RMN 1H (CDCl3, 300 MHz) δ (ppm) : 6,95 (m, 3H), 6,78 (m, 2H), 6,67 (m, 2H), 3,77 (s, 3H), 2,73 (m, 4H), 2,10 (s, 3H), 1,59 (m, 6H)

### Stade 4 : N-(4-Méthoxy-phényl)-N-(2-méthyl-6-pipéridin-1-yl-phényl)-hydrazine (g)

Selon le procédé du stade 4 de l'exemple 1, l'intermédiaire nitroso (270 mg) est obtenu par extraction au dichlorométhane du milieu réactionnel tamponné à pH7 en utilisant 250 mg du dérivé précédent comme produit de départ et 337 mg de nitrite de sodium dans 3 mL d'acide acétique. Une suspension de 126 mg d'hydrure de lithium et d'aluminium (4 équivalents) dans 3 mL de tétrahydrofurane appliquée à cet intermédiaire pendant 1 heure à reflux permet de conduire à 129 mg d'hydrazine après purification.
Rendement : 49 %
SM : MH⁺ 312

### Stade 5 : 5-Bromo-2-méthoxy-4-[N'-(4-méthoxy-phényl)-N'-(2-méthyl-6-pipéridin-1-yl-phényl)-hydrazinocarbonyméthyl]-benzoate de méthyle (7a)

A une solution de 129 mg de l'acide obtenu dans la préparation 2 (1,1 équivalents) dans 4 mL de dichlorométhane sont ajoutés 100 µL de chlorure de thionyle (3,3 équivalents) et 1 goutte de diméthylformamide. L'ensemble est agité 1 h à température ambiante puis évaporé sous pression réduite. Au chlorure d'acide ainsi obtenu, repris dans 4 mL de toluène, sont ajoutés successivement une solution de 129 mg de l'hydrazine précédente dans 3 mL de toluène et 65 µL de triéthylamine (1,1 équivalents). Le milieu est chauffé 1 nuit à 40 °C puis après retour à température ambiante, hydrolysé et extrait plusieurs fois à l'acétate d'éthyle. Les phases organiques réunies sont lavées à l'eau, séchées sur sulfate de magnésium, filtrées et évaporées sous pression réduite. Le résidu obtenu est purifié par chromatographie en phase inverse (conditions : colonne C18, 21,2x150 mm, mode isochratique 35% acétonitrile/H2O + 0,05% TFA, débit : 15 mL/min, longueurs d'onde : 220 et 254 nm) pour conduire à 110 mg du produit attendu sous forme de sel de TFA.
Rendement : 37 %
RMN ¹H (CDCl3, 300 MHz) δ (ppm) : 12,52 (s élargi, 1H), 11,12 (s, 1H), 7,98 (s, 1H), 7,51 (m, 2H), 7,36 (d, 1H), 7,13 (s, 1H), 6,79 (d, 2H), 6,56 (d, 2H), 4,03 (m, 2H), 3,89 (s, 6H), 3,75 (s, 3H), 3,42 (m, 3H), 3,06 (m, 1H), 2,26 (s, 3H), 1,79-2,01 (m, 5H), 1,50 (m, 1H)
HPLC : 93 %
SM : MH⁺ 596/598

### Stade 6: Chlorhydrate d'acide 5-bromo-2-méthoxy-4-[N'-(4-méthoxy-phényl)-N'-(2-méthyl-6-pipéridin-1-yl-phényl)-hydrazinocarbonylméthyl]-benzoïque (7)

Le produit (61 mg) est obtenu sous forme de chlorhydrate selon le procédé du stade 6 de l'exemple 2, en utilisant comme substrat le produit obtenu au stade précédent.
Rendement : 53 %
PF: 155-168 °C
Analyse élémentaire calculée pour C29H32BrN3O5.1HCl.1,75H2O: C, 53,55 ; H, 5,66 ; N, 6, 46. Trouvée : C, 53,28 ; H, 5,69 ; N, 6,12.
HPLC: 100 %
SM : MH⁺ 582/584

### Exemple 8 : Chlorhydrate d'acide 5-bromo-2-méthoxy-4-[N'-(4-méthoxy-benzyl)-N'-(2-pipéridin-1-yl-phényl)-hydrazinocarbonylméthyl]-benzoïque (8)

### Stade 1 : (4-Méthoxy-benzyl)-(2-pipéridin-1-yl-phényl)-amine

Le produit (644 mg) est obtenu selon le procédé du stade 1 de l'exemple 3, en utilisant 1 g de 2-pipéridinoaniline, 982 µL de bromure de 4-méthoxy benzyle (1,2 équivalents) et 1,6 g de carbonate de potassium (2 équivalents) dans 10 mL de DMF.
Rendement : 40 %
RMN 1H (CDC13, 300 MHz) (δ (ppm) : 7,42 (d, 2H), 7,11 (m, 2H), 7,01 (d, 2H), 6,81 (td, 1H), 6,75 (dd, 1 H), 4,41 (s, 2H), 3,88 (s, 3H), 2,99 (s élargi, 4H), 1,82 (m, 6H) HPLC : 100 %
SM : MH⁺ 297

### Stade 2 : N-(4-Méthoxy-benzyl)-N-(2-pipéridin-1-yl-phényl)-hydrazine (h)

Le produit (275 mg) est obtenu selon le procédé du stade 4 de l'exemple 1, en utilisant 290 mg du dérivé précédent comme produit de départ et 392 mg de nitrite de sodium dans 3 mL d'acide acétique conduisant à l'intermédiaire nitroso qui est réduit par 140 mg d'hydrure de lithium et d'aluminium (4 équivalents) dans 4 mL de tétrahydrofurane à reflux.
Rendement : 90%
RMN ¹H (CDCl3, 300 MHz) δ (ppm) : 7,23 (m, 2H), 7,10 (m, 1H), 7,00 (m, 3H), 6,87 (m, 2H), 4,47 (s, 2H), 3,82 (s, 3H), 3,10 (m, 4H), 1,59-1,77 (m, 6H)
HPLC : 96%
SM : MH⁺ 312

### Stade 3 : 5-Bromo-2-méthoxy-4-[N'-(4-méthoxy-benzyl)-N'-(2-pipéridin-1-yl-phényl)-hydrazinocarbonylméthyl]-benzoate de méthyle

Le produit est obtenu selon le procédé du stade 5 de l'exemple 7, en utilisant 161 mg de l'acide de la préparation 2 et 116 µL de chlorure de thionyle en présence d'une goutte de diméthylformamide pour la formation du chlorure d'acide et 150 mg de l'hydrazine précédente en présence de 74 µL de triétylamine pour le couplage. Une purification par chromatographie en phase inverse (conditions : colonne C 18, 21,2x 150 mm, mode isochratique 35% acétonitrile/H2O + 0,05% TFA, débit: 15 mL/min, longueurs d'onde : 220 et 254 nm) a permis d'isoler 80 mg du produit attendu sous forme de sel de
TFA.
Rendement : 23 %
RMN ¹H (CDCl3, 300 MHz) δ (ppm) : 11.90 (s élargi), 10,57 (s, 1H), 8,13 (d, 1H), 7,87 (s, 1H), 7,63 (t, 1H), 7,48 (t, 1H), 7,34 (d, 1H), 7,12 (d, 2H), 6,76 (m, 3H), 4,47 (m, 2H), 3,89 (m, 1H), 3,78, 3,86 et 3,89 (3s, 9H), 3,64 (s, 2H), 2,95-3,15 (m, 3H), 1,93 et 2,09 (2m, 5H), 1,49 (m, 1H)
HPLC : 93 %
SM : MH⁺ 596/598

### Stade 4 : Chlorhydrate d'acide 5-bromo-2méthoxy-4-[N'-(4-méthoxy-benzyl)-N'-(2-pipéridin-1-yl-phényl)-hydrazinocarbonylméthyl]-benzoïque (8)

Le produit (36 mg) est obtenu sous forme de chlorhydrate selon le procédé du stade 6 de l'exemple 2, en utilisant comme substrat le produit obtenu au stade précédent.
Rendement : 43 %
PF : 140-171 °C
Analyse élémentaire calculée pour C29H32BrN3O5.1HCl.1,75H2O: C, 53,55 ; H, 5,66 ; N, 6, 46. Trouvée : C, 53,52 ; H, 5,58 ; N, 5,97.
HPLC : 98 %
SM : MH⁺ 582/584

### Exemple 9 : Chlorhydrate d'acide 5-bromo-4-[N'-(4-cyclohexyl-phényl)-N'-(2-pipéridin-1-yl-phényl)-hydrazinocarbonylméthyl]-2-méthoxy-benzoïque (9)

### Stade 1 : (4-Cyclohexyl-phényl)-(2-nitro-phényl)-amine

Le produit (280 mg) est obtenu selon le procédé du stade 1 de l'exemple 4, en utilisant 400µL de 2-fluoro-nitrobenzène et 1 g de 4-cyclohexylaniline en présence de 683 mg de tert-butanolate de potassium dans 4 mL de DMSO.
Rendement : 25 %
RMN 1H (CDCl3, 300 MHz) δ (ppm) : 9,47 (s élargi, 1H), 8,19 (d, 1H), 7,71 (d, 1H), 7,45 (t, 1H), 7,34 (t, 1H), 7,10-7,24 (m, 3H), 6,71 (t, 1H), 2,53 (m, 1H), 1,35-1,95 (2m, 10H)
HPLC : 87 %
SM : MH⁺ 297

### Stade 2 : N-(4-Cyclohexyl-phényl)-benzène-1,2-diamine

Le produit (154 mg) est obtenu selon le procédé du stade 2 de l'exemple 1, en utilisant 280 mg du dérivé précédent comme produit de départ et 1,07 g de chlorure d'étain hydraté dans 4 mL d'éthanol.
Rendement : 61 %
RMN 1H (CDCl3, 300 MHz) δ (ppm) : 6,95-7,12 (m, 4H), 6,68-6,82 (m, 4H), 2,40 (m, 1H), 1,40 et 1,80 (2m, 10H)
HPLC : 91 %
SM : MH⁺ 267

### Stade 3 : (4-Cyclohexyl-phényl)-(2-pipéridin-1-yl-phényl)-amine

Le produit (395 mg) est obtenu selon le procédé du stade 3 de l'exemple 4, en utilisant 700 mg du dérivé précédent comme produit de départ, 357 µL de dibromopentane et 1,1 mL de DIPEA dans 10 mL de toluène.
Rendement : 45 %
RMN 1H (CDCl3, 300 MHz) δ (ppm) : 7,30 (m,1H), 7,05-7,16 (m, 5H), 6,97 (t, 1H), 6,80 (t, 1H), 6,64 (s élargi, 1H), 2,82 (m, 4H), 2,45 (m, 1H), 1,35-1,90 (m, 16H)

### Stade 4 : N-(4-Cyclohexyl-phényl)-N-(2-pipéridin-1-yl-phényl)-hydrazine (i)

Le produit (80 mg) est obtenu selon le procédé du stade 4 de l'exemple 1, en utilisant 200 mg du dérivé précédent comme produit de départ et 240 mg de nitrite de sodium dans 3 mL d'acide acétique conduisant à l'intermédiaire nitroso qui est réduit par 181 mg d'hydrure de lithium et d'aluminium (8 équivalents) dans 3 mL de tétrahydrofurane à reflux.
Rendement : 38%
RMN ¹H (CDCl3, 300 MHz) δ (ppm) : 6,75-7,25 (massif aromatique, 8H), 4,75 (s élargi), 2,97 (m, 4H), 2,42 (m, 1H), 1,25-1,90 (massifaliphatique, 16H)
SM : MH⁺ 350

### Stade 5 : 5-Bromo-4-[N'-(4-cyclohexyl-phényl)-N'-(2-pipéridin-1-yl-phényl)-hydrazino carbonylméthyl]-2-méthoxy-benzoate de méthyle

Le produit est obtenu selon le procédé du stade 3 de l'exemple 6, en utilisant 70 mg de l'hydrazine précédente et 67 mg de l'acide de la préparation 2 en présence de 42 mg d'EDCI et 30 mg d'HOBt dans 3 mL de diméthylformamide. Une purification par chromatographie en phase inverse (conditions : colonne C 18, 21,2x 150 mm, mode isochratique 50% acétonitrile/H2O + 0,05% TFA, débit: 15 mL/min, longueurs d'onde : 220 et 254 nm) a permis d'isoler 32 mg du produit attendu sous forme de sel de TFA.
Rendement : 21 %
HPLC : 93 %
SM : MH⁺ 634/636

### Stade 6 : Chlorhydrate d'acide 5-bromo-4-[N'-(4-cyclohexyl-phényl)-N'-(2-pipéridin-1-yl-phényl)-hydrazinocarbonylméthyl]-2-méthoxy-benzoïque (9)

Le produit (22 mg) est obtenu sous forme de chlorhydrate selon le procédé du stade 6 de l'exemple 2, en utilisant comme substrat le produit obtenu au stade précédent.
Rendement : 68 %
PF : 163-181 °C
Analyse élémentaire calculée pour C33H38BrN3O4.1HCl.0,75H2O: C, 59,11 ; H, 6,09 ; N, 6, 27. Trouvée : C, 59,23 ; H, 6,25 ; N, 6,03.
HPLC : 93 %
SM : MH⁺ 620/622

### Exemple 10 : Chlorhydrate d'acide 5-bromo-2-méthoxy-4-[N'-(2-méthyl-6-pipéridin-1-yl-phényl)-N'-(4-trifluorométhoxy-phényl)-hydrazinocarbonylméthyl]-benzoïque (10)

### Stade 1 : (2-Méthyl-6-nitro-phényl)-(4-trifluorométhoxy-phényl)-amine

Le produit (1,92 g) est obtenu selon le procédé du stade 1 de l'exemple 4, en utilisant 1 g de 2-fluoro-3-méthyl-nitrobenzène et 1,3 mL de 4-trifluorométhoxy-aniline en présence de 1,16 g de tert-butanolate de potassium dans 10 mL de DMSO.
Rendement : 95 %
RMN 1H (CDCl3, 300 MHz) δ (ppm) : 8,19 (s élargi), 7,97 (d, 1H), 7,45 (d, 1H), 7,10 (m, 3H), 6,74 (d, 2H), 2,11 (s, 3H)
HPLC : 75 %
SM: MH⁺ 313

### Stade 2 : 3-Méthyl-N²-(4-trifluorométhoxy-phényl)-benzène-1,2-diamine

Le produit (901 mg) est obtenu selon le procédé du stade 2 de l'exemple 1, en utilisant 1,92 g du dérivé précédent comme produit de départ et 6,94 g de chlorure d'étain hydraté dans 25 mL d'éthanol.
Rendement : 52 %
RMN 1H (CDCl3, 300 MHz) δ (ppm) : 7,07 (m, 3H), 6,73 (m, 2H), 6,53 (d, 2H), 5,10 (s élargi, 1 H), 3,99 (s élargi, 2H), 2,18 (s, 3H)
HPLC : 90 %
SM : MH⁺ 283

### Stade 3 : (2-Méthyl-6-pipéridin-1-yl-phényl)-(4-trifluorométhoxy-phényl)-amine

Le produit (890 mg) est obtenu selon le procédé du stade 3 de l'exemple 4, en utilisant 900 mg du dérivé précédent comme produit de départ, 433 µL de dibromopentane et 1,3 mL de DIPEA dans 15 mL de toluène.
Rendement : 79 %
RMN 1 H (CDCl3, 300 MHz) δ (ppm) : 7,04 (m, 3H), 6,95 (d, 2H), 6,66 (d, 2H), 6,19 (s élargi, 1H), 2,72 (m, 4H), 2,11 (s, 3H), 1,56 (m, 6H)
HPLC : 96 %
SM : MH⁺ 351 1

### Stade 4: N-(2-Méthyl-6-pipéridin-1-yl-phényl)-N-(4-trifluorométhoxy-phényl)-hydrazine (j)

Le produit (270 mg) - contaminé par l'amine de départ - est obtenu selon le procédé du stade 4 de l'exemple 1, en utilisant 450 mg du dérivé précédent comme produit de départ et 514 mg de nitrite de sodium dans 4 mL d'acide acétique conduisant à l'intermédiaire nitroso qui est réduit par 355 mg d'hydrure de lithium et d'aluminium (8 équivalents) dans 4 mL de tétrahydrofurane à reflux.
Rendement estimé : 23 %
SM : MH⁺ 366

### Stade 5 : 5-Bromo-2-méthoxy-4-[N'-(2-méthyl-6-pipéridin-1-yl-phényl)-N'-(4-trifluoro méthoxy-phényl)-hydrazinocarbonylméthyl]-benzoate de méthyle

Le produit est obtenu selon le procédé du stade 3 de l'exemple 6, en utilisant 400 mg de l'hydrazine précédente et 365 mg de l'acide de la préparation 2 en présence de 231 mg d'EDCI et 163 mg d'HOBt dans 4 mL de diméthylformamide. Une purification par chromatographie en phase inverse (conditions : colonne C18, 21,2x150 mm, mode isochratique 35% acétonitrile/H2O + 0,05% TFA, débit : 15 mL/min, longueurs d'onde : 220 et 254 nm) a permis d'isoler 260 mg du produit attendu sous forme de sel de TFA.
Rendement : 36 %
RMN 1H (CDCl3, 300 MHz) δ (ppm) : 12,26 (s élargi, 1H), 11,42 (s, 1H), 7,94 (s, 1H), 7,50 (m, 3H), 7,09 (m, 3H), 6,62 (d, 2H), 4,02 (m, 2H), 3,87 (s, 6H), 3,60 (m, 1H), 3,31 (m, 2H), 3,15 (m, 1H), 2,24 (s, 3H), 2,14 (m, 1H), 1,79-1,94 (m, 4H), 1,52 (m, 1H) HPLC : 95 %
SM : MH⁺ 650/652

### Stade 6 : Chlorhydrate d'acide 5-bromo-2-méthoxy-4-[N'-(2-méthyl-6-pipéridin-1-yl-phényl)-N'-(4-trifluorométhoxy-phényl)-hydrazinocarbonylméthyl]-benzoïque (10)

Le produit (166 mg) est obtenu sous forme de chlorhydrate selon le procédé du stade 6 de l'exemple 2, en utilisant comme substrat le produit obtenu au stade précédent.
Rendement : 62 %
PF : 128 °C (décomposition)
Analyse élémentaire calculée pour C29H29BrF3N3O5.1HCl.1H2O: C, 50,41 ; H, 4,67 ; N, 6,08. Trouvée : C, 50,02 ; H, 4,68 ; N, 5,82.
HPLC : 96 %
SM : MH⁺ 636/638

### Exemple 11 : Chlorhydrate d'acide 5-bromo-2-méthoxy-4-[N'-(4'-méthoxy-biphényl-4-yl)-N'-(2-pipéridin-1-yl-phényl)-hydrazinocarbonylméthyl]-benzoïque (11)

### Stade 1 : (4-Bromo-phényl)-(2-nitro-phényl)-amine

Le produit (6,49 g) est obtenu selon le procédé du stade 1 de l'exemple 4, en utilisant 3,73 mL de 2-fluoro-nitrobenzène et 7,31 g de 4-bromoaniline en présence de 6,36 g de tert-butanolate de potassium dans 120 mL de DMSO.
Rendement : 62 %
RMN 1H (CDCl3, 300 MHz) δ (ppm) : 9,38 (s élargi, 1H), 8,19 (d, 1H), 7,51 (d, 2H), 7,38 (t, 1H), 7,17 (t, 3H), 6,81 (t, 1H)
HPLC : 99 %
SM : MH⁺ 293/295

### Stade 2 : N-(4-Bromo-phényl)-benzène-1,2-diamine

Le produit (571 mg) est obtenu selon le procédé du stade 2 de l'exemple 1, en utilisant 723 mg du dérivé précédent comme produit de départ et 2,78 g de chlorure d'étain hydraté dans 7 mL d'éthanol.
Rendement : 88 %
RMN 1H (CDCl3, 300 MHz) δ (ppm) : 7,26 (d, 2H), 7,05 (m, 2H), 6,78 (m, 2H), 6,60 (d, 1H)
HPLC : 97 %
SM : MH⁺ 263/265

### Stade 3 : (4-Bromo-phényl)-(2-pipéridin-1-yl-phényl)-amine

Le produit (1,03 g) est obtenu selon le procédé du stade 3 de l'exemple 4, en utilisant 1,35 g du dérivé précédent comme produit de départ, 698 µL de dibromopentane et 2,1 mL de DIPEA dans 13 mL de toluène.
Rendement : 61 %
RMN 1H (CDCl3, 300 MHz) δ (ppm) : 7,36 (m, 2H), 7,26 (m, 1H), 7,01-7,10 (m, 4H), 6,89 (m, 1H), 6,56 (s élargi, 1H), 2,82 (t, 4H), 1,57-1,74 (m, 6H)
HPLC: 100 %
SM : MH⁺ 331/33

### Stade 4 : (4'-Méthoxy-biphényl-4-yl)-(2-pipéridin-1-yl-phényl)-amine

A une solution de 554 mg du dérivé bromé obtenu précédemment dans 14 mL d'un mélange 50 : 50 de méthanol/toluène sont ajoutés successivement 380 mg d'acide phényl boronique (1,5 équivalents), 96 mg de palladium tétrakis (0,05 équivalent), 212 mg de chlorure de lithium (3 équivalents) et 4,17 mL d'une solution 1 molaire de carbonate de calcium. L'ensemble est porté à reflux pendant 2 h. Le brut réactionnel est extrait à l'acétate d'éthyle plusieurs fois, les phases organiques réunies sont lavées à l'eau, puis séchées sur sulfate de magnésium, filtrées et évaporées sous pression réduite. Le résidu obtenu est purifié par chromatographie sur gel de silice (éther de pétrole puis éther de pétrole/acétate d'éthyle : 99/1) pour conduire à 260 mg du produit attendu.
Rendement : 43 %
RMN 1H (CDCl3, 300 MHz) δ (ppm) : 7,53 (m, 4H), 7,38 (dd, 1H), 7,25 (m, 2H), 7,12 (dd, 1H), 6,97-7,10 (m, 3H), 6,88 (td, 1H), 6,76 (s élargi, 1H), 3,86 (s, 3H), 2,87 (t, 4H), 1,52-1,78 (m, 6H)
HPLC : 80 %
SM : MH⁺ 359

### Stade 5 : N-(4'-Méthoxy-biphényl-4-yl)-N-(2-pipéridin-1-yl-phényl)-hydrazine (k)

Le produit (141 mg) est obtenu selon le procédé du stade 4 de l'exemple 1, en utilisant 203 mg du dérivé précédent comme produit de départ et 226 mg de nitrite de sodium dans 1,5 mL d'acide acétique conduisant à l'intermédiaire nitroso qui est réduit par 179 mg d'hydrure de lithium et d'aluminium (8 équivalents) dans 4 mL de tétrahydrofurane à reflux.
Rendement : 64 %
RMN ¹H (CDC13, 300 MHz) δ (ppm) : 7,49 (d, 2H), 7,38 (d, 2H), 7,14 (m, 2H), 6,93 (m, 4H), 6,87 (d, 2H), 3,84 (s, 3H), 2,92 (m, 4H), 1,39 (m, 6H)
HPLC : 90 %
SM : MH⁺ 374

### Stade 6 : 5-Bromo-2-méthoxy-4-[N'-(4'-méthoxy-biphényl-4-yl)-N'-(2-pipéridin-1-yl-phényl)-hydrazinocarbonylméthyl]-benzoate de méthyle

Le produit est obtenu selon le procédé du stade 3 de l'exemple 6, en utilisant 141 mg de l'hydrazine précédente et 126 mg de l'acide de la préparation 2 en présence de 79 mg d'EDCI et 56 mg d'HOBt dans 6 mL de diméthylformamide. Une purification par chromatographie en phase inverse (conditions : colonne C18, 21,2x150 mm, mode isochratique 50% acétonitrile/H2O + 0,05% TFA, débit : 15 mL/min, longueurs d'onde : 220 et 254 nm) a permis d'isoler 9 mg du produit attendu sous forme de sel de TFA.
Rendement (non optimisé) : 3 %
HPLC : 75 %
SM : MH⁺ 658/660

### Stade 7: Chlorhydrate d'acide 5-bromo-2-méthoxy-4-[N'-(4'-méthoxy-biphényl-4-yl)-N'-(2-pipéridin-1-yl-phényl)-hydrazinocarbonylméthyl]-benzoïque (11)

Le produit (7,3 mg) est obtenu sous forme de chlorhydrate selon le procédé du stade 6 de l'exemple 2, en utilisant comme substrat le produit obtenu au stade précédent.
Rendement : 92 %
HPLC : 80 %
SM : MH⁺ 644/646

### Exemple 12 : Chlorhydrate d'acide 5-bromo-4-[N'-(4-cyclohexyloxy-phényl)-N'-(2-pipéridin-1-yl-phényl)-hydrazinocarbonylméthyl]-2-méthoxy-benzoïque (12)

### Stade 1 : 1-Cyclohexyloxy-4-nitro-benzène

A 3,9 g de cyclohexanol (1,1 équivalents) mis en présence de 2,12 g d'hydrure de sodium (1,5 équivalents) sous agitation pendant 10 minutes est ajoutée une solution de 5 g de 4-fluoronitrobenzène dans 75 mL de diméthylformamide. L'ensemble est chauffé à 60 °C pendant 5 h. Après retour à température ambiante, le milieu est hydrolyse et le brut réactionnel extrait à l'acétate d'éthyle plusieurs fois. Les phases organiques sont séchées sur sulfate de magnésium, filtrées et évaporées sous pression réduite. Une chromatographie sur gel de silice du résidu (cyclohexane puis cyclohexane/acétate d'éthyle : 98/2 et 90/10) permet d'isoler 6,06 g du produit désiré.
Rendement : 78 %
RMN ¹H (CDCl3, 300 MHz) δ (ppm) : 8,16 (d, 2H), 6,92 (d, 2H), 4,37 (m, 1H), 1,98 (m, 2H), 1,81 (m, 2H), 1,35-1,65 (m, 6H)
HPLC : 98 %
SM : [M+Na]⁺ 244

### Stade 2 : 4-Cyclohexyloxy-phénylamine

Le produit (4,48 g) est obtenu selon le procédé du stade 2 de l'exemple 5, en utilisant 6,06 g du dérivé précédent comme produit de départ et 30,9 g de chlorure d'étain hydraté dans 59 mL d'éthanol.
Rendement : 87 %
RMN 1 H (CDCl3, 300 MHz) δ (ppm) : 6,76 (d, 2H), 6,62 (d, 2H), 4,06 (m, 1H), 3,42 (s élargi, 2H), 1,95 (m, 2H), 1,78 (m, 2H), 1,28-1,60 (m, 6H)
HPLC : 88 %
SM:MH⁺192

### Stade 3 : (4-Cyclohexyloxy-phényl)-(2-nitro-phényl)-amine

Le produit (4,27 g) est obtenu selon le procédé du stade 1 de l'exemple 4, en utilisant 2,95 mL de 2-fluoro-nitrobenzène et 4,48 g de produit obtenu au stade précédent en présence de 4,18 g de tert-butanolate de potassium dans 108 mL de DMSO.
Rendement : 59 %
RMN 1H (CDCl3, 300 MHz) δ (ppm) : 9,40 (s élargi, 1H), 8,20 (d, 1H), 7,35 (m, 1H), 7,18 (m, 2H), 6,95-7,05 (m, 3H), 6,72 (m, 1H), 4,06 (m, 1H), 2,05 (m, 2H), 1,82 (m, 2H), 1,35-1,60 (m, 6H)
HPLC : 86 %
SM:MH⁺313

### Stade 4 : N-(4-Cyclohexyloxy-phényl)-benzène-1,2-diamine

Le produit (3,43 g) est obtenu selon le procédé du stade 2 de l'exemple 1, en utilisant 4,27 g du dérivé précédent comme produit de départ et 15,42 g de chlorure d'étain hydraté dans 27 mL d'éthanol.
Rendement : 88 %
RMN 1H (CDCl3, 300 MHz) δ (ppm) : 7,04 (d, 1H), 6,94 (m, 1H), 6,72-6,84 (m, 6H), 4,14 (m, 1H), 1,97 (m, 2H), 1,80 (m, 2H), 1,30-1,60 (m, 6H)
HPLC : 92 %
SM : MH⁺ 283

### Stade 5 : (4-Cyclohexyloxy-phényl)-(2-pipéridin-1-yl-phényl)-amine

Le produit (1,55 g) est obtenu selon le procédé du stade 3 de l'exemple 4, en utilisant 3,43 g du dérivé précédent comme produit de départ, 1,65 mL de dibromopentane et 5,1 mL de DIPEA dans 49 mL de toluène.
Rendement : 36 %
RMN 1 H (CDCl3, 300 MHz) δ (ppm) : 7,07 (m, 4H), 6,80-6,98 (m, 3H), 6,78 (m, 1H), 6,52 (s élargi, 1H), 4,21 (m, 1H), 2,86 (m, 4H), 1,35-2,05 (m, 16H)
HPLC : 99%
SM: MH⁺ 351

### Stade 6 : N-(4-Cyclohexyloxy-phényl)-N-(2-pipéridin-1-yl-phényl)-hydrazine (1)

Le produit (118 mg) est obtenu selon le procédé du stade 4 de l'exemple 1, en utilisant 500 mg du dérivé précédent comme produit de départ et 571 mg de nitrite de sodium dans 4 mL d'acide acétique conduisant à l'intermédiaire nitroso qui est réduit par 431 mg d'hydrure de lithium et d'aluminium (8 équivalents) dans 4 mL de tétrahydrofurane à reflux.
Rendement : 23 %
RMN ¹H (CDCl3, 300 MHz) δ (ppm) : 7,00-7,18 (m, 5H), 6,94 (m, 1H),6,90 (m, 2H), 4,77 (s élargi, 1H), 4,09 (m, 1H), 2,98 (m, 4H), 2,00 (m, 2H), 1,80 (m, 2H), 1,24-1,67 (m, 12H)
HPLC : 94 %
SM : MH⁺ 366

### Stade 7 : 5-Brorno-4[N'-(4-cyclohexyloxy-phényl)-N'-(2-pipéridin-1-yl-phényl)-hydrazinocarbonylméthyl]-2-méthoxy-benzoate de méthyle

Le produit est obtenu selon le procédé du stade 3 de l'exemple 6, en utilisant 50 mg de l'hydrazine précédente et 46 mg de l'acide de la préparation 2 en présence de 29 mg d'EDCI et 20 mg d'HOBt dans 1.5 mL de diméthylformamide. Une purification par chromatographie en phase inverse d'une fraction (conditions : colonne C18, 21,2x150 mm, mode isochratique 45% acétonitrile/H2O + 0,05% TFA, débit : 15 mL/min, longueurs d'onde : 220 et 254 nm) a permis d'isoler 57 mg du produit attendu sous forme de sel de TFA.
Rendement estimé : 54 %
HPLC : 92 %
SM : MH⁺ 650/652

### Stade 8 : Chlorhydrate d'acide 5-bromo-4-[N'-(4-cyclohexyloxy-phényl)-N'-(2-pipéridin-1-yl-phényl)-hydrazinocarbonylméthyl]-2-méthoxy-benzoïque (12)

Le produit (61 mg) est obtenu sous forme de chlorhydrate selon le procédé du stade 6 de l'exemple 2, en utilisant comme substrat le produit obtenu au stade précédent.
Rendement : 85 %
PF : 145-180 °C
HPLC : 91 %
SM : MH⁺ 636/638

### Exemple 13 : Chlorhydrate d'acide 5-bromo-2-méthoxy-4-[N'-(4-phénoxy-phényl)-N'-(2-pipéridin-1-yl-phényl)-hydrazinocarbonylméthyl]-benzoïque (13)

### Stade 1 : (2-Nitro-phényl)-(4-phénoxy-phényl)-amine

Le produit (2,03 g) est obtenu selon le procédé du stade 1 de l'exemple 4, en utilisant 3,7 mL de 2-fluoro-nitrobenzène et 7,88 g de 4-phénoxyaniline en présence de 6,36 g de tert-butanolate de potassium dans 34 mL de DMSO.
Rendement : 18 %
RMN 1H (CDCl3, 300 MHz) δ (ppm) : 9,44 (s élargi, 1H), 8,20 (d, 1H), 7,37 (m, 3H), 7,25 (m, 2H), 7,05-7,18 (m, 6H), 6,76 (m, 1H)
HPLC : 80 %
SM : MH⁺ 307

### Stade 2 : N-(4-phénoxy-phényl)-benzène-1,2-diamine

Le produit (709 mg) est obtenu par hydrogénation catalytique en présence de 203 mg de palladium sur charbon à 10% dans 40 mL d'éthanol.
Rendement : 38 %
RMN 1H (CDCl3, 300 MHz) δ (ppm) : 7,30 (m, 2H), 6,90-7,12 (m, 7H), 6,70-6,85 (m, 4H)
HPLC: 100 %
SM : MH⁺ 277

### Stade 3 : (4-Phénoxy-phényl)-(2-pipéridin-1yl-phényl)-amine

Le produit (799 mg) est obtenu selon le procédé du stade 3 de l'exemple 4, en utilisant 709 mg du dérivé précédent comme produit de départ, 350 µL de dibromopentane et 1,07 mL de DIPEA dans 10 mL de toluène.
Rendement : 82 %
RMN 1H (DMSO, 300 MHz) δ (ppm) : 7,40 (d, 1H), 7,25 (m, 4H), 7,03 (m, 4H), 6,95 (m, 4H), 3,37 (m, 4H), 1,90 (m, 4H), 1,61 (m, 2H)
HPLC : 100%
SM : MH⁺ 345

### Stade 4 : N-(4-Phénoxy-phényl)-N-(2-pipéridin-1-yl-phényl)-hydrazine (m)

Le produit (185 mg) est obtenu selon le procédé du stade 4 de l'exemple 1, en utilisant 400 mg du dérivé précédent comme produit de départ et 420 mg de nitrite de sodium dans 3 mL d'acide acétique conduisant à l'intermédiaire nitroso qui est réduit par 319 mg d'hydrure de lithium et d'aluminium (8 équivalents) dans 3 mL de tétrahydrofurane à reflux.
Rendement : 49 %
RMN ¹H (CDCl3, 300 MHz) δ (ppm) : 6,92-7,26 (m, 13H), 4,82 (s élargi, 2H), 2,97 (m, 4H), 1,55-1,69 (2m, 6H)
HPLC : 97 %
SM : MH⁺ 360

### Stade 5 : 5-Bromo-2-méthoxy-4-[N'-(4-phénoxy-phényl)-N'-(2-pipéridin-1-yl-phényl)-hydrazinocarbonylméthyl]-benzoate de méthyle

Le produit (270 mg) est obtenu selon le procédé du stade 3 de l'exemple 6, en utilisant 185 mg de l'hydrazine précédente et 172 mg de l'acide de la préparation 2 en présence de 108 mg d'EDCI et 76 mg d'HOBt dans 3 mL de diméthylformamide.
Rendement : 81 %
RMN ¹H (CDCl3, 300 MHz) δ (ppm) : 9,35 (s élargi, 1H), 7,97 (d, 1H), 6,75-7,50 (massif aromatique, 14H), 3,70-3,94 (3s, 8H), 2,68 (m, 4H), 1,46-1,59 (m, 6H)
HPLC : 77 %
SM : MH⁺ 644/646

### Stade 6 : Chlorhydrate d'acide 5-bromo-2-méthoxy-4-[N'-(4-phénoxy-phényl)-N'-(2-pipéridin-1-yl-phényl)-hydrazinocarbonylméthyl]-benzoïque (13)

Le produit est obtenu selon le procédé du stade 6 de l'exemple 2, en utilisant comme substrat le produit obtenu au stade précédent. Une purification par chromatographie en phase inverse d'une fraction (conditions: colonne C18, 21,2x150 mm, mode isochratique 35% acétonitrile/H2O + 0,05% TFA, débit : 15 mL/min, longueurs d'onde : 220 et 254 nm) suivie d'un traitement par une solution 1 N d'acide chlorhydrique a permis d'isoler le produit attendu sous forme de chlorhydrate (89 mg).
Rendement : 32 %
PF : 232-235 °C
Analyse élémentaire calculée pour C33H32BrN3O5.1HCl.1H2O: C, 57,86 ; H, 5,15 ; N, 6,13. Trouvée : C, 57,74 ; H, 5,01 ; N, 5,89.
HPLC : 97 %
SM : MH⁺ 630/632

### Exemple 14 : Chlorhydrate d'acide 5-bromo-4-{N'-[4-(4-chloro-phénoxy)-phényl]-N'-(2-pipéridin-1-yl-phényl)-hydrazinocarbonylméthyl}-2-méthoxy-benzoïque (14)

### Stade 1 : [4-(4-Chloro-phénoxy)-phényl]-(2-nitro-phényl)-amine

Le produit (609 mg) est obtenu selon le procédé du stade 1 de l'exemple 4, en utilisant 840 µL de 2-fluoro-nitrobenzène et 2,1 g de 4-(chlorophénoxy)aniline en présence de 1,43 g de tert-butanolate de potassium dans 20 mL de DMSO.
Rendement : 22 %
RMN 1H (CDCl3, 300 MHz) δ (ppm) : 8,21 (d, 1H), 7,24-7,40 (m, 5H), 6,97-7,14 (m, 5H), 6,77 (t, 1H)
HPLC : 97 %
SM : MH⁺ 341/343

### Stade 2 : N-[4-(4-Chloro-phénoxy)phényl]-benzène-1,2-diamine

Le produit (524 mg) est obtenu selon le procédé du stade 2 de l'exemple 1, en utilisant 609 mg du dérivé précédent comme produit de départ et 2 g de chlorure d'étain hydraté dans 10 mL d'éthanol.
Rendement : 94 %
RMN 1H (CDCl3, 300 MHz) δ (ppm) : 6,73-7,38 (massif aromatique, 12H)
HPLC: 100 %
SM: MH⁺ 311/313

### Stade 3 : [4-(4-Chloro-phénoxy)-phényl]-(2-pipéridin-1-yl-phényl)-amine

Le produit (596 mg) est obtenu selon le procédé du stade 3 de l'exemple 4, en utilisant 684 mg du dérivé précédent comme produit de départ, 300 µL de dibromopentane et 920 µL de DIPEA dans 15 mL de toluène.
Rendement : 71 %
RMN 1H (CDC13, 300 MHz) δ (ppm) : 6,80-7,28 (massif aromatique, 12H), 6,66 (s élargi, 1H), 2,86 (m, 4H), 1,72 (m, 4H), 1,61 (m, 2H)
HPLC : 99 %
SM : MH⁺ 379/381

### Stade 4 : N-[4-(4-Chloro-phénoxy)-phényl]-N-(2-pipéridin-1-yl-phényl)-hydrazine (n)

Le produit (80 mg) est obtenu selon le procédé du stade 4 de l'exemple 1, en utilisant 300 mg du dérivé précédent comme produit de départ et 317 mg de nitrite de sodium dans 3 mL d'acide acétique conduisant à l'intermédiaire nitroso qui est réduit par 120 mg d'hydrure de lithium et d'aluminium (4 équivalents) dans 5 mL de tétrahydrofurane.
Rendement : 26 %
RMN ¹H (CDCl3, 300 MHz) δ (ppm) : 6,86-7,25 (massif aromatique, 12H), 2,98 (m, 4H), 1,70 (m, 4H), 1,56 (m, 2H)
HPLC : 83 %
SM : MH⁺ 394/396

### Stade 5 : 5-Bromo-4-{N'-[4-(4-chloro-phénoxy)phényl]-N'-(2-pipéridin-1-yl-phényl)-hydrazinocarbonylméthyl}-2-méthoxy-benzoate de méthyle,

Le produit (86 mg) est obtenu selon le procédé du stade 3 de l'exemple 6, en utilisant 80 mg de l'hydrazine précédente et 68 mg de l'acide de la préparation 2 en présence de 43 mg d'EDCI et 30 mg d'HOBt dans 3 mL de diméthylformamide.
Rendement : 62 %
RMN ¹H (CDCl3, 300 MHz) δ (ppm) : 9,35 (s élargi, 1H), 7,97 (d, 1H), 6,75-7,52 (massif aromatique, 13H), 3,67-3,91 (3s, 8H), 2,68 (m, 4H), 1,42-1,59 (m, 6H)
HPLC : 94 %
SM : MH⁺ 678/680

### Stade 6 : Chlorhydrate d'acide 5-bromo-4-{N'-[4-(4-chloro-phénoxy)-phényl]-N'-(2-pipéridin-1-yl-phényl)-hydrazinocarbonylméthyl}-2-méthoxy-benzoïque (14)

Le produit (52 mg) est obtenu sous forme de chlorhydrate selon le procédé du stade 6 de l'exemple 2, en utilisant comme substrat le produit obtenu au stade précédent.
Rendement : 62 %
PF: 153,5-163,5 °C
HPLC : 95 %
SM : MH⁺ 664/666

### Exemple 15 : Chlorhydrate d'acide 4-{N'-[4-(4-fluoro-phénoxy)-phényl]-N'-(2-méthyl-6-pipéridin-1-yl-phényl)-hydrazinocarbonylméthyl}-2-méthoxy-benzoïque (15)

### Stade 1 : [4-(4-Fluoro-phénoxy)-phényl]-(2-méthyl-6-nitro-phényl)-amine

Le produit (790 mg) est obtenu selon le procédé du stade 1 de l'exemple 4, en utilisant 900 µL de 2-fluoro-3-méthyl-nitrobenzène et 2,6 g de 4-(4-fluoro-phénoxy)-phénylamine obtenu au stade 2 de l'exemple 5 en présence de 2,36 g de tert-butanolate de potassium dans 80 mL de DMSO.
Rendement : 40 %
RMN 1H (CDCl3, 300 MHz) δ (ppm) : 8,38 (s élargi, 1H), 1,98 (d, 1H), 7,40 (d, 1H), 6,87-7,06 (massif aromatique, 7H), 6,76 (d, 2H), 2,08 (s, 3H)
HPLC : 84 %
SM : MH⁺ 339

### Stade 2 : N²-[4-(4-Fluoro-phénoxy)-phényl]-3-méthyl-benzène-1,2-diamine

Le produit (1,58 g) est obtenu par hydrogénation selon le procédé du stade 2 de l'exemple 13.
Rendement : 87 %
RMN 1H (CDCl3, 300 MHz) δ (ppm) : 6,83-7,01 (massif aromatique, 7H), 6,67 (d, 2H), 6,55 (d, 2H), 4,92 (s élargi, 1 H), 3,89 (s élargi, 2H), 2,18 (s, 3H)
HPLC: 98 %
SM: MH⁺ 309

### Stade 3: [4-(4-Fluoro-phénoxy)-phényl]-(2-méthyl-6-pipéridin-1-yl-phényl)-amine

Le produit (1,16 g) est obtenu selon le procédé du stade 3 de l'exemple 4, en utilisant 1,58 g du dérivé précédent comme produit de départ, 700 µL de dibromopentane et 2,14 mL de DIPEA dans 30 mL de toluène.
Rendement : 60 %
RMN 1H (CDCl3, 300 MHz) δ (ppm) : 6,84-7,01 (massif aromatique, 9H), 6,70 (d, 2H), 6,18 (s élargi, 1H), 2,74 (m, 4H), 2,13 (s, 3H), 1,58 (m, 6H)
HPLC : 98 %
SM : MH⁺ 377

### Stade 4 : N-[4-(4-Fluoro-phénoxy)-phényl]-N-(2-méthyl-6-pipéridin-1-yl-phényl)-hydrazine (o)

Le produit (198 mg) est obtenu selon le procédé du stade 4 de l'exemple 1, en utilisant 525 mg du dérivé précédent comme produit de départ et 558 mg de nitrite de sodium dans 4 mL d'acide acétique conduisant à l'intermédiaire nitroso qui est réduit par 208 mg d'hydrure de lithium et d'aluminium (4 équivalents) dans 4 mL de tétrahydrofurane.
Rendement : 36 %
RMN ¹H (CDCl3, 300 MHz) δ (ppm) : 6,83-7,18 (massif aromatique, 11H), 4,87 (s élargi, 2H), 2,68 et 2,94 (4H, 2m), 2,12 (s, 3H), 1,50-1,64 (m, 6H)
HPLC : 76 %
SM : MH⁺ 392

### Stade 5 : 4-{N'-[4-(4-Fluoro-phénoxy)-phényl]-N'-(2-méthyl-6-pipéridin-1-yl-phényl)-hydrazinocarbonylméthyl}-2-méthoxy-benzoate de méthyle,

Le produit (141 mg) est obtenu selon le procédé du stade 3 de l'exemple 6, en utilisant 95 mg de l'hydrazine précédente et 60 mg de l'acide de la préparation 1 en présence de 51 mg d'EDCI et 36 mg d'HOBt dans 1,5 mL de diméthylformamide.
Rendement : 97 %
RMN ¹H (CDCl3, 300 MHz) δ (ppm) : 9,76 (s élargi, 1H), 7,74 (d, 1H), 6,50-7,22 (massif aromatique, 13H), 3,87 (s, 3H), 3,70 (m, 5H), 2,72 (m, 2H), 2,44 (s, 3H), 2,30 (m, 2H), 1,30-1,42 (2m, 6H)
HPLC : 97 %
SM : MH⁺ 598

### Stade 6 : Chlorhydrate d'acide 4-{N'-[4-(4-fluoro-phénoxy)-phényl]-N'-(2-méthyl-6-pipéridin-1-yl-phényl)-hydrazinocarbonylméthyl}-2-méthoxy-benzoïque (15)

Le produit (131 mg) est obtenu sous forme de chlorhydrate selon le procédé du stade 6 de l'exemple 2, en utilisant comme substrat le produit obtenu au stade précédent.
Rendement : 90 %
PF: 155-168 °C
HPLC : 100 %
SM : MH⁺ 584

### Exemple 16 : Chlorhydrate d'acide 5-bromo-4-{N'-[4-(4-fluoro-phénoxy)-phényl]-N'-(2-méthyl-6-pipéridin-1-yl-phényl)-hydrazinocarbonylméthyl}-2-méthoxy-benzoïque (16)

### Stade 1 : 5-Bromo-4-{N'-[4-(4-fluoro-phénoxy)-phényl]-N'-(2-méthyl-6-pipéridin-1-yl-phényl)-hydrazinocarbonylméthyl}-2-méthoxy-benzoate de méthyle (16a)

Le produit (154 mg) est obtenu selon le procédé du stade 3 de l'exemple 6, en utilisant 95 mg de l'hydrazine du stade 4 de l'exemple 15 et 81 mg de l'acide de la préparation 2 en présence de 51 mg d'EDCI et 36 mg d'HOBt dans 1,5 mL de diméthylformamide. Rendement : 94 %
RMN ¹H (CDCl3, 300 MHz) δ (ppm) : 9,86 (s élargi, 1H), 7,98 (s, 1H), 6,84-7,23 (massif aromatique, 10H), 6,57 (d, 2H), 3,87 (s, 3H), 3,76 (s, 2H), 3,71 (s, 3H), 2,80 (m, 2H), 2,44 (s, 5H), 1,37-1,48 (2m, 6H)
HPLC : 91 %
SM : MH⁺ 676/678

### Stade 2 : Chlorhydrate d'acide 5-bromo-4-{N'-[4-(4-fluoro-phénoxy)-phényl]-N'-(2-méthyl-6-pipéridin-1-yl-phényl)-hydrazinocarbonylméthyl}-2-méthoxy-benzoïque (16)

Le produit (116 mg) est obtenu sous forme de chlorhydrate selon le procédé du stade 6 de l'exemple 2, en utilisant comme substrat le produit obtenu au stade précédent.
Rendement : 73 %
PF: 155-168 °C
Analyse élémentaire calculée pour C34H33BrFN3O5.1HCl.1,5H2O : C, 56,25 ; H, 5,14 ; N, 5,79. Trouvée : C, 56,31 ; H, 5,04 ; N, 5,61.
HPLC : 98 %
SM : MH⁺ 662/664

### Exemple 17 : Chlorhydrate d'acide 4-[N'-(4-benzyl-phényl)-N'-(2-pipéridin-1-yl-phényl)-hydrazinocarbonylméthyl]-5-bromo-2-méthoxy-benzoïque (17)

### Stade 1 : (4-Benzyl-phényl)-(2-nitro-phényl)-amine

Le produit (621 mg) est obtenu selon le procédé du stade 1 de l'exemple 4, en utilisant 958 µL de 2-fluoro-nitrobenzène et 2 g de 4-benzylaniline en présence de 1,63 g de tert-butanolate de potassium dans 30 mL de DMSO.
Rendement : 22 %
RMN 1H (CDCl3, 300 MHz) δ (ppm) : 9,46 (s élargi, 1H), 8,19 (d, 1H), 7,71 (d, 1H), 7,44 (m, 1H), 7,10-7,40 (massif aromatique, 9H), 6,74 (m, 1H), 4,01 (s, 2H)
HPLC : 78 %
SM : [M+Na]⁺ 327

### Stade 2 : N-(4-Benzyl-phényl)-benzène-1,2-diamine

Le produit (291 mg) est obtenu par hydrogénation selon le procédé du stade 2 de l'exemple 13.
Rendement : 52 %
HPLC: 100 %
SM : MH⁺ 275

### Stade 3 : (4-Benzyl-phényl)-(2-pipéridin-1-yl-phényl)-amine

Le produit (213 mg) est obtenu selon le procédé du stade 3 de l'exemple 4, en utilisant 291 mg du dérivé précédent comme produit de départ, 144 µL de dibromopentane et 444 µL de DIPEA dans 4 mL de toluène.
Rendement : 59 %
RMN 1H (CDCl3, 300 MHz) δ (ppm): 6,79-7,32 (massif aromatique, 13H), 6,63 (s élargi, 1H), 3,94 (s, 2H), 2,83 (m, 4H), 1,70 (m, 4H), 1,58 (m, 2H)
HPLC : 96 %
SM : MH⁺ 343

### Stade 4 : N-(4-Benzyl-phényl]-N-(2-pipéridin-1-yl-phényl)-hydrazine (p)

Le produit (60 mg) est obtenu selon le procédé du stade 4 de l'exemple 1, en utilisant 213 mg du dérivé précédent comme produit de départ et 249 mg de nitrite de sodium dans 3 mL d'acide acétique conduisant à l'intermédiaire nitroso qui est réduit par 189 mg d'hydrure de lithium et d'aluminium (8 équivalents) dans 3 mL de tétrahydrofurane.
Rendement : 27%
RMN ¹H (CDCl3, 300 MHz) δ (ppm) : 6,92-7,31 (massif aromatique, 13H), 4,75 (s élargi, 1 H), 3,91 (s, 2H), 2,96 (m, 4H), 1,66 (m, 4H), 1,54 (m, 2H)
HPLC: 100 %
SM : MH⁺ 358

### Stade 5 : 4-[N'-(4-Benzy/-phényl)-N'-(2-pipéridin-1-y/-phény/)-hydrazinocarbonylméthyl]-5-bromo-2-méthoxy-benzoate de méthyle

Le produit (64 mg) est obtenu selon le procédé du stade 3 de l'exemple 6, en utilisant 60 mg de l'hydrazine précédente et 56 mg de l'acide de la préparation 2 en présence de 35 mg d'EDCI et 25 mg d'HOBt dans 1 mL de diméthylformamide.
Rendement : 59 %
RMN ¹H (CDCl3, 300 MHz) δ (ppm) : 9,33 (s élargi, 1H), 7,98 (d, 1H), 6,71-7,51 (massif aromatique, 14H), 3,95 (s, 6H), 3,70 et 3,76 (2s, 4H), 2,74 (m, 4H), 1,28-1,60 (m, 6H)
HPLC : 90 %
SM : MH⁺ 642/644

### Stade 6 : Chlorhydrate d'acide 4-[N'-(4-benzyl-phényl)-N'-(2-pipéridin-1-yl-phényl)-hydrazinocarbonylméthyl]-5-bromo-2-méthoxy-benzoïque (17)

Le produit (58 mg) est obtenu sous forme de chlorhydrate selon le procédé du stade 6 de l'exemple 2, en utilisant comme substrat le produit obtenu au stade précédent.
Rendement : 88 %
PF : 137,5-162 °C
HPLC : 97 %
SM : MH⁺ 628/630

### Exemple 18 : Chlorhydrate d'acide 4-[N'-(4-bromo-phényl)-N'-(2-pipéridin-1-yl-phényl)-hydrazinocarbonylméthyl]-5-chloro-2-méthoxy-benzoïque (18)

### Stade 1 : N-(4-Bromo-phényl)-N-(2-pipéridin-1-yl-phényl)-hydrazine (q)

Le produit (370 mg) est obtenu selon le procédé du stade 4 de l'exemple 1, en utilisant 500 mg du dérivé obtenu au stade 3 de l'exemple 11 comme produit de départ et 604 mg de nitrite de sodium dans 3 mL d'acide acétique conduisant à l'intermédiaire nitroso qui est réduit par 458 mg d'hydrure de lithium et d'aluminium (8 équivalents) dans 3 mL de tétrahydrofurane.
Rendement : 71 %
RMN ¹H (CDCl3, 300 MHz) δ (ppm) : 6,98-7,25 (massif aromatique, 8H), 4,77 (s élargi, 2H), 2,92 (m, 4H), 1,66 (m, 4H), 1,55 (m, 2H)
HPLC : 88 %
SM : MH⁺ 346/348

### Stade 2 : 4-[N'-(4-Bromo-phényl)-N'-(2-pipéridin-1-yl-phényl)-hydrazinocarbonylméthyl]-5-chloro-2-méthoxy-benzoate de méthyle

Le produit (64 mg) est obtenu selon le procédé du stade 3 de l'exemple 6, en utilisant 370 mg de l'hydrazine précédente et 304 mg de l'acide de la préparation 3 en présence de 225 mg d'EDCI et 159 mg d'HOBt dans 5 mL de diméthylformamide.
Rendement : 89 %
RMN ¹H (CDCl3, 300 MHz) δ (ppm) : 9,34 (s élargi, 1H), 7,78 (d, 1H), 6,63-7,49 (massif aromatique, 9H), 3,87 (2s, 6H), 3,70 (s, 2H), 2,75 (m, 4H), 1,28 et 1,60 (2m, 6H)
HPLC : 96 %
SM : MH⁺ 586/588

### Stade 3 : Chlorhydrate d'acide 4-[N'-(4-bromo-phényl)-N'-(2-pipéridin-1-yl-phényl)-hydrazinocarbonylméthyl]-5-chloro-2-méthoxy-benzoïque (18)

Le produit (39 mg) est obtenu sous forme de chlorhydrate selon le procédé du stade 6 de l'exemple 2, en utilisant comme substrat le produit obtenu au stade précédent.
Rendement : 75 %
PF : 177,2-189 °C
HPLC : 95 %
SM : MH⁺ 572/574

### Exemple 19 : Chlorhydrate d'acide 4-[N'-(3'-acétyl-biphényl-4-yl)-N'-(2-pipéridin-1-yl-phényl)-hydrazinocarbonylméthyl]-5-chloro-2-méthoxy-benzoïque (19)

### Stade 1 : 4-[N'-(3'-Acétyl-biphényl-4-yl)-N'-(2-pipéridin-1-yl-phényl)-hydrazinocarbonylméthyl]-5-chloro-2-méthoxy-benzoate de méthyle

A une solution de 200 mg du dérivé bromé obtenu au stade 2 de l'exemple 18 dans 2 mL d'un mélange 50 : 50 de méthanol/toluène sont ajoutés successivement 84 mg d'acide 3-acétylphényl boronique (1,5 équivalents), 20 mg de palladium tétrakis (0,05 équivalent) et 85 µL d'une solution 1 molaire de carbonate de sodium. L'ensemble est porté à reflux pendant 3 h. Le brut réactionnel repris dans l'eau est extrait à l'acétate d'éthyle plusieurs fois, les phases organiques réunies sont lavées avec une solution 1 molaire de soude, puis séchées sur sulfate de magnésium, filtrées et évaporées sous pression réduite. Le résidu obtenu est purifié par chromatographie sur gel de silice (éther de pétrole/acétate d'éthyle : 90/10 puis 80/20 jusqu'à 50/50) pour conduire à 110 mg du produit attendu.
Rendement : 51 %
RMN 1H (CDC13, 300 MHz) δ (ppm) : 9,35 (s, 1H), 8,04 (d, 1H), 7,74 (m, 3H), 6,94-7,62 (massif aromatique, 8H), 6,80 (m, 2H), 2,79-3,62 (3s, 8H), 2,56 (m, 4H), 2,05 (s, 3H), 1,18-1,53 (m, 6H)
HPLC : 92 %
SM : MH⁺ 626/628

### Stade 2 : Chlorhydrate d'acide 4-[N'-(3'-acétyl-biphényl-4-yl)-N'-(2-pipéridin-1-yl-phényl)-hydrazinocarbonylméthyl]-5-chloro-2-méthoxy-benzoïque (19)

Le produit (69 mg) est obtenu sous forme de chlorhydrate selon le procédé du stade 6 de l'exemple 2, en utilisant comme substrat le produit obtenu au stade précédent.
Rendement : 66 %
PF : 140,2-165,3 °C
HPLC : 91 %
SM: MH⁺ 612/614

### Exemple 20 : Chlorhydrate d'acide 4-[N'-(4'-acétyl-biphényl-4-yl)-N'-(2-pipéridin-1-yl-phényl)-hydrazinocarbonylméthyl]-5-chloro-2-méthoxy-benzoïque (20)

### Stade 1 : 4-[N'-(4'-Acétyl-biphényl-4-yl)-N'-(2-pipéridin-1-yl-phényl)-hydrazinocarbonylméthyl]-5-chloro-2-méthoxy-benzoate de méthyle

Le produit (167 mg) est obtenu selon le procédé du stade 1 de l'exemple 19, en utilisant comme substrat le produit obtenu au stade 2 de l'exemple 18 et comme co-substrat l'acide 4-acétylphényl boronique.
Rendement : 78 %
RMN 1H (CDCl3, 300 MHz) δ (ppm) : 9,35 (s, 1H), 6,70-7,91 (massif aromatique, 14H), 3,61-3,78 (3s, 8H), 2,60 (m, 4H), 2,28 (s, 3H), 1,20-1,51 (m, 6H)
HPLC : 87 %
SM : MH⁺ 626/628

### Stade 2 : Chlorhydrate d'acide 4-[N'-(4'-acétyl-biphényl-4-yl)-N'-(2-pipéridin-1-yl-phényl)-hydrazinocarbonylméthyl]-5-chloro-2-méthoxy-benzoïque (20)

Le produit (82 mg) est obtenu sous forme de chlorhydrate selon le procédé du stade 6 de l'exemple 2, en utilisant comme substrat le produit obtenu au stade précédent.
Rendement : 47 %
PF: 165,3-190,1 °C
HPLC : 100 %
SM: MH⁺ 612/614

### Exemple 21 : Chlorhydrate d'acide 5-bromo-2-méthoxy-4-[N'-(3-phénoxy-phényl)-N'-(2-pipéridin-1-yl-phényl)-hydrazinocarbonylméthyl]-benzoïque (21)

### Stade 1 : (2-Nitro-phényl)-(3-phénoxy-phényl)-amine

Le produit (2,08 g) est obtenu selon le procédé du stade 1 de l'exemple 4, en utilisant 1,23 mL de 2-fluoro-nitrobenzène et 3,3 g de 3-phénoxyaniline en présence de 2,13 g de tert-butanolate de potassium dans 10 mL de DMSO.
Rendement : 57 %
RMN 1H (CDCl3, 300 MHz) δ (ppm) : 9,38 (s élargi, 1H), 8,12 (d, 1H), 7,63 (d, 1H), 6,70-7,43 (m, 11H)
HPLC : 79 %
SM : MH⁺ 307

### Stade 2 : N-(3-phénoxy-phényl)-benzène-1,2-diamine

Le produit (1,88 g) est obtenu par hydrogénation catalytique en présence de 210 mg de palladium sur charbon à 10% dans 40 mL d'un mélange acétate d'éthyle/ éthanol (1 : 1).
Rendement : quantitatif
HPLC : 91 %
SM : MH⁺ 277

### Stade 3 : (3-Phénoxy-phényl)-(2-pipéridin-1-yl-phényl)-amine

Le produit (1,22 g) est obtenu selon le procédé du stade 3 de l'exemple 4, en utilisant 1,88 g du dérivé précédent comme produit de départ, 925 µL de dibromopentane et 2,84 mL de DIPEA dans 40 mL de toluène.
Rendement : 52 %
RMN 1H (CDCl3, 300 MHz) δ (ppm): 6,83-7,19 (massif aromatique, 11H), 6,72 (s, 1H), 6,53 (dd, 1H), 2,81 (m, 4H), 1,50-1,74 (m, 6H)
HPLC: 100 %
SM : MH⁺ 345

### Stade 4 : N-(3-Phénoxy-phényl)-N-(2-pipéridin-1-yl-phényl)-hydrazine (r)

Le produit (99 mg) est obtenu selon le procédé du stade 4 de l'exemple 1, en utilisant 400 mg du dérivé précédent comme produit de départ et 465 mg de nitrite de sodium dans 3 mL d'acide acétique conduisant à l'intermédiaire nitroso qui est réduit par 159 mg d'hydrure de lithium et d'aluminium (4 équivalents) dans 4 mL de tétrahydrofurane à reflux.
Rendement : 24 %
RMN ¹H (CDCl3, 300 MHz) δ (ppm) : 6,70-7,25 (massif aromatique, 12H), 6,28 (dd, 1H), 4,68 (s élargi, 2H), 2,85 (m, 4H), 1,46-1,63 (m, 6H)
HPLC : 76 %
SM : MH⁺ 360

### Stade 5 : 5-Bromo-2-méthoxy-4-[N'-(3-phénoxy-phényl)-N'-(2-pipéridin-1-yl-phényl)-hydrazinocarbonylméthyl]-benzoate de méthyle

Le produit (168 mg) est obtenu selon le procédé du stade 3 de l'exemple 6, en utilisant 99 mg de l'hydrazine précédente et 92 mg de l'acide de la préparation 2 en présence de 58 mg d'EDCI et 41 mg d'HOBt dans 1 mL de diméthylformamide.
Rendement : 95 %
RMN ¹H (CDC13, 300 MHz) δ (ppm) : 9,35 et 9,40 (2 s élargis, 1H), 7,98 (2s, 1H), 6,41-7,40 (massif aromatique, 14H), 3,70-3,95 (3s, 8H), 2,68 (m, 4H), 1,46-1,59 (m, 6H)
HPLC : 96 %
SM : MH⁺ 644/646

### Stade 6 : Chlorhydrate d'acide 5-bromo-2-méthoxy-4-[N'-(3-phénoxy-phényl)-N'-(2-pipéridin-1-yl-phényl)-hydrazinocarbonylméthyl]-benzoïque (21)

Le produit (124 mg) est obtenu sous forme de chlorhydrate selon le procédé du stade 6 de l'exemple 2, en utilisant comme substrat le produit obtenu au stade précédent.
Rendement : 71 %
PF : 142-159 °C
Analyse élémentaire calculée pour C33H32BrN3O5.0,75HCl : C, 60,25 ; H, 5,02 ; N, 6,39. Trouvée : C, 59,98 ; H, 5,11 ; N, 6,27.
HPLC : 96 %
SM : MH⁺ 630/632

### Exemple 22 : Chlorhydrate d'acide 5-bromo-2-méthoxy-4-[N'-(4-phénylsulfanyl-phényl)-N'-(2-pipéridin-1-yl-phényl)-hydrazinocarbonyl méthyl]-benzoïque (22)

### Stade 1 : 4-Phénylsulfanyl-phénylamine

A une solution de 5 g de 4-nitrophényl phényl sulfure dans 100 mL d'un mélange éthanol/ acétate d'éthyle (1 : 1) sont additionnés 500 mg de palladium sur charbon à 10%. L'ensemble est placé sous atmosphère d'hydrogène (P = 10 bars) pendant 1 nuit. L'ensemble est filtré sur célite, rinsé et le filtrat concentré sous pression réduite pour conduire à 3,57 g du produit désiré.
Rendement : 82%
RMN 1H (CDCl3, 300 MHz) δ (ppm): 7,10-7,35 (massif aromatique, 7H), 6,69 (d, 2H), 3,98 (s élargi, 2H)
HPLC : 86 %
SM : MH⁺ 202

### Stade 2 : (2-Nitro-phényl)-(4-phénylsulfanyl-phényl)-amine

Le produit (2,50 g) est obtenu selon le procédé du stade 1 de l'exemple 4, en utilisant 1,25 mL de 2-fluoro-nitrobenzène et 3,57 g de produit obtenu au stade précédent en présence de 2,12 g de tert-butanolate de potassium dans 10 mL de DMSO.
Rendement : 66 %
RMN 1H (CDCl3, 300 MHz) δ (ppm) : 9,48 (s élargi, 1H), 8,22 (d, 1H), 7,11-7,42 (massif aromatique, 1 1 H), 6,83 (m, 1 H),
HPLC : 96 %
SM : [M+Na]⁺ 345

### Stade 3 : N-[4-phénylsulfanyl-phényl]-benzène-1,2-diamine

Le produit (2 g) est obtenu par hydrogénation catalytique en utilisant 2,50 g du dérivé obtenu précédemment en présence de 250 mg de palladium sur charbon à 10% dans 30 mL d'un mélange éthanol/ acétate d'éthyle (1 : 1).
Rendement : 88 %
HPLC : 92 %
SM : MH⁺ 293

### Stade 4 : (4-Phénylsulfanyl-phényl)-(2-pipéridin-1-yl-phényl)-amine

Le produit (1,37 g) est obtenu selon le procédé du stade 3 de l'exemple 4, en utilisant 2 g du dérivé précédent comme produit de départ, 932 µL de dibromopentane et 2,86 mL de DIPEA dans 40 mL de toluène.
Rendement : 55 %
RMN 1H (CDCl3, 300 MHz) δ (ppm) : 6,87-7,41 (massif aromatique, 13H), 6,78 (s élargi, 1H), 2,84 (m, 4H), 1,74 (m, 4H), 1,60 (m, 2H)
HPLC : 98 %
SM : MH⁺ 361

### Stade 5: N-(4-phénylsulfanyl-phényl)-N-(2-pipéridin-1-yl-phényl)-hydrazine (s)

Le produit (300 mg) est obtenu selon le procédé du stade 4 de l'exemple 1, en utilisant 800 mg du dérivé précédent comme produit de départ et 888 mg de nitrite de sodium dans 5 mL d'acide acétique conduisant à l'intermédiaire nitroso qui est réduit par 316 mg d'hydrure de lithium et d'aluminium (4 équivalents) dans 5 mL de tétrahydrofurane.
Rendement : 36 %
RMN ¹H (CDC13, 300 MHz) δ (ppm) : 6,92-7,26 (massif aromatique, 13H), 4,71 (s élargi, 2H), 2,84 (m, 4H), 1,58 (m, 4H), 1,47 (m, 2H)
HPLC : 90 %
SM : MH⁺ 376

### Stade 6 : 5-Bromo-2-méthoxy-4-[N'-(4-phénylsulfanyl-phényl)-N'-(2-pipéridin-1-yl-phényl)-hydrazinocarbonylméthyl]-benzoate de méthyle

Le produit (340 mg) est obtenu selon le procédé du stade 3 de l'exemple 6, en utilisant 300 mg de l'hydrazine précédente comme substrat et 266 mg de l'acide de la préparation 2 comme co-substrat en présence de 168 mg d'EDCI et 118 mg de HOBt dans 2,5 mL de diméthylformamide.
Rendement : 64 %
RMN ¹H (CDCl3, 300 MHz) δ (ppm): 9,34 (s élargi, 1H), 7,99 (2s, 1H), 6,79-7,53 (massif aromatique, 14H), 3,70-3,92 (3s, 8H), 2,71 (m, 4H), 1,48 et 1,60 (2m, 6H)
HPLC : 91 %
SM : MH⁺ 660/662

### Stade 7 : Chlorhydrate d'acide 5-bromo-2-méthoxy-4-[N'-(4-phénylsulfanyl-phényl)-N'-(2-pipéridin-1-yl-phényl)-hydrazinocarbonylméthyl]-benzoïque (22)

Le produit (108 mg) est obtenu sous forme de chlorhydrate selon le procédé du stade 6 de l'exemple 2, en utilisant comme substrat le produit obtenu au stade précédent.
Rendement : 83 %
PF : 158-166 °C
Analyse élémentaire calculée pour C33H32BrN3O4S.1HCl.1H2O : C, 56,54 ; H, 5,03 ; N, 5,99. Trouvée : C, 56,59 ; H, 4,96 ; N, 5,84.
HPLC : 95 %
SM : MH⁺ 646/648

### Exemples 23 et 24: Chlorhydrate d'acide 4-[N'-(4-benzènesulfonyl-phényl)-N'-(2-pipéridin-1-yl-phényl)-hydrazinocarbonylméthyl]-5-bromo-2-méthoxy-benzoïque (23) et chlorhydrate d'acide 4-[N'-(4-benzènesulfinyl-phényl)-N'-(2-pipéridin-1-yl-phényl)-hydrazinocarbonylméthyl]-5-bromo-2-méthoxy-benzoïque (24)

### Stade 1 : 4-[N'-(4-benzènesulfonyl-phényl)-N'-(2-pipéridin-1-yl-phényl)-hydrazinocarbonylméthyl]-5-bromo-2-méthoxy-benzoate de méthyle et 4-[N'-(4-benzènesulfinyl-phényl)-N'-(2-pipéridin-1-yl-phényl)-hydrazinocarbonylméthyl]-5-bromo-2-méthoxy-benzoate de méthyle

A une solution de 100 mg du produit obtenu au stade 6 de l'exemple 22 dans 3 mL de dichlorométhane est additionné un excès d'acide *méta*chloroperbenzoïque (2 équivalents puis 2 autres équivalents au cours du temps) jusqu'à disparition totale du produit de départ (suivi CCM). Le brut réactionnel est filtré, le filtrat est lavé avec une solution saturée de sulfite de sodium puis bicarbonate de sodium. Les phases organiques réunies sont séchées sur sulfate de magnésium puis évaporées sous pression réduite pour conduire à un mélange qui est purifié par chromatographie sur gel de silice (éther de pétrole/acétate d'éthyle 60 :40). 35 mg sont obtenus de chacun des esters - forme sulfonyle et sulfynile respectivement -.
Rendement (sulfonyle) : 33 %
HPLC : 83 %
SM : MH⁺ 692/694
Rendement (sulfynile) : 34%
HPLC: 83 %
SM : MH⁺ 676/778

### Stade 2 : Chlorhydrate d'acide 4-[N'-(4-benzènesulfonyl-phényl)-N'-(2-pipéridin-1-yl-phényl)-hydrazinocarbonylméthyl]-5-bromo-2-méthoxy-benzoïque (23)

Le produit (20 mg) est obtenu sous forme de chlorhydrate selon le procédé du stade 6 de l'exemple 2, en utilisant comme substrat le dérivé sulfonyle obtenu au stade précédent.
Rendement : 55 %
Analyse élémentaire calculée pour C33H32BrN3O6S.0,75HCl : C, 56 ,15 ; H, 4,68 ; N, 5,95. Trouvée : C, 56,09 ; H, 4,65 ; N, 5,67.
HPLC: 84 %
SM : MH⁺ 678/680

### Stade 2 : Chlorhydrate d'acide 4-[N'-(4-benzènesulfinyl-phényl)-N'-(2-pipéridin-1-yl-phényl)-hydrazinocarbonylméthyl]-5-bromo-2-méthoxy-benzoïque (24)

Le produit (23 mg) est obtenu sous forme de chlorhydrate selon le procédé du stade 6 de l'exemple 2, en utilisant comme substrat la sulfinyle obtenu au stade précédent.
Rendement : 63 %
Analyse élémentaire calculée pour C33H32BrN3O5S.1HCl.1,5H2O : C, 54,59; H, 5,00 ; N, 5,79. Trouvée : C, 54,56 ; H, 4,91 ; N, 5,54.
HPLC : 85 %
SM : MH⁺ 662/664

### Exemple 25 : Chlorhydrate d'acide 2-méthoxy-4-{(E)-2-[N'-(4-méthoxy-phényl)-N'-(2-méthyl-6-pipéridin-1-yl-phényl)-hydrazinocarbonyl]-vinyl}-benzoïque (25)

### Stade 1 : 2-méthoxy-4-{(E)-2-[N'-(4-méthoxy-phényl)-N'-(2-méthyl-6-pipéridin-1-yl-phényl)-hydrazinocarbonyl]-vinyl}-benzoate de méthyle

Le produit (200 mg) est obtenu selon le procédé du stade 3 de l'exemple 6, en utilisant 150 mg de l'hydrazine de l'exemple 7 comme substrat et 125 mg de l'acide de la préparation 4 comme co-substrat en présence de 102 mg d'EDCI et 72 mg de HOBt dans 1,5 mL de diméthylformamide.
Rendement : 78 %
RMN ¹H (CDCl3, 300 MHz) δ (ppm) : 10,47 et 10,10 (2s élargis, 1H), 7,95 (m, 2H), 7,20-7,48 (m, 6H), 6,65-7,10 (m, 4H), 4,11 (2s, 6H), 3,98 (s, 3H), 2,91 et 3,11 (m, 3H), 2,61 (m, 4H), 1,85 (m, 6H)
HPLC : 98 %
SM : MH⁺ 530

### Stade 2 : Chlorhydrate d'acide 2-méthoxy-4-{(E)-2-(N'-(4-méthoxy-phényl)-N'-(2-méthyl-6-pipéridin-1-yl-phényl)-hydrazinocarbonyl]-vinyl}-benzoïque (25)

Le produit (149 mg) est obtenu sous forme de chlorhydrate selon le procédé du stade 6 de l'exemple 2, en utilisant comme substrat le produit obtenu au stade précédent.
Rendement : 71 %
Analyse élémentaire calculée pour C30H33N3O5.1HCl.1,5H2O : C, 62,22 ; H, 6,44; N, 7,26. Trouvée : C, 62,45 ; H, 6,34 ; N, 7,12.
HPLC : 94 %
SM : MH⁺ 516

### Exemple 26 : Chlorhydrate d'acide 5-bromo-2-méthoxy-4-{(E)-2-[N'-(4-méthoxy-phényl)-N'-(2-méthyl-6-pipéridin-1-yl-phényl)-hydrazinocarbonyl]-vinyl}-benzoïque (26)

### Stade 1 : 5-Bromo-2-méthoxy-4-{(E)-2-[N'-(4-méthoxy-phényl)-N'-(2-méthyl-6-pipéridin-1-yl-phényl)-hydrazinocarbonyl]-vinyl}-benzoate de méthyle

Le produit (234 mg) est obtenu selon le procédé du stade 3 de l'exemple 6, en utilisant 120 mg de l'hydrazine de l'exemple 7 comme substrat et 134 mg de l'acide de la préparation 5 comme co-substrat en présence de 81 mg d'EDCI et 57 mg de HOBt dans 2 mL de diméthylformamide.
Rendement : quantitatif
RMN ¹H (CDCl3, 300 MHz) δ (ppm) : 10,37 et 9,95 (2s élargis, 1H), 8,02 (m, 2H), 7,00-7,29 (m, 5H), 6,43-6,88 (m, 4H), 3,95 (3s, 9H), 2,70 et 2,90 (m, 3H), 2,40 (m, 4H), 1,60 (m, 6H)
HPLC : 87 %
SM: MH⁺ 608/610

### Stade 2 : Chlorhydrate d'acide 5-bromo-2-méthoxy-4-{(E)-2-[N'-(4-méthoxy-phényl)-N'-(2-méthyl-6-pipéridin-1-yl-phényl)-hydrazinocarbonyl]-vinyl}-benzoïque (26)

Le produit (217 mg) est obtenu sous forme de chlorhydrate selon le procédé du stade 6 de l'exemple 2, en utilisant comme substrat le produit obtenu au stade précédent.
Rendement : 86 %
PF : 192-206 °C
Analyse élémentaire calculée pour C30H32BrN3O5.1HCl.1H2O : C, 55,52 ; H, 5,44 ; N, 6,47. Trouvée : C, 55,43 ; H, 5,51 ; N, 6,37.
HPLC : 91 %
SM : MH⁺ 594/596

### Exemple 27: Chlorhydrate d'acide 4-(N'-(4-benzyl-phényl)-[N'-(2-méthyl-6-pipéridin-1-yl-phényl)-hydrazinocarbonylméthyl]-5-bromo-2-méthoxy-benzoïque (27)

### Stade 1 : (4-Benzyl-phényl)-(2-méthyl-6-nitro-phényl)-amine

Le produit (1,01 g) est obtenu selon le procédé du stade 1 de l'exemple 4, en utilisant 1,13 g de 2-fluoro-3-méthyl-nitrobenzène et 2 g de 4-benzylaniline en présence de 1,31 g de tert-butanolate de potassium dans 30 mL de DMSO.
Rendement estimé : 35 %
RMN ¹H (CDC13, 300 MHz) δ (ppm) : 8,32 (s élargi, 1H), 7,96 (d, 2H), 6,68-7,51 (massif aromatique, 10 H), 3,93 (s, 2H), 2,06 (s, 3H)
HPLC : 79 %
SM : MH⁺ 319

### Stade 2 : N²-(4-Benzyl-phényl)-3-méthyl-benzène-1,2-diamine

Le produit (530 mg) est obtenu par hydrogénation selon le procédé du stade 2 de l'exemple 13.
Rendement : 58 %
RMN ¹H (CDCl3, 300 MHz) δ (ppm) : 6,99-7,32 (massif aromatique, 8H), 6,68 (d, 2H), 6,52 (d, 2H), 3,89 (s, 2H), 2,18 (s, 3H)

### Stade 3 : (4-Benzyl-phényl)-(2-méthyl-6-pipéridin-1-yl-phényl)-amine

Le produit (460 mg) est obtenu selon le procédé du stade 3 de l'exemple 4, en utilisant 525 mg du dérivé précédent comme produit de départ, 247 µL de dibromopentane et 760 uL de DIPEA dans 10 mL de toluène.
Rendement : 71 %
RMN 1H (CDCl3, 300 MHz) δ (ppm) : 6,92-7,31 (massif aromatique, 10H), 6,62 (d, 2H), 6,17 (s élargi, 1H), 3,90 (s, 2H), 2,72 (m, 4H), 2,10 (s, 3H), 1,56 (m, 6H)
HPLC : 98 %
SM : MH⁺ 357

### Stade 4 : N-(4-Benzyl-phényl]-N-(2-méthyl-6-pipéridin-1-yl-phényl)-hydrazine (t)

Le produit (102 mg) est obtenu selon le procédé du stade 4 de l'exemple 1, en utilisant 450 mg du dérivé précédent comme produit de départ et 505 mg de nitrite de sodium dans 3 mL d'acide acétique conduisant à l'intermédiaire nitroso qui est réduit par 192 mg d'hydrure de lithium et d'aluminium (4 équivalents) dans 5 mL de tétrahydrofurane.
Rendement estimé : 22 %
RMN ¹H (CDCl3, 300 MHz) δ (ppm) : 7,12-7,29 (massif aromatique, 6H), 6,97 (dd, 4H), 6,75 (d, 2H), 3,88 (s, 2H), 2,80 (m, 4H), 2,08 (s, 3H), 1,52 (m, 6H)
HPLC : 64 %
SM : MH⁺ 372

### Stade 5 : 4-[N'-(4-Benzyl-phényl)-N'-(2-méthyl-6-pipéridin-1-yl-phényl)-hydrazinocarbonylméthyl]-5-bromo-2-méthoxy-benzoate de méthyle

Le produit (91 mg) est obtenu selon le procédé du stade 3 de l'exemple 6, en utilisant 101 mg de l'hydrazine précédente et 91 mg de l'acide de la préparation 2 en présence de 58 mg d'EDCI et 41 mg d'HOBt dans 4 mL de diméthylformamide.
Rendement : 51 %
RMN ¹H (CDCl3, 300 MHz) δ (ppm) : 9,97 et 9,79 (s élargis, 1H), 7,97 (s, 1H), 6,75-7,27 (massif aromatique, 11H), 6,51 (d, 2H), 3,87 (s, 6H), 3,72 (s, 4H), 2,74 (m, 2H), 2,37 (s, 3H), 2,36 (m, 2H), 1,40 (m, 6H)
HPLC: 96 %
SM : MH⁺ 656/658

### Stade 6 : Chlorhydrate d'acide 4-[N'-(4-benzyl-phényl)-N'-(2-méthyl-6-pipéridin-1-yl-phényl)-hydrazinocarbonylméthyl]-5-bromo-2-méthoxy-benzoïque (27)

Le produit (74 mg) est obtenu sous forme de chlorhydrate selon le procédé du stade 6 de l'exemple 2, en utilisant comme substrat le produit obtenu au stade précédent.
Rendement : 84 %
PF : 173-187 °C
Analyse élémentaire calculée pour C35H36BrN3O4.1HCl.1H2O: C, 60,31 ; H, 5,64 ; N, 6,03. Trouvée : C, 60,36 ; H, 5,62 ; N, 5,99.
HPLC : 98 %
SM : MH⁺ 642/644

### Exemple 28 : Chlorhydrate d'acide 5-bromo-2-méthoxy-4-[N'-(4'-méthoxy-biphényl-4-yl)-N'-(2-méthyl-6-pipéridin-1-yl-phényl)-hydrazino carbonylméthyl]-benzoïque (28)

### Stade 1 : (4-Bromo-phényl)-(2-méthyl-6-nitro-phényl)-amine

Le produit (2,44 g) est obtenu selon le procédé du stade 1 de l'exemple 4, en utilisant 1,8 g de 2-fluoro-3-méthyl-nitrobenzène et 3 g de 4-bromoaniline en présence de 2,09 g de tert-butanolate de potassium dans 20 mL de DMSO.
Rendement : 68 %
RMN 1H (CDCl3, 300 MHz) δ (ppm) : 8,15 (s élargi, 1H), 7,96 (d, 1H), 7,07-7,51 (massif aromatique, 4H), 6,61 (d, 2H), 2,09 (s, 3H)
HPLC : 90 %
SM : MH⁺ 307/309

### Stade 2 : N²-(4-Bromo-phényl)-3-méthyl-benzène-1,2-diamine

Le produit (2,2 g) est obtenu selon le procédé du stade 2 de l'exemple 1, en utilisant 2,44 g du dérivé précédent comme produit de départ et 9 g de chlorure d'étain hydraté dans 30 mL d'éthanol.
Rendement : quantitatif
RMN 1H (CDCl3, 300 MHz) δ (ppm) : 7,22 (s, 1H), 7,02 (t, 1H), 6,45 (m, 3H), 6,43 (d, 2H), 4,98 (s élargi, 1 H), 2,14 (s, 3H)
HPLC : 99 %
SM : MH⁺ 277/279

### Stade 3 : (4-Bromo-phényl)-(2-méthyl-6-pipéridin-1-yl-phényl)-amine

Le produit (2 g) est obtenu selon le procédé du stade 3 de l'exemple 4, en utilisant 2,2 g du dérivé précédent comme produit de départ, 1,08 mL de dibromopentane et 3,3 mL de DIPEA dans 40 mL de toluène.
Rendement : 73 %
RMN 1H (CDCl3, 300 MHz) δ (ppm) : 7,26 (m, 2H), 7,03 (t, 1H), 6,94 (d, 2H), 6,56 (d, 2H), 6,14 (s élargi, 1H), 2,70 (m, 4H), 2,10 (s, 3H), 1,55 (m, 6H)
HPLC : 99 %
SM : MH⁺ 345/347

### Stade 4 : (4'-Méthoxy-biphényl-4-yl)-(2-méthyl-6-pipéridin-1-yl-phényl)-amine

Le produit (248 mg) est obtenu selon le procédé du stade 4 de l'exemple 11, en utilisant 300 mg du dérivé précédent comme produit de départ, 198 mg d'acide phényl boronique, 50 mg de palladium tétrakis, 110 mg de chlorure de lithium en présence de 2,17 mL d'une solution 1 M de carbonate de calcium dans 6 mL d'un mélange méthanol/toluène (1 : 1).
Rendement : 76 %
RMN 1H (CDCl3, 300 MHz) δ (ppm) : 7,48 (d, 2H), 7,39 (d, 2H), 6,92-7,03 (m, 5H), 6,74 (d, 2H), 6,24 (s élargi, 1H), 3,84 (s, 3H), 2,74 (m, 4H), 2,16 (s, 3H), 1,56 (m, 6H) HPLC : 81 %
SM : MH⁺ 373

### Stade 5: N-(4'-Méthoxy-biphényl-4-yl)-N-(2-méthyl-6-pipéridin-1-yl-phényl)-hydrazine (u)

Le produit (92 mg) est obtenu selon le procédé du stade 4 de l'exemple 1, en utilisant 247 mg du dérivé précédent comme produit de départ et 265 mg de nitrite de sodium dans 3 mL d'acide acétique conduisant à l'intermédiaire nitroso qui est réduit par 101 mg d'hydrure de lithium et d'aluminium (4 équivalents) dans 5 mL de tétrahydrofurane à reflux.
Rendement : 35 %
RMN ¹H (CDCl3, 300 MHz) δ (ppm) : 7,48 (d, 2H), 7,38 (d, 2H), 7,17 (t, 1H), 6,86-7,03 (m, 6H), 4,90 (s élargi, 2H), 3,83 (s, 3H), 2,95 (m, 2H), 2,69 (m, 2H), 2,11 (s , 3H), 1,51-1,66 (m, 6H)
HPLC : 95 %
SM : MH⁺ 388

### Stade 6 : 5-Bromo-2-méthoxy-4-[N'-(4'-méthoxy-biphényl-4-yl)-N'-(2-méthyl-6-pipéridin-1-yl-phényl)-hydrazinocarbonylméthyl]-benzoate de méthyle

Le produit (24 mg) est obtenu selon le procédé du stade 3 de l'exemple 6, en utilisant 92 mg de l'hydrazine précédente et 79 mg de l'acide de la préparation 2 en présence de 50 mg d'EDCI et 35 mg d'HOBt dans 2,5 mL de diméthylformamide.
Rendement : 15 %
RMN ¹H (CDCl3, 300 MHz) δ (ppm) : 8,00 (s, 1H), 6,85-7,51 (massif aromatique, 10 H), 6,62 (d, 2H), 3,72-3,88 (m, 11H), 2,81 (m, 2H), 2,43 (m, 5H), 1,25-1,56 (m, 6H) HPLC : 87 %
SM : MH⁺ 672/674

### Stade 7 : Chlorhydrate d'acide 5-bromo-2-méthoxy-4-[N'-(4'-méthoxy-biphényl-4-yl)-N'-(2-méthyl-6-pipéridin-1-yl-phényl)-hydrazinocarbonylméthyl]-benzoïque (28)

Le produit (13 mg) est obtenu sous forme de chlorhydrate selon le procédé du stade 6 de l'exemple 2, en utilisant comme substrat le produit obtenu au stade précédent.
Rendement : 52 %
Analyse élémentaire calculée pour C35H36BrN3O5.1HCl.1,5H2O : C, 58,22 ; H, 5,58 ; N, 5,82. Trouvée : C, 58,06 ; H, 5,63 ; N, 5,58.
HPLC : 82 %
SM : MH⁺ 658/660

### Exemple 29 : Chlorhydrate d'acide 5-bromo-4-[N'-cyano-phényl)-N'-(2-méthyl-6-pipéridin-1-yl-phényl)-hydrazinocarbonylméthyl]-2-méthoxy benzoïque (29)

### Stade 1 : N'(2-Méthyl-6-nitro-phényl)-hydrazinocarboxylate de tert-butyle

A 250 mg de 2-fluoro-3-méthyl-nitrobenzène dans 5 mL de DMSO sont ajoutés 1,065 g de tert-butoxycarbonylhydrazine commerciale (5 équivalents). L'ensemble est porté à 100 °C pendant 10 minutes sous chauffage micro-ondes. Le milieu est hydrolyse puis extrait plusieurs fois à l'acétate d'éthyle. Les phases organiques sont réunies, séchées sur sulfate de magnésium, filtrées et évaporées sous pression réduite pour conduire à un résidu qui est purifié par chromatographie sur gel de silice (cyclohexane/ acétate d'éthyle : 80 /20). 342 mg de produit correspondant à l'attendu sont obtenus.
Rendement : 80 %
RMN ¹H (CDCl3, 250 MHz) δ (ppm) : 7,86 (d, 1H), 7,35 (d, 1H), 6,97 (t, 1H), 6,40 (s élargi, 1H), 1,34 (s élargi, 9H)
HPLC : 98 %

### Stade 2 : N'-(4-Cyano-phényl)-N'-(2-méthyl-6-nitro-phényl)-hydrazinocarboxylate de tert-butyle

A 1,5 g de produit obtenu au stade précédent dans 10 mL de dichlorométhane sont ajoutés à 0°C 2,45 g d'oxyde de manganèse activé (5 équivalents). L'ensemble est agité à température ambiante pendant 30 minutes, jusqu'à disparition complète du produit de départ (suivi CCM). L'intermédiaire oxydé est filtré sur célite, rincé au dichlorométhane et concentré sous pression réduite sans autre forme de purification. Le dérivé azocarboxylate de tert-butyle ainsi obtenu est immédiatement repris dans 10 mL de méthanol auxquels sont ajoutés successivement 1,27 g d'acide 4-cyanophényl boronique (1,6 équivalents) et 54 mg d'acétate de cuivre hydraté (0,05 équivalents). L'ensemble est porté à reflux pendant 24 heures jusqu'à disparition complète de l'intermédaire oxydé. Le milieu réactionnel est hydrolysé puis extrait à l'acéte d'éthyle plusieurs fois. Les phases organiques sont lavées à l'eau, puis à l'eau salée et enfin séchées sur sulfate de magnésium, filtrées et évaporées sous pression réduite. Le résidu obtenu est purifié par chromatographie sur gel de silice (dichlorométhane / cyclohexane : 1/1) pour conduire à 1,13 g du produit attendu.
Rendement : 57 %
RMN ¹H (CDC13, 300 MHz) δ (ppm) : 7,98 (d, 1H), 7,67 (t, 2H), 7,49 (m, 3H), 6,60 (d, 2H), 2,49 (s, 3H), 1,48 (s, 9H)
HPLC : 78 %

### Stade 3 : N'-(2-Amino-6-méthyl-phényl)-N'-(4-cyano-phényl)-hydrazino carboacylate de tert-butyle

982 mg de produit sont obtenus par hydrogénation catalytique en présence de 110 mg de palladium sur charbon à 5% dans 50 mL d'éthanol à partir de 1,13 g de produit obtenu au stade précédent.
Rendement : 97 %
RMN ¹H (CDCl3, 300 MHz) δ (ppm) : 7,48 (d, 2H), 7,09 (m, 1H), 6,62 (m, 4H), 4,78 (s élargi, 2H), 4,40 (s élargi, 1H), 2,02 (s, 3H), 1,50 (s, 9H)
HPLC : 80 %

### Stade 4 : N'-[2-(5-Bromo-pentanoylamino)-6-méthyl-phényl]-N'-(4-cyano-phényl)-hydrazinocarboxylate de tert-butyle

A 980 mg du produit obtenu au stade précédent dans 8 mL de dichlorométhane en présence de 820 µL de DIPEA (2 équivalents) sont ajoutés goutte à goutte et à température ambiante 388 µL de chlorure de 5-bromovaléryle. Après 20 minutes, le brut réactionnel hydrolysé par une solution d'acide chlorhydrique 1N est extrait au dichlorométhane plusieurs fois. Les phases organiques sont séchées sur sulfate de magnésium, filtrées et concentrées sous pression réduite pour conduire à 1,4 g de produit attendu.
Rendement (estimation) : 96 %
RMN ¹H (CDCl3, 300 MHz) δ (ppm) : 10,03 (s, 1H), 8,28 (d, 1H), 7,49 (d, 1H), 7,34 (t, 2H), 6,95-7,20 (m, 3H), 3,43 (m, 2H), 3,30 (t, 2H), 2,51 (t, 2H), 2,34 (m, 2H), 2,07 (s, 3H), 1,53 (s, 9H)
HPLC : 56 %

### Stade 5 : N'-(4-Cyano-phényl)-N'-[2-méthyl-6-(2-oxo-pipéridin-1-yl)-phényl]-hydrazinocarboxylate de tert-butyle

A 1,4 g de produit obtenu précédemment dans 5 mL de DMF sont ajoutés 225 mg d'hydrure de sodium (2 équivalents) à 0°C. Après 20 minutes à température ambiante, le brut réactionnel est hydrolysé puis extrait plusieurs fois à l'acétate d'éthyle. Les phases organiques réunies sont lavées à l'eau puis à l'eau salée et enfin séchées sur sulfate de magnésium, filtrées et concentrées sous pression réduite pour conduire à 1,1 g de produit attendu.
Rendement (estimation) : quantitatif

### S t a d e 6 : N'-[4-(Amino-méthyl)-phényl]-N'-(2-méthyl-6-pipéridin-1-yl-phényl)-hydrazinocarboxylate de tert-butyle

A 440 mg de produit obtenu au stade précédent dans 3 mL de THF sont ajoutés 493 µL de borane diméthylsulfure (5 équivalents). L'ensemble est porté à reflux pendant 1 heure. Le brut réactionnel est hydrolysé et extrait plusieurs fois à l'acétate d'éthyle. Les phases organiques sont réunies, lavées à l'eau puis à l'aide d'une solution d'acide chlorhydrique 1N. La phase aqueuse est reprise par une solution saturée de bicarbonate de sodium puis extraite à l'acétate d'éthyle. Les phases organiques sont réunies, séchées sur sulfate de magnésium, filtrées et concentrées sous pression réduite pour conduire à 308 mg de produit attendu.
Rendement (estimation) : 71 %

### Stade 7: N'-[4-(Acétylamino-méthyl)-phényl]-N'-(2-méthyl-6-pipéridin-1-yl-phényl)-hydrazinocarboxylate de tert-butyle

A une solution de 300 mg du produit obtenu au stade précédent dans 10 mL de tétrahydrofurane sous atmosphère inerte sont ajoutés 267 µL de DIPEA (2 équivalents) et 76 µL d'anhydride acétique (1,4 équivalents). L'ensemble est agité à température ambiante pendant 30 minutes jusqu'à disparition complète du produit de départ (suivi CCM). Le milieu réactionnel repris dans l'acétate d'éthyle est hydrolysé avec une solution d'acide chlorhydrique 1N et extrait plusieurs. La phase aqueuse est ensuite reprise par une solution de bicarbonate de sodium jusqu'à pH 8 et extraite à l'acétate d'éthyle. Les phases organiques sont réunies, séchées sur sulfate de magnésium, filtrées et évaporées sous pression réduite. Le résidu obtenu est purifié par chromatographie sur gel de silice (éther puis dichlorométhane) pour conduire à 165 mg de produit désiré.
Rendement (estimation) : 51 %
RMN ¹H (CDCl3, 300 MHz) δ (ppm) : 8,92 (s élargi, 1H), 7,18 (d, 1H), 7,07 (m, 4H), 6,62 (d, 2H), 5,60 (s élargi, 1H), 4,32 (m, 2H), 2,80 (m, 2H), 2,65 (m, 2H), 2,31 (s, 3H), 2,01 (s, 3H), 1,60 (m, 6H), 1,44 (s, 9H)

### Stade 8: 4-{N'-(4-(Acétylamino-méthyl)-phényl]-N'-(2-méthyl-6-pipéridin-1-yl-phényl)-hydrazinocarbonylméthyl}-5-bromo-2-méthoxy-benzoate de méthyle

A une solution de 51 mg d'acide obtenu dans la préparation 2 (2 équivalents) dans 2 mL de dichlorométhane sont ajoutés 14,4 µL de chlorurue d'oxylale (2 équivalents) et 1 goutte de diméthylformamide. L'ensemble est agité 20 minutes à température ambiante puis évaporé sous pression réduite. Au chlorure d'acide ainsi obtenu, reprise dans 1 mL d'acétonitrile, sont ajoutés successivement une solution de 38 mg de l'ester précédent dans 1 mL d'acétonitrile et 1 mL d'acide chlorhydrique 4N dans le dioxane. L'ensemble est placé 10 minutes sous chauffage micro-ondes à 100°C. Le milieu réactionnel est hydrolysé et extrait à l'acétate d'éthyle plusieurs fois. La phase aqueuse est ensuite basifiée par une solution de soude 1N puis extraite à l'acétate d'éthyle puis dichlorométhane. Les phases organiques sont réunies, séchées sur sulfate de magnésium, filtrées puis évaporées sous pression réduite. Une chromatographie sur gel de silice du résidu (dichlorométhane/éthanol : 98/2) permet d'obtenir 18 mg de produit attendu.
Rendement : 34 %
RMN ¹H (CDCl3, 300 MHz) δ (ppm) : 10,05 et 9,83 (2s élargis, 1H), 7,99 (s, 1H), 7,18 (m, 2H), 7,05 (m, 4H), 6,52 (d, 2H), 5,67 (s élargi, 1H), 4,29 (d, 2H), 3,88 (s, 3H), 3,73 (m,s, 2H), 3,71 (s, 3H), 2,75 (m, 2H), 2,40 (m, 2H), 2,37 (s, 3H), 1,98 (s, 3H), 1,46 (m, 6H)
HPLC : 97,3 %
SM : MH⁺ 637/639

### Stade 9 : Chlorhydrate d'acide 4-{N'-[4-(acétylamino-méthyl)-phényl]-N'-(2-méthyl-6-pipéridin-1-yl-phényl)-hydrazinocarbonylméthyl}-5-bromo-2-méthoxy-benzoïque (29)

Le produit (13,8 mg) est obtenu sous forme de chlorhydrate selon le procédé du stade 6 de l'exemple 2, en utilisant comme substrat le produit obtenu au stade précédent.
Rendement : 75 %
HPLC: 91 %
SM : MH⁺ 623/625

### Exemple 30 : Chlorhydrate d'acide 4-[N'-(4-benzoyl-phényl)-N'-(2-pipéridin-1-yl-phényl)-hydrazinocarbonylméthylJ-5-bromo-2-méthoxy benzoïque (30)

### Stade 1 : (4-Benzoyl-phényl)-(2-nitro-phényl)-amine

Le produit (770 mg) est obtenu selon le procédé du stade 1 de l'exemple 4, en utilisant 1,5 mL de 2-fluoro-nitrobenzène et 4,2 g de 4-aminobenzophénone en présence de 2,54 g de tert-butanolate de potassium dans 40 mL de DMSO.
Rendement : 17 %
HPLC : 86 %
SM : MH⁺ 319

### Stade 2 : N-(4-Benzoyl-phényl)-benzène-1,2-diamine

Le produit (880 mg) est obtenu selon le procédé du stade 2 de l'exemple 1, en utilisant 770 mg du dérivé précédent comme produit de départ et 2,73 g de chlorure d'étain hydraté dans 15 mL d'éthanol.
RMN 1H (CDCl3, 300 MHz) δ (ppm) : 7,74 (t, 4H), 7,36-7,53 (m, 3H), 7,13 (m, 2H), 6,70-6,85 (m, 4H), 5,63 (s élargi, 1H), 3,80 (s élargi, 1H)
HPLC : 82 %
SM : MH⁺ 289

### Stade 3 : (4-Benzoyl-phényl)-(2-pipéridin-1-yl-phényl)-amine

Le produit (530 mg) est obtenu selon le procédé du stade 3 de l'exemple 4, en utilisant 697 mg du dérivé précédent comme produit de départ, 330 µL de dibromopentane et 1,01 mL de DIPEA dans 15 mL de toluène.
Rendement : 62 %
RMN 1H (CDCl3, 300 MHz) δ (ppm) : 7,80 (dd, 4H), 7,45-7,56 (m, 4H), 6,96-7,18 (m, 6H), 2,84 (m, 4H), 1,60-1,74 (m, 6H)
HPLC: 100 %
SM : MH⁺ 357

### Stade 4 : N-(4-Benzoyl-phényl)-N-(2-pipéridin-1-yl-phényl)-hydrazine (v)

A une solution de 100 mg du composé précédent dans 10 mL de DMF sont ajoutés 14 mg d'hydrure de sodium (1,2 équivalents) et l'ensemble est agité à température ambiante pendant 45 minutes. 2,3 mL d'une solution à 0.15 M dans l'éther de monochloramine (1,2 équivalents) fraîchement préparée (J. Org. Chem. 2004, 69, 1368-1371) est ajoutée. Après 5 minutes, le milieu est traité par une solution saturée de Na2S2O3, repris à l'eau puis extrait à l'éther plusieurs fois. Les phases organiques sont séchées sur sulfate de magnésium, filtrées et concentrées sous pression réduite. Le résidu obtenu est purifié par chromatographie sur gel de silice (éther de pétrole/acétate d'éthyle : 95/5) pour conduire à 75 mg du produit attendu.
Rendement : 72 %
RMN ¹H (CDCl3, 250 MHz) δ (ppm) : 7,73 (d, 4H), 7,48 (m, 3H), 7,25 (m, 2H), 7,05-7,17 (m, 4H), 4,89 (s élargi, 2H), 2,91 (m, 4H), 1,57 (m, 6H)
HPLC: 100 %
SM : MH⁺ 372

### Stade 5 : 4-[N'-(4-Benzoyl-phényl)-N'-(2-pipéridin-1-yl-phényl)-hydrazinocarbonylméthyl]-5-bromo-2-méthoxy-benzoate de méthyle

Le produit (83 mg) est obtenu selon le procédé du stade 3 de l'exemple 6, en utilisant 70 mg de l'hydrazine précédente et 64 mg de l'acide de la préparation 2 en présence de 41 mg d'EDCI et 28 mg d'HOBt dans 1,5 mL de diméthylformamide.
Rendement : 66 %
RMN ¹H (CDCl3, 300 MHz) δ (ppm) : 9,36 (s élargi, 1H), 7,99 (s, 1H), 6,80-7,76 (massif aromatique, 14 H), 3,89 (s, 3H), 3,80 (s, 2H), 3,72 (s, 3H), 2,75 (m, 4H), 1,54 (m, 6H)
HPLC: 100 %
SM : MH⁺ 656/658

### Stade 6 : Chlorhydrate d'acide 4-[N'-(4-benzoyl-phényl)-N'-(2-pipéridin-1-yl-phényl)-hydrazinocarbonylméthyl]-5-bromo-2-méthoxy benzoïque (30)

Le produit (31 mg) est obtenu selon le procédé du stade 6 de l'exemple 2, en utilisant comme substrat le produit obtenu au stade précédent.
Rendement : 37 %
Analyse élémentaire calculée pour C34H32BrN3O5.1HCl.1,5H2O : C, 57,84 ; H, 5,14 ; N, 5,95. Trouvée : C, 58,04 ; H, 5,13 ; N, 5,55.
HPLC: 100 %
SM : MH⁺ 642/644

### Exemple 31 : Chlorhydrate d'acide 5-bromo-4-[N'-cyano-phényl)-N'-(2-méthyl-6-pipéridin-1-yl-phényl)-hydrazinocarbonylméthyl]-2-méthoxy benzoïque (31)

### Stade 1 : N'-(4-Cyano-phényl)-N'-(2-méthyl-6-pipérirlin-1-yl-phényl)-hydrazinocarboxylate de tert-butyle

A 280 mg de produit obtenu au stade 5 de l'exemple 29 dans 2 mL de tétrahydrofurane sont ajoutés 3,3 mL d'une solution 1M de borane dans le tétrahydrofurane (5 équivalents). L'ensemble est porté à reflux pendant 3 heures. Le brut réactionnel est versé sur une solution d'acide chlorhydrique 1N et extrait plusieurs fois à l'acétate d'éthyle. La phase aqueuse est basifiée par une solution de soude 1N et extraite au dichlorométhane. Les différentes phases organiques sont réunies, lavées à l'eau puis par une solution saturée de NaCl, séchées sur sulfate de magnésium, filtrées et concentrées sous pression réduite. Le résidu obtenu est purifié par chromatographie sur gel de silice (dichlrométhane/acétate d'éthyle : 98/2) pour conduire à 82 mg de produit attendu.
Rendement : 30 %
RMN ¹H (CDCl3, 300 MHz) δ (ppm) : 8,92 (s élargi, 1 H), 7,46 (d, 2H), 7,21 (m, 1 H), 7,06 (t, 2H), 6,72 (m, 2H), 2,74 (m, 4H), 2,29 (s, 3H), 1,60 (m, 6H), 1,43 (s, 9H)

### Stade 2 : 5-Bromo-4-[N'-(4-cyano-phényl)-N'-(2-méthyl-6-pipéridin-1-yl-phényl)-hydrazinocarbonylméthyl]-2-méthoxy-benzoate de méthyle

Le produit (32 mg) est obtenu selon le procédé du stade 8 de l'exemple 31, en utilisant 42 mg du produit obtenu au stade précédent comme substrat et 94 mg de l'acide de la préparation 2 comme co-substrat.
Rendement : 56 %
RMN ¹H (CDCl3, 300 MHz) δ (ppm) : 9,78 (s, 1H), 7,98 (s, 1H), 7,45 (d, 2H), 7,24 (m, 2H), 7,01 (m, 2H), 6,62 (m, 2H), 3,88 (s, 2H), 3,37 (s, 6H), 2,70 (m, 2H), 2,45 (m, 2H), 2,35 (s, 3H), 1,42 (m, 6H)
HPLC : 94 %
SM : MH⁺ 591/593

### Stade 3 : Chlorhydrate d'acide 5-bromo-4-[N'-(4-cyano-phényl)-N'-(2-méthyl-6-pipéridin-1-yl-phényl)-hydrazinocarbonylméthyl]-2-méthoxy-benzoïque (31)

Le produit (16,4 mg) est obtenu sous forme de chlorhydrate selon le procédé du stade 6 de l'exemple 2, en utilisant comme substrat le produit obtenu au satde précédent.
Rendement : 49 %
HPLC : 98 %
SM : MH⁺ 577/579

### Exemple 32 : Chlorhydrate d'acide 4-[N'-(4'-acétyl-biphényl-4-yl)-N'-(2-méthyl-6-pipéridin-1-yl-phényl)-hydrazinocarbonylméthyl]-5-bromo-2-méthoxy-benzoïque (32)

### Stade 1 : (4'-Acétyl-biphényl-4-yl)-(2-méthyl-6-pipéridin-1-yl-phényl)-amine

Le produit (1,35 g) est obtenu selon le procédé du stade 4 de l'exemple 11, en utilisant 2,6 g du dérivé obtenu au stade 3 de l'exemple 28 comme produit de départ, 2,48 g d'acide 4-acétyl-phényl boronique, 307 mg de [1,1'-bis(diphénylphosphino)ferrocène]dichloropalladium (II) et 4,58 g de fluorure de césium dans 150 mL de dioxane.
Rendement : 47 %
RMN 1H (CDCl3, 200 MHz) δ (ppm) : 7,98 (d, 2H), 7,65 (d, 2H), 7,51 (d, 2H), 7,03 (m, 3H), 6,76 (d, 2H), 6,29 (s élargi, 1H), 2,75 (m, 4H), 2,63 (s, 3H), 2,17 (s, 3H), 1,58 (m, 6H)
SM: MH⁺ 385

### Stade 2 : [4'-(2-Méthyl-[1,3]dithian-2-yl)-biphenyl-4-yl]-(2-méthyl-6-pipéridin-1-yl-phényl)-amine

A une solution de 788 mg du dérivé obtenu au stade précédent dans 14 mL de dichlorométhane sont ajoutés successivement 260 µL de propanedithiol (1,25 équivalents) et 380 µL de trifluorure de bore complexé au diéthyl éther. L'ensemble est agité 2 jours à température ambiante jusqu'à disparition du produit de départ. Le milieu réactionnel est versé sur une solution de soude 2 N puis extrait plusieurs fois au dichlorométhane. Les phases organiques sont lavées avec une solution saturée en chlorure de sodium, séchées sur sulfate de sodium, filtrées et concentrées sous pression réduite pour conduire à 970 mg de produit attendu.
Rendement : quantitatif
RMN 1H (CDCl3, 200 MHz) δ (ppm) : 7,96 (d, 2H), 7,58 (d, 2H), 7,48 (d, 2H), 7,03 (m, 3H), 6,77 (d, 2H), 6,26 (s élargi, 1H), 2,76 (m, 8H), 2,18 (s, 3H), 1,95 (m, 2H), 1,84 (s, 3H), 1,54 (m, 6H)

### Stade 3 : N-[4'-(2-Méthyl-[1,3]dithian-2-yl)-biphenyl-4-yl]-N-(2-méthyl-6-pipéridin-1-yl-phényl)-hydrazine (w)

Le produit (835 mg) est obtenu selon le procédé du stade 4 de l'exemple 1, en utilisant 970 mg du dérivé précédent comme produit de départ et 1,11 g de nitrite de sodium dans 6 mL d'acide acétique conduisant à l'intermédiaire nitroso qui est réduit par 8,2 mL d'une solution 1 M dans l'éther d'hydrure de lithium et d'aluminium (4 équivalents) dans 8 mL d'éther à reflux.
Rendement : 83 %
RMN ¹H (CDCl3, 200 MHz) δ (ppm) : 7,92 (d, 2H), 7,57 (d, 2H), 7,46 (d, 2H), 7,19 (d, 1H), 7,05 (t, 2H), 6,90 (d, 2H), 2,70-2,95 (m, 8H), 2,12 (s, 3H), 1,96 (m, 2H), 1,82 (s , 3H), 1,65 (m, 6H)

### Stade 4 : 5-Bromo-2-méthoxy-4{N'-[4'-(2-méthyl-[1,3]dithian-2-yl)-biphenyl-4-yl]-N'-(2-méthyl-6-pipéridin-1-yl-phényl)-hydrazinocarbonylméthyl}-benzoate de méthyle

Le produit (637 mg) est obtenu selon le procédé du stade 3 de l'exemple 6, en utilisant 600 mg de l'hydrazine précédente et 406 mg de l'acide de la préparation 2 en présence de 257 mg d'EDCI et 181 mg d'HOBt dans 4 mL de diméthylformamide.
Rendement : 67 %
RMN ¹H (CDCl3, 200 MHz) δ (ppm) : 9,87 (s élargi, 1H), 7,96 (m, 3H), 7,50 (m, 5H), 7,20 (d, 1H), 7,03 (m, 2H), 6,65 (d, 2H), 3,88 (s, 3H), 3,70 (2s, 5H), 2,75 (m, 8H), 2,44 (s, 3H), 1,97 (m, 2H), 1,81 (s, 3H), 1,25-1,55 (m, 6H)
SM : MH⁺ 774/776

### Stade 5 : 4-[N'-(4'-Acétyl-biphényl-4-yl)-N'-(2-méthyl-6-pipéridin-1-yl-phényl)-hydrazinocarbonylméthyl]- 5-bromo-2-méthoxy-benzoate de méthyle

A une solution de 30 mg du dérivé obtenu au stade précédent dans 100 µL d'un mélange tétrahydrofurane/eau (1 : 1) sont ajoutés successivement 17 mg d'oxyde de mercure (II) (2 équivalents) et 10 µL de trifluorure de bore complexé à l'éther (2 équivalents). L'ensemble est agité à température ambiante pendant 1 heure, jusqu'à disparition du produit de départ. Le milieu réactionnel est versé sur une solution de bicarbonate de soude 2 M puis extrait plusieurs fois à l'acétate d'éthyle. Les phases organiques sont lavées avec une solution saturée en chlorure de sodium, séchées sur sulfate de sodium, filtrées et concentrées sous pression réduite. Une chromatographie sur gel de silice du résidu (cyclohexane/ acétate d'éthyle : 70/30) permet d'isoler 19,5 mg du produit désiré.
Rendement : 73 %
RMN 1H (CDCl3, 200 MHz) δ (ppm) : 9,88 (s élargi, 1H), 7,97 (m, 3H), 7,62 (d, 2H), 7,48 (d, 2H), 7,21 (d, 1H), 7,03 (m, 2H), 6,67 (d, 2H), 3,89 (s, 3H), 3,77 (2s, 5H), 2,80 (m, 2H), 2,62 (s, 3H), 2,42 (m+s, 5H), 1,30-1,50 (m, 6H)
SM : MH⁺ 684/686

### Stade 6 : Chlorhydrate d'acide 4-[N'-(4'-acétyl-biphényl-4 yl)-N'-(2-méthyl-6-pipéridin-1-yl-phényl)-hydrazinocarbonylméthyl]-5-bromo-2-méthoxy-benzoïque (32)

Le produit (115 mg) est obtenu sous forme de chlorhydrate selon le procédé du stade 6 de l'exemple 2, en utilisant comme substrat le produit obtenu au stade précédent.
Rendement : 34 %
RMN ¹H (DMSO, 400 MHz) δ (ppm) : 7,99 (m, 3H), 7,37-7,81 (m, 7H), 7,37 (s, 1H), 6,78 (m, 2H), 4,24 (d, 1H), 4,03 (d, 1H), 3,79 (s, 3H), 3,20-3,60 (m, 4H), 2,57 (s, 3H), 2,28 (s, 3H), 1,51-1,86 (m, 6H)
HPLC : 96 %
SM : MH⁺ 670/672

### Exemple 33 : Chlorhydrate d'acide 5-bromo-2-méthoxy-4-[N'-(4'-méthoxy-2-méthyl-biphényl-4-yl)-N'-(2-méthyl-6-pipéridin-1-yl-phényl)-hydrazino carbonylméthyl]-benzoïque (33)

### Stade 1 : (4-Bromo-3-méthyl-phényl)-(2-méthyl-6-nitro-phényl)-amine

Le produit (3,90 g) est obtenu selon le procédé du stade 1 de l'exemple 4, en utilisant 2,78 g de 2-fluoro-3-méthyl-nitrobenzène et 5 g de 4-bromo-3-méthyl-aniline en présence de 3,22 g de tert-butanolate de potassium dans 70 mL de DMSO.
Rendement : 68 %
RMN 1H (CDCl3, 200 MHz) δ (ppm) : 8,15 (s élargi), 7,96 (d, 1H), 7,43 (d, 1H), 7,37 (m, 1H), 7,10 (t, 1H), 6,65 (m, 1H), 6,42 (dd, 1H), 2,33 (s, 3H), 2,10 (s, 3H)

### Stade 2 : N²-(4-Bromo-3-méthyl-phényl)-3-méthyl-benzène-1,2-diamine

Le produit (3,2 g) est obtenu selon le procédé du stade 2 de l'exemple 1, en utilisant 3,9 g du dérivé précédent comme produit de départ et 13,7 g de chlorure d'étain hydraté dans 60 mL d'éthanol.
Rendement : 90 %
RMN 1H (CDCl3, 200 MHz) δ (ppm) : 7,29 (d, 1H), 7,03 (t, 1H), 6,68 (d, 2H), 6,47 (d, 1H), 6,28 (dd, 1H), 4,94 (s élargi, 1H), 2,29 (s, 3H), 2,16 (s, 3H)

### Stade 3 : (4-Bromo-3-méthyl-phényl)-(2-méthyl-6-pipéridin-1-yl-phényl)-amine

Le produit (3,21 g) est obtenu selon le procédé du stade 3 de l'exemple 4, en utilisant 3,2 g du dérivé précédent comme produit de départ, 1,50 mL de dibromopentane et 4,30 mL de DIPEA dans 50 mL de toluène.
Rendement : 81 %
RMN 1H (CDCl3, 200 MHz) δ (ppm) : 7,28 (d, 1H), 7,04 (m, 3H), 6,58 (d, 1H), 6,41 (dd, 1H), 6,13 (s élargi, 1H), 2,72 (m, 4H), 2,32 (s, 3H), 2,11 (s, 3H), 1,57 (m, 6H)

### Stade 4 : (4'-Méthoxy-2-méthyl-biphényl-4-yl)-(2-méthyl-6-pipéridin-1-yl-phényl)-amine

Le produit (1,921 g) est obtenu selon le procédé du stade 4 de l'exemple 11, en utilisant 3 g du dérivé précédent comme produit de départ, 1,904 g d'acide 4-méthoxy-phényl boronique, 392 mg de palladium tétrakis, 1,062 g de chlorure de lithium en présence de 9 mL d'une solution 1 M de carbonate de sodium dans 60 mL d'un mélange méthanol/toluène (1 : 1).
Rendement : 60 %
RMN 1H (CDCl3, 200 MHz) δ (ppm) : 7,28 (d, 2H), 7,03 (m, 6H), 6,59 (m, 2H), 6,26 (s élargi, 1H), 3,86 (s, 3H), 2,77 (m, 4H), 2,25 (s, 3H), 2,20 (s, 3H), 1,63 (m, 6H)

### Stade 5 : N- (4'-Méthoxy-2-méthyl-biphényl-4-yl)-N- (2-méthyl-6-pipéridin-1-yl-phényl)-hydrazine (x)

Le produit (385 mg) est obtenu selon le procédé du stade 4 de l'exemple 1, en utilisant 490 mg du dérivé précédent comme produit de départ et 515 mg de nitrite de sodium dans 4 mL d'acide acétique conduisant à l'intermédiaire nitroso qui est réduit par 4,8 mL d'une solution 1 M dans l'éther d'hydrure de lithium et d'aluminium (4 équivalents) dans 4,5 mL d'éther à reflux.
Rendement : 80 %
RMN ¹H (CDCl3, 200 MHz) δ (ppm): 6,90-7,27 (massif aromatique, 10H), 6,78 (s élargi, 1H), 6,57 (d, 1H), 3,83 (s, 3H), 2,92 (m, 4H), 2,22 (s, 3H), 2,12 (s, 3H), 1,68 (m, 4H), 1,53 (m, 2H)

### S t a d e 6 : 5-Bromo-2-méthoxy-4-[N'-(4'-méthoxy-2-méthyl-biphényl-4-yl)-N'-(2-méthyl-6-pipéridin-1-yl-phényl)-hydrazinocarbonylméthyl]-benzoate de méthyle

Le produit (450 mg) est obtenu selon le procédé du stade 3 de l'exemple 6, en utilisant 381 mg de l'hydrazine précédente et 316 mg de l'acide de la préparation 2 en présence de 200 mg d'EDCI et 144 mg d'HOBt dans 3,8 mL de diméthylformamide.
Rendement : 69 %
RMN ¹H (CDCl3, 200 MHz) δ (ppm) : 10,06 et 9,84 (2 s élargis, 1H), 8,01 (s, 1H), 7,20 (m, 4H), 6,89-7,02 (m, 5H), 6,50 (s, 1H), 6,38 (d, 1H), 3,89 (s, 3H), 3,84 (s, 3H), 3,76 (m, 2H), 3,72 (s, 3H), 2,80 (m, 2H), 2,45 (m, 5H), 2,18 (s, 3H), 1,30-1,58 (m, 6H)

### Stade 7 : Chlorhydrate d'acide 5-bromo-2-méthoxy-4-[N'-(4'-méthoxy-2-méthyl-biphényl-4-yl)-N'-(2-méthyl-6-pipéridin-1-yl-phényl)-hydrazinocarbonylméthyl]-benzoïque (33)

Le produit (264 mg) est obtenu sous forme de chlorhydrate selon le procédé du stade 6 de l'exemple 2, en utilisant comme substrat le produit obtenu au stade précédent.
Rendement : 67 %
RMN ¹H (DMSO, 200 MHz) δ (ppm) : 11,79 (s élargi, 1H), 11,44 (s élargi, 1H), 7,96 (d, 1H), 7,81 (s, 1H), 7,70 (m, 2H), 7,34 (s, 1H), 7,23 (d, 2H), 6,99 (d, 1H), 6,75 (d, 2H), 6,44 (s, 1H), 6,42 (d, 1H), 4,10 (m, 2H), 3,78 (s, 6H), 3,40 (m, 4H), 2,27 (s, 3H), 2,19 (s, 3H), 1,53-1,86 (m, 6H)
HPLC : 98,5 %
SM : MH⁺ 672/674

### RESULTATS D'ACTIVITE BIOLOGIQUE

L'activité des molécules contre le virus du papillome peut être évaluée dans différents tests *in vitro* et cellulaires comme ceux décrits par Chiang et al. (1992), Proc. Natl. Acad. Sci. USA, 89 :5799-5803 ou encore par White et al. (2003), Journal of Biological Chemistry, 278 :26765-26772.

### Exemple 34 : études pharmacologiques des composés de l'invention dans des tests cellulaires de réplication de l'ADN viral des VPH

Ces tests mesurent la réplication de l'ADN génomique viral dans des cellules humaines. Ils reposent sur la co-transfection d'un vecteur rapporteur contenant une origine de réplication virale (ori) et de vecteurs d'expression codant pour les protéines E1 et E2 de VPH. Ils permettent de suivre l'ensemble des fonctions biologiques de E1 et E2 nécessaires à la réplication du génome des VPH.

Il a été construit un vecteur rapporteur 'réplicon' contenant l'origine de réplication virale de VPH11 / VPH6 (appelée aussi LCR qui portent des sites de fixation des protéines E1 et E2 du VPH) et le gène codant pour la luciférase de luciole sous le contrôle transcriptionnel du promoteur de SV40. Il a été vérifié que la présence de l'origine de réplication du VPH n'a aucun effet transcriptionnel sur l'expression du gène de la luciférase, ceci en présence ou en absence des protéines virales E1 ou de E2. La co-transfection de ce vecteur-réplicon et de vecteurs d'expression des protéines E1 et E2 de VPH conduit à une augmentation de l'activité luciférase dépendante de la présence de E1 et de E2 et traduit l'augmentation du nombre de vecteurs rapporteurs. Ceci est dû à l'activité des protéines virales E1 et E2 qui permettent la réplication, dans les cellules mammifères, de ce vecteur-réplicon contenant une origine de réplication virale.

Les composés chimiques ont été évalués pour leur activité inhibitrice de la réplication virale dépendante de E1 et E2 de VPH11 / VPH6 dans ces tests cellulaires en co-transfectant, dans des lignées de cellules humaines dérivées de cellules épithéliales de rein ou de carcinome cervical, le vecteur rapporteur-réplicon et des couples de vecteurs d'expression de E1 et E2 de VPH11 VPH6. Des doses variées des composés ont été incubées pendant 2 à 6 jours après la transfection dans le milieu cellulaire et l'activité luciférase a été déterminée à l'aide d'un luminométre afin d'évaluer l'IC₅₀ des composés sur la réplication du génome des VPH.

Tous les composés présentés dans les exemples ci-dessus inhibent la réplication dépendante de E1 et E2 de VPH11 / VPH6 dans les cellules avec une IC₅₀ inférieure à 20 µM. Les composés préférés sont ceux dont l'IC₅₀ pourrait être inférieure à 750 nM.

Des essais cellulaires complémentaires ont permis de montrer que les composés présentés dans les exemples ci-dessus inhibent l'interaction E1/E2 de VPH11 et de VPH6.

## Revendications

1. Composés de formule (1) : 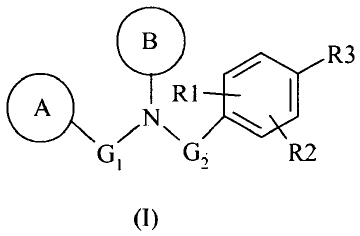 ainsi que leurs stéréoisomères,
dans laquelle :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
**G₂** représente un groupement 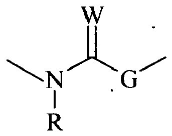 dans lequel :
• **R** représente un atome d'hydrogène, un groupement alkyle, halogénoalkyle, ou un radical pro-drogue tel que carbamate, acétyle, dialkylaminométhyle ou -CH₂-O-CO-Alk,
• G représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
• W représente un atome d'oxygène, de soufre ou NH,
**R₁** et **R₂,** identiques ou différents, représentent chacun un groupement choisi parmi un atome d'hydrogène, un atome d'halogène, un groupement hydroxyle, thio, alkoxy, halogénoalkoxy, alkylthio, halogénoalkylthio, amino, monoalkylamino, dialkylamino, cycloalkyle, alkyle ou halogénoalkyle,
**R₃** représente un groupement acide ou un radical pro-drogue de la fonction acide tel qu'ester, ou bien un bioisostère de la fonction acide tel que tétrazole, phosphonate, phosphonamide, sulfonate ou sulfonamide,
**A** représente un groupement aryle, cycloalkyle, cycloalkényle ou un hétérocycle, chacun éventuellement substitué, et
**B** représente un groupement aryle ou un hétérocycle à 6 chaînons, chacun éventuellement substitué,
ainsi que leurs sels pharmaceutiquement acceptables.

2. Composés selon la revendication 1, **caractérisés en ce que** A est substitué par un ou deux groupements, identiques ou différents, choisis parmi :
• un atome d'hydrogène, un atome d'halogène,
• un groupement alkoxy, alkylthio, halogénoalkoxy, halogénoalkylthio, hydroxyle, thio, cyano, amino, monoalkylamino ou dialkylamino,
• un groupement -SOnR', -COR', -CO₂R', -OCOR', -CONR'R", -NR'COR" ou -NR'SO₂R", dans lequel R' et R" représentent chacun indépendamment l'un de l'autre un atome d'hydrogène, un groupement alkyle, halogénoalkyle, et n vaut 1 ou 2,
• un groupement alkyle ou halogénoalkyle, le groupement alkyle étant éventuellement substitué par un groupement cyano, amino, monoalkylamino, dialkylamino ou acylamino,
• un groupement aryle, arylalkyle, -X-aryle, -X-arylalkyle ou -Alk-X-aryle, où X représente -O-, -NH-, -N(Alk)-, -N(COCH₃)-, -S-, -SO-, -SO₂-, -CO- ou-CONH-, chacun substitué sur la partie aryle par un ou deux substituants, identiques ou différents, choisis parmi :
✔ un atome d'hydrogène ou un atome d'halogène,
✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide,
✔ un groupement -SOnR', -COR', -CO₂R', -OCOR', -CONR'R", -NR'COR" ou -NR'SO₂R", dans lequel R' et R" représentent chacun indépendamment l'un de l'autre un atome d'hydrogène, un groupement alkyle, halogénoalkyle, et n vaut 1 ou 2,
• un groupement hétérocycle, -Alk-hétérocycle, -X-hétérocycle, -X-Alk-hétérocycle ou -Alk-X-hétérocycle, où X représente -O-, -NH-, -N(Alk)-, -N(COCH₃)-, -S-, -SO-, -SO₂-, -CO- ou -CONH-, chacun éventuellement substitué sur la partie hétérocycle par un ou deux substituants, identiques ou différents, choisis parmi :
✔ un atome d'hydrogène ou un atome d'halogène,
✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, ou
✔ un groupement -SOnR', -COR', -CO₂R', -OCOR', -CONR'R", -NR'COR" ou -NR'SO₂R", dans lequel R' et R" représentent chacun indépendamment l'un de l'autre un atome d'hydrogène, un groupement alkyle, halogénoalkyle, et n vaut 1 ou 2,
• un groupement cycloalkyle, -Alk-cycloalkyle, cycloalkényle, -Alk-cycloalkényle, -X-cycloalkyle, -X-Alk-cycloalkyle, -X-cycloalkényle, -X-Alk-cycloalkényle, -Alk-X-cycloalkyle, -Alk-X-cycloalkényle, où X représente -O-, -NH-, -N(Alk)-, -N(COCH₃)-, -S-, -SO-, -SO₂-, -CO- ou -CONH-, chacun éventuellement substitué sur la partie cyclique par un ou deux substituants, identiques ou différents, choisis parmi :
✔ un atome d'hydrogène ou un atome d'halogène,
✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide ou oxo, ou
✔ un groupement -SOnR', -COR', -CO₂R', -OCOR', -CONR'R", -NR'COR" ou -NR'SO₂R", dans lequel R' et R" représentent chacun indépendamment l'un de l'autre un atome d'hydrogène, un groupement alkyle, halogénoalkyle, et n vaut 1 ou 2,
et G₁, G₂, R₁, R₂, R₃ et B sont tels que définis à la revendication 1.

3. Composés selon la revendication 1 ou 2, **caractérisés en ce que B** est un aryle ou hétérocycle à 6 chaînons, substitué en position ortho par un groupement **R₄** et éventuellement substitué par un groupement **R₅,** dans lequel :
• **R₄** représente :
✔ un groupement alkyle, -NHAlk, -NAlkAlk', -NHcycloalkyle ou -NAlkcycloalkyle, Alk et Alk' étant identiques ou différents,
✔ un groupement cycloalkyle, cycloalkényle, N-cycloalkyle ou N-cycloalkényle, chacun éventuellement substitué par un ou deux substituants, identiques ou différents, choisis parmi un atome d'hydrogène, un atome d'halogène, un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, oxo ou -X-Aryle et où X représente -O-, -NH-, -N(Alk)-, -N(COCH₃)-, -S-, - SO-, -SO₂-, -CO- ou -CONH-, ou
✔ un groupement aryle éventuellement substitué par un ou deux groupements substituants, identiques ou différents, un atome d'hydrogène, un atome d'halogène, un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide ou -X-Aryle et où X représente -O-, -NH-, -N(Alk)-, -N(COCH₃)-, -S-, -SO-, - SO₂-, -CO- ou -CONH-,
• **R₅** représente :
✔ un atome d'hydrogène ou un atome d'halogène,
✔ un groupement hydroxyle, alkoxy, halogénoalkoxy, thio, alkylthio, halogénoalkylthio, amino, monoalkylamino, dialkylamino, -NHAcyle, cyano, acyle, acide, ester, amide, monoalkylamide ou dialkylamide,
✔ un groupement alkyle ou halogénoalkyle, le groupement alkyle pouvant être substitué un groupement cyano, hydroxyle, alkoxy, acide ou ester,
✔ un groupement -SOₙAlk, -SOₙNH₂, -SOₙNHAlk ou -SOₙNAlkAlk', où n vaut 1 ou 2 et Alk et Alk' sont identiques ou différents, ou
✔ un groupement pipéridine, oxopipéridine, morpholine ou bien un groupement pipérazine éventuellement substitué par un groupement alkyle ou acyle,
et **G₁, G₂, R₁, R₂, R₃** et A sont tels que définis à la revendication 1 ou 2.

4. Composés selon l'une quelconque des revendications 1 à 3, **caractérisés en ce que** :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
**G₂** représente un groupement 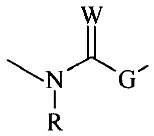 dans lequel:
• **R** représente un atome d'hydrogène, un groupement alkyle, halogénoalkyle, ou un radical pro-drogue tel que carbamate, acétyle, dialkylaminométhyle ou -CH₂-O-CO-Alk,
• G représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques, et
• W représente un atome d'oxygène, de soufre ou NH,
**R₁** et **R₂,** identiques ou différents, représentent chacun un groupement choisi parmi un atome d'hydrogène, un atome d'halogène, un groupement hydroxyle, thio, alkoxy, halogénoalkoxy, alkylthio, halogénoalkylthio, amino, monoalkylamino, dialkylamino, cycloalkyle, alkyle ou halogénoalkyle,
**R₃** représente un groupement acide ou un radical pro-drogue de la fonction acide tel qu'ester, ou bien un bioisostère de la fonction acide tel que tétrazole, phosphonate, phosphonamide, sulfonate ou sulfonamide,
**A** représente un groupement aryle ou un hétérocycle, chacun éventuellement substitué par un ou deux groupements, identiques ou différents, choisis parmi :
• un atome d'hydrogène, un atome d'halogène,
• un groupement alkoxy, alkylthio, halogénoalkoxy, halogénoalkylthio, hydroxyle, thio, cyano, amino, monoalkylamino ou dialkylamino,
• un groupement -SOnR', -COR', -CO₂R', -OCOR', -CONR'R", -NR'COR" ou -NR'SO₂R", dans lequel R' et R" représentent chacun indépendamment l'un de l'autre un atome d'hydrogène, un groupement alkyle, halogénoalkyle, et n vaut 1 ou 2,
• un groupement alkyle ou halogénoalkyle, le groupement alkyle étant éventuellement substitué par un groupement cyano, amino, monoalkylamino, dialkylamino ou acylamino,
• un groupement aryle, arylalkyle, -X-aryle, où X représente -O-, -NH-, -N(Alk)-, -N(COCH₃)-, -S-, -SO-, -SO₂-, -CO- ou -CONH-, chacun substitué sur la partie aryle par un ou deux substituants, identiques ou différents, choisis parmi :
✔ un atome d'hydrogène ou un atome d'halogène,
✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide, ou
✔ un groupement -SOnR', -COR', -CO₂R', -OCOR', -CONR'R", -NR'COR" ou -NR'SO₂R", dans lequel R' et R" représentent chacun indépendamment l'un de l'autre un atome d'hydrogène, un groupement alkyle, halogénoalkyle, et n vaut 1 ou 2,
• un groupement hétérocycle, -X-hétérocycle, où X représente -O-, -NH-, -N(Alk)-, -N(COCH₃)-, -S-, -SO-, -SO₂-, -CO- ou -CONH-, chacun éventuellement substitué sur la partie hétérocycle par un ou deux substituants, identiques ou différents, choisis parmi :
✔ un atome d'hydrogène ou un atome d'halogène,
✔ un groupement alkyle, halogénoalkyle, alkoxy, halogénoalkoxy, thio, alkylthio, halogénoalkylthio,hydroxyle, cyano, acyle, amino, monoalkylamino ou dialkylamino, acide, ester, amide, mono ou dialkylamide, ou
✔ un groupement -SOnR', -COR', -CO₂R', -OCOR', -CONR'R", - NR'COR" ou -NR'SO₂R", dans lequel R' et R" représentent chacun indépendamment l'un de l'autre un atome d'hydrogène, un groupement alkyle, halogénoalkyle, et n vaut 1 ou 2, ou
• un groupement cycloalkyle, cycloalkényle, -X-cycloalkyle, -X-cycloalkényle, où X représente -O-, -NH-, -N(Alk)-, -N(COCH₃)-, -S-, -SO-, -SO₂-, -CO- ou - CONH-, chacun éventuellement substitué sur la partie cyclique par un ou deux substituants, identiques ou différents, choisis parmi :
✔ un atome d'hydrogène ou un atome d'halogène,
✔ un groupement alkyle, halogénoalkyle, alkoxy, halogénoalkoxy, thio, alkylthio, halogénoalkylthio, hydroxyle, cyano, acyle, amino, monoalkylamino ou dialkylamino, acide, ester, amide, mono ou dialkylamide ou oxo, ou
✔ un groupement -SOnR', -COR', -CO₂R', -OCOR', -CONR'R", - NR'COR" ou -NR'SO₂R", dans lequel R' et R" représentent chacun indépendamment l'un de l'autre un atome d'hydrogène, un groupement alkyle, halogénoalkyle, et n vaut 1 ou 2, et
B représente un groupement phényle ou pyridine :
- substitué en position ortho par un groupement N-cycloalkyle tel que pipéridine ou par un cyclohexyle, chacun éventuellement substitué par un ou deux substituants, identiques ou différents, choisis parmi un atome d'hydrogène, un groupement alkyle, halogénoalkyle, alkoxy, halogénoalkoxy, -X-Aryle où X représente -O-, - NH-, -N(Alk)-, -N(COCH₃)-, -S-, -SO-, -SO₂-, -CO- ou -CONH-, et/ou
- éventuellement substitué par un atome d'halogène ou par un groupement alkyle ou halogénoalkyle.

5. Composés selon l'une quelconque des revendications 1 à 4, **caractérisés en ce que** :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
de préférence une liaison ou une chaîne hydrocarbonée comprenant 1 ou 2 atomes de carbone,
**G₂** représente un groupement 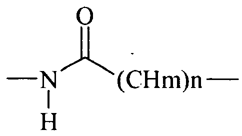 où n est un entier compris entre 1 et 4 et m est un entier valant 1 ou 2, de préférence n vaut 1 ou 2,
**R₁** représente un groupement alkoxy, tel que méthoxy, de préférence en position ortho par rapport à R₃,
**R₂** représente un atome d'hydrogène ou d'halogène, tel que chlore ou brome, ou un groupement alkyle, tel que méthyle, de préférence en position méta par rapport à R₃,
**R₃** représente un groupement acide ou ester,
**A** représente un groupement aryle, tel que phényle, substitué de préférence :
- en position méta ou para par :
• Un atome d'halogène ou un groupement alkyle, halogénoalkyle, cyano, alkoxy, halogénoalkoxy, acylaminoalkyle ou un groupement -XR où X représente -O-, - NH-, -N(Alk)-, -N(COCH₃)-, -S-, -SO-, -SO₂-, -CO- ou -CONH- et R représente un groupement arylalkyle, cycloalkyle ou aryle, chacun éventuellement substitué par un ou deux substituants, identiques ou différents, tels que atome d'halogène, groupement alkoxy, alkyle, halogénoalkyle, cyano, acyle, amino, monoalkylamino ou dialkylamino, acide, ester, amide, mono ou dialkylamide, ou un groupement -SOnR', -OCOR', -NR'COR" ou -NR'SO₂R", dans lequel R' et R" représentent chacun indépendamment l'un de l'autre un atome d'hydrogène, un groupement alkyle, halogénoalkyle, et n vaut 1 ou 2, ou
• Un groupement cycloalkyle, aryle, arylalkyle ou hétérocycle, de préférence N-cycloalkyle, chacun éventuellement substitué par un ou deux substituants, identiques ou différents, tels que atome d'halogène, groupement alkoxy, alkyle, halogénoalkyle, cyano, acyle, amino, monoalkylamino ou dialkylamino, acide, ester, amide, mono ou dialkylamide, ou un groupement -SOnR', -OCOR', - NR'COR" ou -NR'SO₂R", dans lequel R' et R" représentent chacun indépendamment l'un de l'autre un atome d'hydrogène, un groupement alkyle, halogénoalkyle, et n vaut 1 ou 2,
- et/ou en position ortho ou méta par un groupement alkyle, et
B représente un groupement aryle, de préférence un phényle,
• substitué en position ortho par un hétérocycle, de préférence un N-cycloalkyle, tel qu'un groupement pipéridine, et/ou
• substitué en position ortho' par un groupement alkyle, tel qu'un méthyle.

6. Composés selon l'une quelconque des revendications 1 à 5, **caractérisés en ce que** :
**G₁** représente une liaison ou une chaîne hydrocarbonée comprenant 1 à 4 atomes de carbone, linéaire ou ramifiée, saturée ou insaturée, éventuellement substituée par un ou deux groupements alkyle, de préférence identiques,
de préférence une liaison ou une chaîne hydrocarbonée comprenant 1 ou 2 atomes de carbone,
**G₂** représente un groupement 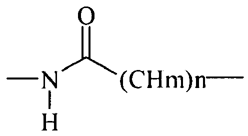 où n est un entier compris entre 1 et 4 et m est un entier valant 1 ou 2, de préférence n vaut 1 ou 2,
**R₁** représente un groupement alkoxy, tel que méthoxy, de préférence en position ortho par rapport à R₃,
**R₂** représente un atome d'hydrogène ou d'halogène, tel que chlore ou brome, ou un groupement alkyle, tel que méthyle, de préférence en position méta par rapport à R₃,
**R₃** représente un groupement acide ou ester,
**A** représente un groupement aryle, tel que phényle, substitué de préférence :
- en position méta ou para par :
• Un atome d'halogène ou un groupement cyano, alkoxy, halogénoalkoxy, acylaminoalkyle ou -XR où X représente -O-, -S-, -SO-, -SO₂- ou -CO- et R représente un groupement arylalkyle, cycloalkyle ou aryle, chacun éventuellement substitué par un ou deux substituants, identiques ou différents, tels que atome d'halogène, groupement alkoxy ou acyle, ou
• Un groupement cycloalkyle, aryle ou arylalkyle, chacun éventuellement substitué par un ou deux substituants, identiques ou différents, tels qu'un groupement acyle ou alkoxy, et
- et/ou en positio ortho ou méta par un groupement alkyle, et
**B** représente un groupement aryle, de préférence un phényle,
• substitué en position ortho par un hétérocycle, de préférence un N-cycloalkyle, tel qu'un groupement pipéridine, et/ou
• substitué en position ortho' par un groupement alkyle, tel qu'un méthyle.

7. Composés selon l'une quelconque des revendications 1 à 6, choisis dans le groupe suivant :
1) Chlorhydrate d'acide 5-bromo-2-méthoxy-4-[N'-(4-méthoxy-phényl)-N'-(2-pipéridin-1-yl-phényl)-hydrazinocarbonylméthyl]-benzoïque
2) Chlorhydrate d'acide 5-bromo-2-méthoxy-4-[N'-(2-pipéridin-1-yl-phényl)-N'-(4-trifluorométhoxy-phényl)-hydrazinocarbonylméthyl]-benzoïque
3) Chlorhydrate d'acide 5-bromo-2-méthoxy-4-[N'-(3-méthoxy-benzyl)-N'-(2-pipéridin-1-yl-phényl)-hydrazinocarbonylméthyl]-benzoïque
4) Chlorhydrate d'acide 4-[N'-(4-benzyloxy-phényl)-N'-(2-pipéridin-1-yl-phényl)-hydrazinocarbonylméthyl]-5-bromo-2-méthoxy-benzoïque
5) Chlorhydrate d'acide 5-bromo-4-{N'-[4-(4-fluoro-phénoxy)-phényl]-N'-(2-pipéridin-1-yl-phényl)-hydrazinocarbonylméthyl}-2-méthoxy-benzoïque
6) Chlorhydrate d'acide 5-bromo-2-méthoxy-4-{N'-[2-(4-méthoxy-phényl)-éthyl]-N'-(2-pipéridin-1-yl-phényl)-hydrazinocarbonylméthyl -benzoïque
7) Chlorhydrate d'acide 5-bromo-2-méthoxy-4-[N'-(4-méthoxy-phényl)-N'-(2-méthyl-6-pipéridin-1-yl-phényl)-hydrazinocarbonylméthyl]-benzoïque
7a) 5-Bromo-2-méthoxy-4-[N'-(4-méthoxy-phényl)-N'-(2-méthyl-6-pipéridin-1-yl-phényl)-hydrazinocarbonylméthyl]-benzoate de méthyle
8) Chlorhydrate d'acide 5-bromo-2-méthoxy-4-[N'-(4-méthoxy-benzyl)-N'-(2-pipéridin-1-yl-phényl)-hydrazinocarbonylméthyl]-benzoïque
9) Chlorhydrate d'acide 5-bromo-4-[N'-(4-cyclohexyl-phényl)-N'-(2-pipéridin-1-yl-phényl)-hydrazinocarbonylméthyl]-2-méthoxy-benzoïque
10) Chlorhydrate d'acide 5-bromo-2-méthoxy-4-[N'-(2-méthyl-6-pipéridin-1-yl-phényl)-N'-(4-trifluorométhoxy-phényl)-hydrazinocarbonylméthyl]-benzoïque
11) Chlorhydrate d'acide 5-bromo-2-méthoxy-4-[N'-(4'-méthoxy-biphényl-4-yl)-N'-(2-pipéridine-1-yl-phényl)-hydrazinocarbonylméthyl]-benzoïque
12) Chlorhydrate d'acide 5-bromo-4-[N'-(4-cyclohexyloxy-phényl)-N'-(2-pipéridin-1-yl-phényl)-hydrazinocarbonylméthyl]-2-méthoxy-benzoïque
13) Chlorhydrate d'acide 5-bromo-2-méthoxy-4-[N'-(4-phénoxy-phényl)-N'-(2-pipéridin-1-yl-phényl)-hydrazinocarbonylméthyl]-benzoïque
14) Chlorhydrate d'acide 5-bromo-4-{N'-[4-(4-chloro-phénoxy)-phényl]-N'-(2-pipéridin-1-yl-phényl)-hydrazinocarbonylméthyl}-2-méthoxy-benzoïque
15) Chlorhydrate d'acide 4-{N'-[4-(4-fluoro-phénoxy)-phényl]-N'-(2-méthyl-6-pipéridin-1-yl-phényl)-hydrazinocarbonylméthyl}-2-méthoxy-benzoïque
16) Chlorhydrate d'acide 5-bromo-4-{N'-[4-(4-fluoro-phénoxy)-phényl]-N'-(2-méthyl-6-pipéridin-1-yl-phényl)-hydrazinocarbonylméthyl}-2-méthoxy-benzoïque 16a) 5-Bromo-4-{N'-[4-(4-fluoro-phénoxy)-phényl]-N'-(2-méthyl-6-pipéridin-1-yl-phényl)-hydrazinocarbonylméthyl}-2-méthoxy-benzoate de méthyle
17) Chlorhydrate d'acide 4-[N'-(4-benzyl-phényl)-N'-(2-pipéridin-1-yl-phényl)-hydrazinocarbonylméthyl]-5-bromo-2-méthoxy-benzoïque
18) Chlorhydrate d'acide 4-[N'-(4-bromo-phényl)-N'-(2-pipéridin-1-yl-phényl)-hydrazinocarbonylméthyl]-5-chloro-2-méthoxy-benzoïque
19) Chlorhydrate d'acide 4-[N'-(3'-acétyl-biphényl-4-yl)-N'-(2-pipéridin-1-yl-phényl)-hydrazinocarbonylméthyl]-5-chloro-2-méthoxy-benzoïque
20) Chlorhydrate d'acide 4-[N'-(4'-acétyl-biphényl-4-yl)-N'-(2-pipéridin-1-yl-phényl)-hydrazinocarbonylméthyl]-5-chloro-2-méthoxy-benzoïque
21) Chlorhydrate d'acide 5-bromo-2-méthoxy-4-[N'-(3-phénoxy)-phényl]-N'-(2-pipéridin-1-yl-phényl)-hydrazinocarbonylméthyl]-benzoïque
22) Chlorhydrate d'acide 5-bromo-2-méthoxy-4-[N'-(4-phénylsulfanyl)-phényl]-N'-(2-pipéridin-1-yl-phényl)-hydrazinocarbonylméthyl]-benzoïque
23) Chlorhydrate d'acide 4-[N'-(4-benzènesulfonyl-phényl)-N'-(2-pipéridin-1-yl-phényl)- hydrazinocarbonylméthyl]-5-bromo-2-méthoxy-benzoïque
24) Chlorhydrate d'acide 4-[N'-(4-benzènesulfinyl-phényl)-N'-(2-pipéridin-1-yl-phényl)-hydrazinocarbonylméthyl]-5-bromo-2-méthoxy-benzoïque
25) Chlorhydrate d'acide 2-méthoxy-4-{(E)-2-[N'-(4-méthoxy-phényl)-N'-(2-méthyl-6-pipéridin-1-yl-phényl)-hydrazinocarbonyl]-vinyl}-benzoïque
26) Chlorhydrate d'acide 5-bromo-2-méthoxy-4-{(E)-2-[N'-(4-méthoxy-phényl)-N'-(2-méthyl-6-pipéridin-1-yl-phényl)-hydrazinocarbonyl]-vinyl}-benzoïque
27) Chlorhydrate d'acide 4-[N'-(4-benzyl-phényl)-N'-(2-méthyl-6-pipéridin-1-yl-phényl)-hydrazinocarbonylméthyl]-5-bromo-2-méthoxy-benzoïque
28) Chlorhydrate d'acide 5-bromo-2-méthoxy-4-[N'-(4'-méthoxy-biphényl-4-yl)-N'-(2-méthyl-6-pipéridin-1-yl-phényl)-hydrazinocarbonylméthyl]-benzoïque
29) Chlorhydrate d'acide 4-[N'-(acétylamino-méthyl)-phényl]-N'-(2-méthyl-6-pipéridin-1-yl-phényl)-hydrazinocarbonylméthyl]-5-bromo-2-méthoxy-benzoïque
30) Chlorhydrate d'acide 4-[N'-(4-benzoyl-phényl]-N'-(2-pipéridin-1-yl-phényl)-hydrazinocarbonylméthyl]-5-bromo-2-méthoxy-benzoïque
31) Chlorhydrate d'acide 5-bromo-4-[N'-(4-cyano-phényl)-N'-(2-méthyl-6-pipéridin-1-yl-phényl)-hydrazinocarbonylméthyl]-2-méthoxy-benzoïque
32) Chlorhydrate d'acide 4-[N'-(4'-acétyl-biphényl-4-yl)-N'-(2-méthyl-6-pipéridin-1-yl-phényl)-hydrazinocarbonylméthyl]-5-bromo-2-méthoxy-benzoïque
33) Chlorhydrate d'acide 5-bromo-2-méthoxy-4-[N'-(4'-méthoxy-2-méthyl-biphényl-4-yl)-N'-(2-méthyl-6-pipéridin-1-yl-phényl)-hydrazinocarbonylméthyl]-benzoïque.

8. Intermédiaires pour la synthèse des composés selon la revendication 7 choisis dans le groupe suivant :
a) *N*-(4-Méthoxy-phényl)-*N*-(2-pipéridin-1-yl-phényl)-hydrazine
b) *N*-(2-Pipéridin-1-yl-phényl)-*N*-(4-trifluorométhoxy-phényl)-hydrazine
c) *N-*(3-Méthoxy-benzyl)-*N-*(2-pipéridin-1-yl-phényl)-hydrazine
d) *N*-(4-Benzyloxy-phényl)-*N*-(2-pipéridin-1-yl-phényl)-hydrazine
e) *N*-[4-(4-Fluoro-phénoxy)-phényl]-*N*-(2-pipéridin-1-yl-phényl)-hydrazine
f) *N*-[2-(4-Méthoxy-phényl)-éthyl]-*N*-(2-pipéridin-1-yl-phényl)-hydrazine
g) *N-*(4-Méthoxy-phényl)-*N-*(2-méthyl-6-pipéridin-1-yl-phényl)-hydrazine
h) *N*-(4-Méthoxy-benzyl)-*N*-(2-pipéridin-1-yl-phényl)-hydrazine
i) *N*(4-Cyclohexyl-phényl)-*N-*(2-pipéridin-1-yl-phényl)-hydrazine
j) *N-*(2-Méthyl-6-pipéridin-1-yl-phényl)-*N-*(4-trifluorométhoxy-phényl)-hydrazine
k) *N*-(4'-Méthoxy-biphényl-4-yl)-*N*-(2-pipéridin-1-yl-phényl)-hydrazine
l) *N*-(4-Cyclohexyloxy-phényl)-*N*-(2-pipéridin-1-yl-phényl)-hydrazine
m) *N-*(4-Phénoxy-phényl)-*N-*(2-pipéridin-1-yl-phényl)-hydrazine
n) *N-*[4-(4-Chloro-phénoxy)-phényl]-*N*-(2-pipéridin-1-yl-phényl)-hydrazine
o) *N*-[4-(4-Fluoro-phénoxy)-phényl]-*N*-(2-méthyl-6-pipéridin-1-yl-phényl)-hydrazine
p) *N-*(4-Benzyl-phényl]-*N-*(2-pipéridin-1-yl-phényl)-hydrazine
q) *N-*(4-Bromo-phényl)-*N-*(2-pipéridin-1-yl-phényl)-hydrazine
r) *N*-(3-Phénoxy-phényl)-*N*-(2-pipéridin-1-yl-phényl)-hydrazine
s) *N-*(4-Phénylsulfanyl-phényl)-*N-*(2-pipéridin-1-yl-phényl)-hydrazine
t) *N*-(4-Benzyl-phényl)-*N*-(2-méthyl-6-pipéridin-1-yl-phényl)-hydrazine
u) *N*-(4'-Méthoxy-biphényl-4-yl)-*N-*(2-méthyl-6-pipéridin-1-yl-phényl)-hydrazine
v) *N-*(4-Benzoyl-phényl)-*N-*(2-pipéridin-1-yl-phényl)-hydrazine
w) *N*-[4'-(2-Méthyl-[1,3]dithian-2-yl)-biphenyl-4-yl]-*N*-(2-méthyl-6-pipéridin-1-yl-phényl)-hydrazine
x) *N*-(4'-Méthoxy-2-méthyl-biphényl-4-yl)-*N*-(2-méthyl-6-pipéridin-1-yl-phényl)-hydrazine.

9. Procédé de préparation des composés de formule (I) selon l'une quelconque des revendications 1 à 7, où G₂ représente le radical 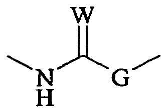 tel que défini dans la revendication 1,
**caractérisé en ce qu'**on effectue les étapes suivantes :
1) Réaction de la fonction hydrazine d'un composé de formule (II) 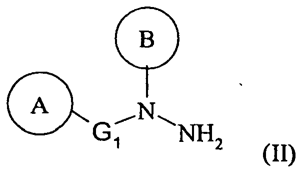 dans laquelle A, B et G₁ sont tels que définis à la revendication 1, avec la fonction acide d'un composé de formule (III) 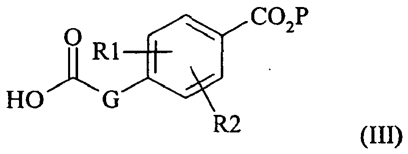 dans laquelle R₁, R₂ et G sont tels que définis à la revendication 1 et P représente un groupe protecteur de fonction acide,
pour obtenir le composé de formule (IV) 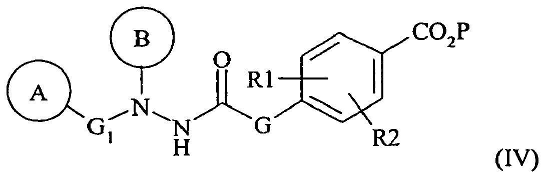
2) Déprotection du groupement -CO₂P du composé de formule (IV) par hydrolyse, pour obtenir le composé de formule (I) 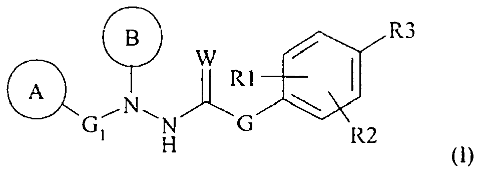 dans laquelle W représente un atome d'oxygène et R₃ est tel que défini à la revendication 1,
3) Le cas échéant, réaction du composé de formule (1) obtenu à l'étape 2) ou bien du composé de formule (IV) obtenu à l'étape 1) avec un réactif de Lawesson, ce qui permet d'obtenir un composé de formule (I) dans laquelle W représente un atome de soufre.

10. Procédé de préparation des composés de formule (I) selon l'une quelconque des revendications 1 à 7, où G₂ représente le radical 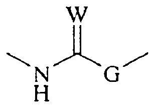 tel que défini dans la revendication 1,
**caractérisé en ce qu'**on effectue les étapes suivantes :
1) Réaction dans un milieu acide du composé de formule (V) : 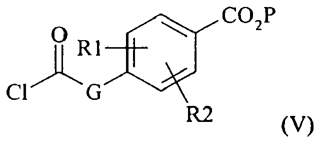 dans laquelle R₁, R₂ et G sont tels que définis à la revendication 1 et P représente un groupe protecteur de fonction acide, tel que (C₁-C₄)alkyle linéaire ou ramifié,
sur le composé de formule (XXIV) : 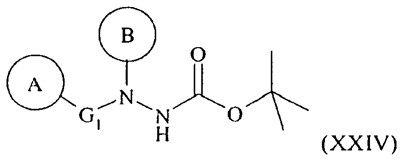 dans laquelle A, B et G₁, sont tels que définis dans la revendication 1,
pour obtenir le composé de formule (IV) : 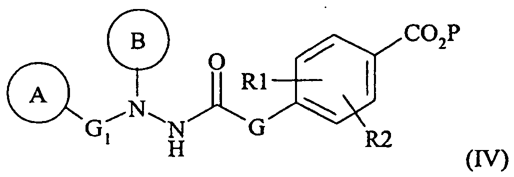
2) Deprotection du groupement -CO₂P du composé de formule (IV) par hydrolyse, pour obtenir le composé de formule (I) : 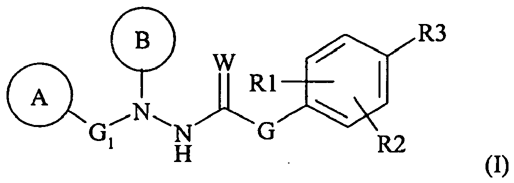
dans laquelle W représente un atome d'oxygène et R₃ est tel que défini à la revendication 1.

11. Composition pharmaceutique comprenant au moins un composé de formule (I) selon l'une quelconque des revendications 1 à 7, en association avec un excipient pharmaceutiquement acceptable.

12. Composé de formule (I) selon l'une quelconque des revendications 1 à 7 pour son utilisation comme médicament.

13. Utilisation d'un composé de formule (I) selon l'une quelconque des revendications 1 à 7 pour la préparation d'un médicament destiné à traiter ou prévenir une infection par le virus du papillome.

14. Utilisation selon la revendication 13, pour le traitement ou la prévention des lésions et maladies associées aux infections par le virus du papillome.

15. Utilisation selon la revendication 13 ou 14, pour le traitement ou la prévention des verrues ano-génitales, comme les condylomes acuminés et condylomes plans, les papillomes laryngés, conjonctivaux ou buccaux et d'autres lésions épithéliales, comme des papillomatoses récurrentes respiratoires et des néoplasies intra-épithéliales de bas grade et de haut grande, des papuloses bowénoïdes, des verrues (vulgaires, plantaires, myrmécies, superficielles, plates...), des épidermodysplasies verruciformes, des carcinomes, en particulier ano-génitaux, et toutes les lésions qui sont associées au virus du papillome.

## Patentansprüche

1. Verbindungen der Formel (I): 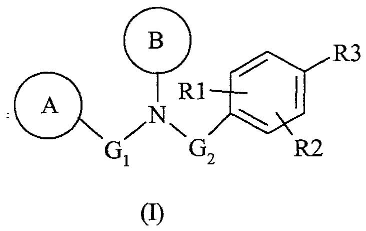 sowie deren Stereoisomere,
wobei
**G₁** eine Bindung oder eine gesättigte oder ungesättigte, verzweigte oder lineare Kohlenwasserstoffkette mit 1-4 Kohlenstoffatomen repräsentiert, ggf. substituiert durch eine oder zwei Alkylgruppen, die bevorzugt identisch sind,
**G₂** eine Gruppe 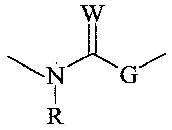 repräsentiert, wobei:
• R ein Wasserstoffatom, eine Alkylgruppe, eine Halogenalkylgruppe oder ein Prodrug-Radikal, wie z.B. Carbamat, Acetyl, Dialkylaminomethyl oder -CH₂-O-CO-Alk, repräsentiert,
• **G** eine Bindung oder eine gesättigte oder ungesättigte, lineare oder verzweigte Kohlenwasserstoffkette mit 1-4 Kohlenstoffatomen repräsentiert, ggf. substituiert durch ein oder zwei Alkylgruppen, die bevorzugt identisch sind,
• **W** ein Sauerstoffatom, Schwefelatom oder NH repräsentiert,
**R₁** und **R₂,** identisch oder verschieden, jeweils eine Gruppe repräsentieren, die ausgewählt ist aus einem Wasserstoffatom, einem Halogenatom, einer Hydroxyl-, Thio-, Alkoxy-, Halogenalkoxy-, Alkylthio-, Halogenalkylthio-, Amino-, Monoalkylamino-, Dialkylamino-, Cycloalkyl-, Alkyl- oder Halogenalkylgruppe,
**R₃** eine Säuregruppe oder ein Prodrug-Radikal der Säurefunktion, wie z.B. Ester, oder auch ein Bioisoster der Säurefunktion, wie z.B. Tetrazol, Phosphonat, Phosphonamid, Sulfonat oder Sulfonamid, repräsentiert,
**A** eine Aryl-, Cycloalkyl-, Cycloalkenyl-Gruppe oder einen Heterozyklus repräsentiert, jeweils ggf. substituiert, und
**B** eine Arylgruppe oder einen 6-gliedrigen Heterozyklus repräsentiert, jeweils ggf. substituiert,
sowie deren pharmazeutisch akzeptable Salze.

2. Verbindungen nach Anspruch 1, **dadurch gekennzeichnet, dass A** durch eine oder zwei identische oder verschiedene Gruppen substituiert ist, die ausgewählt sind aus:
• einem Wasserstoffatom, einem Halogenatom,
• einer Alkoxy-, Alkylthio-, Halogenalkoxy-, Halogenalkylthio-, Hydroxyl-, Thio-, Cyano-, Amino-, Monoalkylamino- oder Dialkylaminogruppe,
• einer -SOₙR', -COR', -CO₂R', -OCOR', -CONR'R", -NR'COR" oder -NR'SO₂R"-Gruppe, wobei R' und R" jeweils unabhängig voneinander ein Wasserstoffatom, eine Alkylgruppe oder eine Halogenalkylgruppe repräsentieren, und wobei n gleich 1 oder 2 ist,
• einer Alkyl- oder Halogenalkylgruppe, wobei die Alkylgruppe ggf. substituiert ist durch eine Cyano-, Amino-, Monoalkylamino-, Dialkylamino- oder Acylamino-Gruppe,
• einer Aryl-, Arylalkyl-, -X-Aryl-, -X-Arylalkyl- oder -Alk-X-Aryl-Gruppe, wobei X -O-, -NH-, -N(Alk)-, -N(COCH₃)-, -S-, -SO-, -SO₂-, -CO- oder -CONH- repräsentiert, jeweils substituiert im Arylbereich durch einen oder zwei identische oder verschiedene Substituenten, ausgewählt aus:
✔ einem Wasserstoffatom oder einem Halogenatom,
✔ einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester-, Amid-, Mono- oder Dialkylamid-Gruppe,
✔ einer -SOₙR', -COR', -CO₂R', -OCOR', -CONR'R",-NR'COR" oder -NR'SO₂R"-Gruppe,
wobei R' und R" jeweils unabhängig voneinander ein Wasserstoffatom, eine Alkylgruppe oder eine Halogenal-kylgruppe repräsentieren, und wobei n gleich 1 oder 2 ist,
• einer Heterozyklus-, -Alk-Heterozyklus-, -X-Heterozyklus-, -X-Alk-Heterozyklus- oder -Alk-X-Heterozyklus-Gruppe, wobei X-O-, -NH-, -N(Alk)-, -N(COCH₃)-, -S-, -SO-, -SO₂-, -CO- oder -CONH- repräsentiert, jeweils ggf. substituiert im Heterozyklusbereich durch einen oder zwei gleiche oder verschiedene Substituenten, ausgewählt aus:
✔ einem Wasserstoffatom oder einem Halogenatom,
✔ einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester-, Amid-, Mono- oder Dialkylamid-Gruppe, oder
✔ einer -SOₙR', -COR', -CO₂R', -OCOR', -CONR'R", - NR'COR" oder -NR'SO₂R"-Gruppe,
wobei R' und R" jeweils unabhängig voneinander ein Wasserstoffatom, eine Alkylgruppe oder eine Halogenal-kylgruppe repräsentieren, und wobei n gleich 1 oder 2 ist,
• einer Cycloalkyl-, -Alk-Cycloalkyl-, Cycloalkenyl-, -Alk-Cyclo-alkenyl-, -X-Cycloalkyl-, -X-Alk-Cycloalkyl-, -X-Cycloalkenyl-,-X-Alk-Cycloalkenyl-, -Alk-X-Cycloalkyl- oder -Alk-X-Cycloalke-nyl-Gruppe, wobei X -O-, -NH-, -N(Alk)-, -N(COCH₃)-, -S-,-SO-, -SO₂-, -CO- oder -CONH- repräsentiert, jeweils ggf. substituiert im zyklischen Bereich durch einen oder zwei gleiche oder verschiedene Substituenten, ausgewählt aus:
✔ einem Wasserstoffatom oder einem Halogenatom,
✔ einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester-, Amid-, Mono- oder Dialkylamid-Gruppe oder Oxo oder,
✔ einer -SOₙR', -COR', -CO₂R', -OCOR', -CONR'R",-NR'COR" oder -NR'SO₂R"-Gruppe,
wobei R' und R" jeweils unabhängig voneinander ein Wasserstoffatöm, eine Alkylgruppe oder eine Halogenal-kylgruppe repräsentieren, und wobei n gleich 1 oder 2 ist,
und G₁, G₂, R₁, R₂, R₃ und B wie im Anspruch 1 definiert sind.

3. Verbindungen nach Anspruch 1 oder 2, **dadurch gekennzeichnet, dass B** ein Aryl oder ein 6-gliedriger Heterozyklus ist, substituiert in der Ortho-Position durch eine Gruppe **R₄,** und ggf. substituiert durch eine Gruppe **R₅,** wobei:
• **R₄** repräsentiert:
✔ eine Alkyl-, -NHAlk-, -NAlkAlk'-, -NHCycloalkyl- oder-NAlkCycloalkyl-Gruppe, wobei Alk and Alk' gleich oder verschieden sind,
✔ eine Cycloalkyl-, Cycloalkenyl-, N-Cycloalkyl- oder N-Cycloalkenyl-Gruppe, jeweils ggf. substituiert durch einen oder zwei identische oder verschiedene Substituenten, ausgewählt aus einem Wasserstoffatom, einem Halogenatom, einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester-, Amid-, Mono- oder Dialkylamid-, Oxo- oder -X-Aryl-Gruppe, und wobei X -O-, -NH-, -N(Alk)-, -N(COCH₃)-,-S-, -SO-, -SO₂-, -CO- oder -CONH- repräsentiert, oder
✔ eine Aryl-Gruppe, ggf. substituiert durch einen oder zwei identische oder verschiedene Substituenten, ausgewählt aus einem Wasserstoffatom, einem Halogenatom, einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester-, Amid-, Mono- oder Dialkylamid- oder -X-Aryl-Gruppe, und wobei X -O-, -NH-, -N(Alk)-, -N(COCH₃)-, -S-,-SO-, -SO₂-, -CO- oder -CONH- repräsentiert,
• **R₅** repräsentiert:
✔ ein Wasserstoffatom oder ein Halogenatom,
✔ eine Hydroxyl-, Alkoxy-, Halogenalkoxy-, Thio-, Alkylthio-, Halogenalkylthio-, Amino-, Monoalkylamino-, Dialkylamino-, -NH-Acyl-, Cyano-, Acyl-, Säure-, Ester-, Amid-, Monoalkylamid- oder Dialkylamid-Gruppe,
✔ eine Alkyl- oder Halogenalkyl-Gruppe, wobei die AlkylGruppe durch eine Cyano-, Hydroxyl-, Alkoxy-, Säure- oder Ester-Gruppe substituiert sein kann,
✔ eine -SOₙAlk, -SOₙNH₂, -SOₙNHAlk oder -SOₙNAlkAlk'-Gruppe, wobei n gleich 1 oder 2 ist und Alk und Alk' identisch oder verschieden sind, oder
✔ eine Piperidin-, Oxopiperidin-, Morpholin-Gruppe oder auch eine Piperazin-Gruppe, ggf. substituiert durch eine Alkyl-oder Acyl-Gruppe,
und G₁, G₂, R₁, R₂, R₃ und A wie im Anspruch 1 oder 2 definiert sind.

4. Verbindungen nach einem der Ansprüche 1 bis 3, **dadurch gekennzeichnet, dass**:
**G₁** eine Bindung oder eine gesättigte oder ungesättigte, verzweigte oder lineare Kohlenwasserstoffkette mit 1-4 Kohlenstoffatomen repräsentiert, ggf. substituiert durch eine oder zwei Alkylgruppen, die bevorzugt identisch sind,
**G₂** eine Gruppe 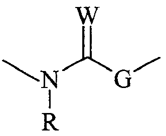 repräsentiert, wobei:
• **R** ein Wasserstoffatom, eine Alkylgruppe, eine Halogenalkylgruppe oder ein Prodrug-Radikal, wie z.B. Carbamat, Acetyl, Dialkylaminomethyl oder -CH₂-O-CO-Alk, repräsentiert,
• **G** eine Bindung oder eine gesättigte oder ungesättigte, lineare oder verzweigte Kohlenwasserstoffkette mit 1-4 Kohlenstoffatomen repräsentiert, ggf. substituiert durch ein oder zwei Alkylgruppen, die-bevorzugt identisch sind,
• **W** ein Sauerstoffatom, Schwefelatom oder NH repräsentiert,
**R₁** und **R₂,** identisch oder verschieden, jeweils eine Gruppe repräsentieren, die ausgewählt ist aus einem Wasserstoffatom, einem Halogenatom, einer Hydroxyl-, Thio-, Alkoxy-, Halogenalkoxy-, Alkylthio-, Halogenalkylthio-, Amino-, Monoalkylamino-, Dialkylamino-, Cycloalkyl-, Alkyl- oder Halogenalkylgruppe,
**R₃** eine Säuregruppe oder eine Prodrug-Radikal der Säurefunktion, wie z.B. Ester, oder auch ein Bioisoster der Säurefunktion, wie z.B. Tetrazol, Phosphonat, Phosphonamid, Sulfonat oder Sulfonamid,
**A** eine Arylgruppe oder einen Heterozyklus repräsentiert, jeweils ggf. substituiert durch eine oder zwei identische oder verschiedene Gruppen, die ausgewählt sind aus:
• einem Wasserstoffatom, einem Halogenatom,
• einer Alkoxy-, Alkylthio-, Halogenalkoxy-, Halogenalkylthio-, Hydroxyl-, Thio-, Cyano-, Amino-, Monoalkylamino- oder Dialkylaminogruppe,
• einer -SOₙR', -COR', -CO₂R', -OCOR', -CONR'R", -NR'COR" oder -NRSO₂R"-Gruppe, wobei R' und R" jeweils unabhängig voneinander ein Wasserstoffatom, eine Alkylgruppe oder eine Halogenalkylgruppe repräsentieren, und wobei n gleich 1 oder 2 ist,
• einer Alkyl- oder Halogenalkylgruppe, wobei die Alkylgruppe ggf. substituiert ist durch eine Cyano-, Amino-, Monoalkylamino-, Dialkylamino- oder Acylamino-Gruppe,
• einer Aryl-, Arylalkyl-, -X-Aryl-Gruppe, wobei X -O-, -NH-, - N(Alk)-, -N(COCH₃)-, -S-, -SO-, -SO₂-, -CO- oder -CONH- repräsentiert, jeweils substituiert im Arylbereich durch einen oder zwei identische oder verschiedene Substituenten, ausgewählt aus:
✔ einem Wasserstoffatom oder einem Halogenatom,
✔ einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester-, Amid-, Mono- oder Dialkylamid-Gruppe, oder
✔ einer -SOₙR', -COR', -CO₂R', -OCOR', -CONR'R",-NR'COR" oder -NR'SO₂R"-Gruppe,
wobei R' und R" jeweils unabhängig voneinander ein Wasserstoffatom, eine Alkylgruppe oder eine Halogenalkylgruppe repräsentieren, und wobei n gleich 1 oder 2 ist,
• einer Heterozyklus-, -X-Heterozyklus-Gruppe, wobei X -O-, - NH-, -N(Alk)-, -N(COCH₃)-, -S-, -SO-, -SO₂-, -CO- oder - CONH- repräsentiert, jeweils ggf. substituiert im Heterozyklusbereich durch einen oder zwei identische oder verschiedene Substituenten, ausgewählt aus:
✔ einem Wasserstoffatom oder einem Halogenatom,
✔ einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester-, Amid-, Mono- oder Dialkylamid-Gruppe,
✔ einer -SOₙR', -COR', -CO₂R', -OCOR', -CONR'R", - NR'COR" oder -NR'SO₂R"-Gruppe,
wobei R' und R" jeweils unabhängig voneinander ein Wasserstoffatom, eine Alkylgruppe oder eine Halogenalkylgruppe repräsentieren, und wobei n gleich 1 oder 2 ist,
• einer Cycloalkyl-, Cycloalkenyl-,-X-Cycloalkyl-, -X-Cycloalkenyl-Gruppe, wobei X -O-, -NH-, -N(Alk)-, -N(COCH₃)-, -S-, -SO-, -SO₂-, -CO- oder -CONH- repräsentiert, jeweils ggf. substituiert im zyklischen Bereich durch einen oder zwei identische oder verschiedene Substituenten, ausgewählt aus:
✔ einem Wasserstoffatom oder einem Halogenatom,
✔ einer Alkyl-, Halogenalkyl-, Alkoxy-, Halogenalkoxy-, Thio-, Alkylthio-, Halogenalkylthio-, Hydroxyl-, Cyano-, Acyl-, Amino-, Monoalkylamino- oder Dialkylamino-, Säure-, Ester, Amid-, Mono- oder Dialkylamid- oder Oxo-Gruppe, oder
✔ einer -SOₙR', -COR', -CO₂R', -OCOR', -CONR'R", - NR'COR" oder -NR'SO₂R"-Gruppe,
wobei R' und R" jeweils unabhängig voneinander ein Wasserstoffatom, eine Alkylgruppe oder eine Halogenalkylgruppe repräsentieren, und wobei n gleich 1 oder 2 ist, und
**B** eine Phenyl- oder Pyridin-Gruppe repräsentiert:
- substituiert in Ortho-Position durch eine N-Cycloalkyl-Gruppe wie z.B. Piperidin oder durch ein Cyclohexyl, jeweils ggf. substituiert durch einen oder Zwei identische oder verschiedene Substituenten, ausgewählt aus einem Wasserstoffatom, einer Alkyl-, Halogenalkyl-, Alkoxy-, Halogenalkoxy- oder -X-Aryl-Gruppe, wobei X -O-, -NH-, -N(Alk)-, -N(COCH₃)-, -S-, -SO-, -SO₂-,-CO- oder -CONH- repräsentiert, und/oder
- ggf. substituiert durch ein Halogenatom oder durch eine Alkyl-oder Halogenalkylgruppe.

5. Verbindungen nach einem der Ansprüche 1 bis 4, **dadurch gekennzeichnet, dass**:
**G₁** eine Bindung oder eine lineare oder verzweigte, gesättigte oder ungesättigte Kohlenwasserstoffkette mit 1 bis 4 Kohlenstoffatomen repräsentiert, ggf. substituiert durch eine oder zwei Alkylgruppen, die bevorzugt identisch sind,
vorzugsweise eine Bindung oder eine Kohlenwasserstoffkette mit 1 oder 2 Kohlenstoffatomen,
**G₂** eine Gruppe 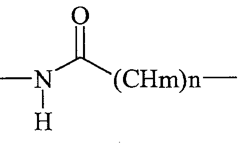 repräsentiert,
wobei n eine ganze Zahl zwischen 1 und 4 und m eine ganze Zahl gleich 1 oder 2 ist, wobei n vorzugsweise gleich 1 oder 2 ist,
**R₁** eine Alkoxygruppe wie z.B. Methoxy repräsentiert, bevorzugt in Ortho-Position in Bezug auf R₃,
**R₂** ein Wasserstoffatom oder ein Halogenatom wie z.B. Chlor oder Brom repräsentiert, oder eine Alkylgruppe wie z.B. Methyl, bevorzugt in Meta-Position in Bezug auf R₃,
**R₃** eine Säure- oder Ester-Gruppe repräsentiert,
**A** eine Arylgruppe wie z.B. Phenyl repräsentiert, bevorzugt substituiert:
- in Meta- oder Para-Position durch:
• ein Halogenatom oder eine Alkyl-, Halogenalkyl-, Cyano-, Alkoxy-, Halogenalkoxy- oder Acylaminoalkyl-Gruppe, oder eine -XR-Gruppe, wobei X -O-, -NH-, -N(Alk)-,-N(COCH₃)-, -S-, -SO-, -SO₂-, -CO- oder -CONH- repräsentiert, und R eine Arylalkyl-, Cycloalkyl- oder Aryl-Gruppe repräsentiert, jeweils ggf. substituiert durch einen oder zwei identische oder verschiedene Substituenten, wie z.B. ein Halogenatom, eine Alkoxy-, Alkyl-, Halogenalkyl-, Cyano-, Acyl-, Amino-, Monoalkylamino- oder Dialkylamino-, Säure-, Ester-, Amid-, Mono- oder Dialkylamid-Gruppe, oder eine -SOₙR', -OCOR', -NR'COR" oder - NR'SO₂R"-Gruppe, wobei R' und R" jeweils unabhängig voneinander ein Wasserstoffatom, eine Alkylgruppe oder eine Halogenalkylgruppe repräsentieren, und wobei n gleich 1 oder 2 ist, oder
• eine Cycloalkyl-, Aryl- oder Arylalkyl-Gruppe oder einen Heterozyklus, bevorzugt N-Cycloalkyl, jeweils ggf. substituiert durch einen oder zwei identische oder verschiedene Substituenten, wie z.B. ein Halogenatom, eine Alkoxy-, Alkyl-, Halogenalkyl-, Cyano-, Acyl-, Amino-, Monoalkylamino- oder Dialkylamino-, Säure-, Ester-, Amid-, Mono-oder Dialkylamid-Gruppe, oder eine -SOₙR', -OCOR', - NR'COR" oder -NR'SO₂R"-Gruppe, wobei R' und R" jeweils unabhängig voneinander ein Wasserstoffatom, eine Alkylgruppe oder eine Halogenalkylgruppe repräsentieren, und wobei n gleich 1 oder 2 ist,
- und/oder in Ortho- oder Meta-Position durch eine Alkylgruppe, und
**B** eine Arylgruppe repräsentiert, bevorzugt ein Phenyl,
• substituiert in Ortho-Position durch einen Heterozyklus, bevorzugt durch ein N-Cycloalkyl wie z.B. eine Piperidin-Gruppe, und/oder
• substituiert in Ortho'-Position durch eine Alkylgruppe wie z.B. Methyl.

6. Verbindungen nach einem der Ansprüche 1 bis 5, **dadurch gekennzeichnet, dass**:
**G₁** eine Bindung oder eine lineare oder verzweigte, gesättigte oder ungesättigte Kohlenwasserstoffkette mit 1 bis 4 Kohlenstoffatomen repräsentiert, ggf. substituiert durch eine oder zwei Alkylgruppen, die bevorzugt identisch sind,
vorzugsweise eine Bindung oder eine Kohlenwasserstoffkette mit 1 oder 2 Kohlenstoffatomen,
**G₂** eine Gruppe 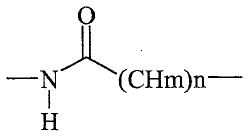 repräsentiert,
wobei n eine ganze Zahl zwischen 1 und 4 und m eine ganze Zahl gleich 1 oder 2 ist, wobei n vorzugsweise gleich 1 oder 2 ist,
**R₁** eine Alkoxygruppe wie z.B. Methoxy repräsentiert, bevorzugt in Ortho-Position in Bezug auf R₃,
**R₂** ein Wasserstoffatom oder ein Halogenatom wie z.B. Chlor oder Brom repräsentiert, oder eine Alkylgruppe wie z.B. Methyl, bevorzugt in Meta-Position in Bezug auf R₃,
**R₃** eine Säure- oder Ester-Gruppe repräsentiert,
**A** eine Arylgruppe wie z.B. Phenyl repräsentiert, bevorzugt substituiert:
- in Meta- oder Para-Position durch:
• ein Halogenatom oder eine Cyano-, Alkoxy-, Halogenalkoxy- oder Acylaminoalkyl-Gruppe, oder eine -XR-Gruppe, wobei X -O-, -S-, -SO-, -SO₂- oder -CO- repräsentiert, und R eine Arylalkyl-, Cycloalkyl- oder Aryl- Gruppe repräsentiert, jeweils ggf. substituiert durch einen oder zwei identische oder verschiedene Substituenten, wie z.B. ein Halogenatom, eine Alkoxy- oder Acyl-Gruppe, oder
• eine Cycloalkyl-, Aryl- oder Arylalkyl-Gruppe, jeweils ggf. substituiert durch einen oder zwei identische oder verschiedene Substituenten, wie z.B. eine Acyl- oder AlkoxyGruppe, und
- und/oder in Ortho- oder Meta-Position durch eine Alkylgruppe, und
**B** eine Arylgruppe repräsentiert, bevorzugt ein Phenyl,
• substituiert in Ortho-Position durch einen Heterozyklus, bevorzugt durch ein N-Cycloalkyl wie z.B. eine Piperidin-Gruppe, und/oder
• substituiert in Ortho'-Position durch eine Alkylgruppe wie z.B. Methyl.

7. Verbindungen nach einem der Ansprüche 1 bis 6, ausgewählt aus der folgenden Gruppe:
1) 5-Brom-2-methoxy-4-[*N*'-(4-methoxy-phenyl)-*N*'-(2-piperidin-1-yl-phenyl)-hydrazinocarbonylmethyl]-benzoesäure-Hydrochlorid
2) 5-Brom-2-methoxy-4-[N'-(2-piperidin-1-yl-phenyl)-N'-(4-triflu-ormethoxy-phenyl)-hydrazinocarbonylmethyl]-benzoesäure-Hyd-rochlorid
3) 5-Brom-2-methoxy-4-[N'-(3-methoxy-benzyl)-N'-(2-piperidin-1-yl-phenyl)-hydrazinocarbonylmethyl]-benzoesäure-Hydrochlorid
4) 4-[N-(4-Benzyloxy-phenyl)-N'-(2-piperidin-1-yl-phenyl)-hydrazinocarbonylmethyl]-5-brom-2-methoxy-benzoesäure-Hydrochlorid
5) 5-Brom-4-{N'-[4-(4-fluor-phenoxy)-phenyl]-N'-(2-piperidin-1-yl-phenyl)-hydrazinocarbonylmethyl}-2-methoxy-benzoesäure-Hydrochlorid
6) 5-Brom-2-methoxy-4-{N'-[2-(4-methoxy-phenyl)-ethyl]-N'-(2-piperidin-1-yl-phenyl)-hydrazinocarbonylmethyl}-benzoesäure-Hydrochlorid
7) 5-Brom-2-methoxy-4-[N'-(4-methoxy-phenyl)-N'-(2-methyl-6-piperidin-1-yl-phenyl)-hydrazinocarbonyl-methyl]-benzoesäure-Hydrochlorid
7a) 5-Brom-2-methoxy-4-[N'-(4-methoxy-phenyl)-N'-(2-methyl-6-piperidin-1-yl-phenyl)-hydrazinocarbonyl-methyl]-Benzoesäure-Methylester
8) 5-Brom-2-methoxy-4-[N'-(4-methoxy-benzyl)-N'-(2-piperidin-1-yl-phenyl)-hydrazinocarbonylmethyl]-benzoesäure-Hydrochlorid
9) 5-Brom-4-[N'-(4-cyclohexyl-phenyl)-N'-(2-piperidin-1-yl-phenyl)-hydrazinocarbonylmethyl]-2-methoxy-benzoesäure-Hydrochlorid
10) 5-Brom-2-methoxy-4-[N'-(2-methyl-6-piperidin-1-yl-phenyl)-N'-(4-trifluormethoxy-phenyl)-hydrazino-carbonylmethyl]-benzoesäure-Hydrochlorid
11) 5-Brom-2-methoxy-4-[N'-(4'-methoxy-biphenyl-4-yl)-N'-(2-piperidin-1-yl-phenyl)-hydrazinocarbonylmethyl]-benzoesäure-Hydrochlorid
12) 5-Brom-4-[N'-(4-cyclohexyloxy-phenyl)-N'-(2-piperidin-1-yl-phenyl)-hydrazinocarbonylmethyl]-2-methoxy-benzoesäure-Hydrochlorid
13) 5-Brom-2-methoxy-4-[N'-(4-phenoxy-phenyl)-N'-(2-piperidin-1-yl-phenyl)-hydrazinocarbonylmethyl]-benzoesäure-Hydrochlorid
14) 5-Brom-4-{N'-[4-(4-chloro-phenoxy)-phenyl]-N'-(2-piperidin-1-yl-phenyl)-hydrazinocarbonylmethyl}-2-methoxy-benzoesäure-Hydrochlorid
15) 4-{N'-[4-(4-Fluor-phenoxy)-phenyl]-N'-(2-methyl-6-piperidin-1-yl-phenyl)-hydrazinocarbonylmethyl}-2-methoxy-benzoesäure-Hydrochlorid
16) 5-Brom-4-{N'-[4-(4-fluor-phenoxy)-phenyl]-N'-(2-methyl-6-pi-peridin-1-yl-phenyl)-hydrazino-carbonyl-methyl}-2-methoxy-benzoesäure-Hydrochlorid
16a) 5-Brom-4-{N'-[4-(4-fluor-phenoxy)-phenyl]-N'-(2-methyl-6-piperidin-1-yl-phenyl)-hydrazino-carbonyl-methyl}-2-methoxy-benzoesäure-Methylester
17) 4-[N'-(4-Benzyl-phenyl)-N'-(2-piperidin-1-yl-phenyl) hydrazinocarbonylmethyl]-5-brom-2-methoxy-benzoesäure-Hydrochlorid
18) 4-[N'-(4-Brom-phenyl)-N'-(2-piperidin-1-yl-phenyl) hydrazinocarbonylmethyl]-5-chlor-2-methoxy-benzoesäure-Hydrochlorid
19) 4-[N'-(3'-Acetyl-biphenyl-4-yl)-N'-(2-piperidin-1-yl-phenyl)-hydrazinocarbonylmethyl]-5-chlor-2-methoxy-benzoesäure-Hydrochlorid
20) 4-[N'-(4'-Acetyl-biphenyl-4-yl)-N'-(2-piperidin-1-yl-phenyl)-hydrazinocarbonylmethyl]-5-chlor-2-methoxy-benzoesäure-Hydrochlorid
21) 5-Brom-2-methoxy-4-[N'-(3-phenoxy)-phenyl]-N'-(2 piperidin-1-yl-phenyl)-hydrazinocarbonylmethyl]-benzoesäure-Hydrochlorid
22) 5-Brom-2-methoxy-4-[N'-(4-phenylsulfanyl)-phenyl]-N'-(2-piperidin-1-yl-phenyl)-hydrazinocarbonylmethyl]-benzoesäure-Hydrochlorid
23) 4-[N'-(4-Benzensulfonyl-phenyl)-N'-(2-piperidin-1-yl-phenyl)-hydrazinocarbonylmethyl]-5-brom-2-methoxy -benzoesäure-Hydrochlorid
24) 4-[N'-(4-Benzensulfinyl-phenyl)-N'-(2-piperidin-1-yl-phenyl)-hydrazinocarbonylmethyl]-5-brom-2-methoxy-benzoesäure-Hydrochlorid
25) 2-Methoxy-4-{(E)-2-[N'-(4-methoxy-phenyl)-N'-(2-methyl-6-piperidin-1-yl-phenyl)-hydrazinocarbonyl]-vinyl}-benzoesäure-Hydrochlorid
26) 5-Brom-2-methoxy-4-{(E)-2-[N'-(4-methoxy-phenyl)-N'-(2-methyl-6-piperidin-1-yl-phenyl)-hydrazinocarbonyl]-vinyl}-ben-zoesäure-Hydrochlorid
27) 4-[N'-(4-Benzyl-phenyl]-N'-(2-methyl-6-piperidin-1-yl-phenyl)-hydrazinocarbonylmethyl]-5-brom-2-methoxy-benzoesäure-Hydrochlorid
28) 5-Brom-2-methoxy-4-[N'-(4'-methoxy-biphenyl-4-yl)-N'-(2-methyl-6-piperidin-1-yl-phenyl)-hydrazino-carbonyl-methyl]-benzoesäure-Hydrochlorid
29) 4-[N'-(Acetylamino-methyl)-phenyl]-N'-(2-methyl-6-piperidin-1-yl-phenyl)-hydrazinocarbonylmethyl]-5-brom-2-methoxy-benzoesäure-Hydrochlorid
30) 4-[N'-(4-Benzoyl)-phenyl]-N'-(2-piperidin-1-yl-phenyl)-hydrazinocarbonylmethyl]-5-brom-2-methoxy-benzoesäure-Hydrochlorid
31) 5-Brom-4-[N'-(4-cyano-phenyl)-N'-(2-methyl-6-piperidin-1-yl-phenyl)-hydrazinocarbonylmethyl]-2-methoxy-benzoesäure-Hydrochlorid
32) 4-[N'-(4'-Acetyl-biphenyl-4-yl)-N'-(2-methyl-6-piperidin-1-yl-phenyl)-hydrazinocarbonylmethyl]-5-brom-2-methoxy-benzoesäure-Hydrochlorid
33) 5-Brom-2-methoxy-4-[N'-(4'-methoxy-2-methyl-biphenyl-4-yl)-N'-(2-methyl-6-piperidin-1-yl-phenyl)-hydrazinocarbonylmethyl]-benzoesäure-Hydrochlorid.

8. Zwischenprodukte für die Synthese der Verbindungen nach Anspruch 7, ausgewählt aus der folgenden Gruppe:
a) N-(4-Methoxy-phenyl)-N-(2-piperidin-1-yl-phenyl)-hydrazin
b) N-(2-Piperidin-1-yl-phenyl)-N-(4-trifluormethoxy-phenyl)-hydrazin
c) N-(3-Methoxy-benzyl)-N-(2-piperidin-1-yl-phenyl)-hydrazin
d) N-(4-Benzyloxy-phenyl)-N-(2-piperidin-1-yl-phenyl)-hydrazin
e) N-[4-(4-Fluor-phenoxy)-phenyl]-N-(2-piperidin-1-yl-phenyl)-hydrazin
f) N-[2-(4-Methoxy-phenyl)-ethyl]-N-(2-piperidin-1-yl-phenyl)-hydrazin
g) N-(4-Methoxy-phenyl)-N-(2-methyl-6-piperidin-1-yl-phenyl)-hydrazin
h) N-(4-Methoxy-benzyl)-N-(2-piperidin-1-yl-phenyl)-hydrazin
i) N-(4-Cyclohexyl-phenyl)-N-(2-piperidin-1-yl-phenyl)-hydrazin
j) N-(2-Methyl-6-piperidin-1-yl-phenyl)-N-(4-trifluor-methoxy-phenyl)-hydrazin
k) N-(4'-Methoxy-biphenyl-4-yl)-N-(2-piperidin-1-yl-phenyl)-hydrazin
l) N-(4-Cyclohexyloxy-phenyl)-N-(2-piperidin-1-yl-phenyl)-hydrazin
m) N-(4-Phenoxy-phenyl)-N-(2-piperidin-1-yl-phenyl)-hydrazin
n) N-[4-(4-Chlor-phenoxy)-phenyl]-N-(2-piperidin-1-yl-phenyl)-hydrazin
o) N-[4-(4-Fluor-phenoxy)-phenyl]-N-(2-methyl-6-piperidin-1-yl-phenyl)-hydrazin
p) N-(4-Benzyl-phenyl]-N-(2-piperidin-1-yl-phenyl)-hydrazin
q) N-(4-Brom-phenyl)-N-(2-piperidin-1-yl-phenyl)-hydrazin
r) N-(3-Phenoxy-phenyl)-N-(2-piperidin-1-yl-phenyl)-hydrazin
s) N-(4-Phenylsulfanyl-phenyl)-N-(2-piperidin-1-yl-phenyl)-hydrazin
t) N-(4-Benzyl-phenyl)-N-(2-methyl-6-piperidin-1-yl-phenyl)-hydrazin
u) N-(4'-Methoxy-biphenyl-4-yl)-N-(2-methyl-6-piperidin-1-yl-phenyl)-hydrazin
v) N-(4-Benzoyl-phenyl)-N-(2-piperidin-1-yl-phenyl)-hydrazin
w) N-[4'-(2-Methy1-[1,3]dithian-2-yl)-biphenyl-4-yl]-N-(2-methy1-6-piperidin-1-yl-phenyl)-hydrazin
x) N-(4'-Methoxy-2-methyl-bipheny1-4-yl)-N-(2-methy1-6-piperidin-l-yl-phenyl)hydrazin.

9. Verfahren zur Herstellung von Verbindungen der Formel (I) nach einem der Ansprüche 1 bis 7, wobei
**G₂** das Radikal 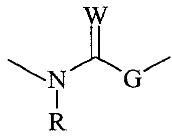 repräsentiert, wie in Anspruch 1 definiert,
**dadurch gekennzeichnet, dass** die folgenden Schritte durchgeführt werden:
1) Reaktion der Hydrazinfunktion einer Verbindung der Formel (II) 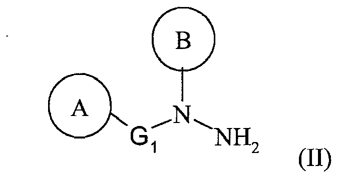 wobei A, B und G₁ wie in Anspruch 1 definiert sind,
mit der Säurefunktion einer Verbindung der Formel (III) 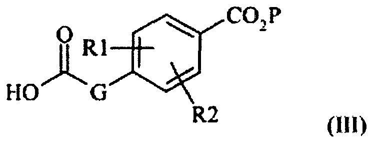 wobei R₁, R₂ und G wie in Anspruch 1 definiert sind und P eine Schutzgruppe der Säurefunktion repräsentiert,
um die Verbindung der Formel (IV) zu erhalten 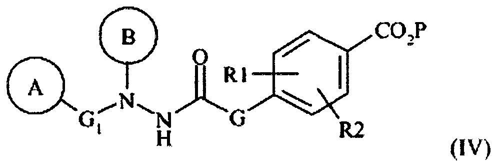
2) Entschützen der -CO₂P-Gruppe der Verbindung der Formel (IV) durch Hydrolyse, um die Verbindung der Formel (I) zu erhalten 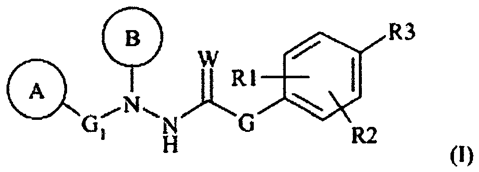 wobei W ein Sauerstoffatom repräsentiert und R₃ wie in Anspruch 1 definiert ist,
3) gegebenenfalls Reaktion der in Schritt 2) erhaltenen Verbindung der Formel (I) oder auch der in Schritt 1) erhaltenen Verbindung der Formel (IV) mit einem Lawessons Reagenz, wodurch eine Verbindung der Formel (I) erhalten werden kann, bei der W ein Schwefelatom repräsentiert.

10. Verfahren zur Herstellung von Verbindungen der Formel (I) nach einem der Ansprüche 1 bis 7, wobei
**G₂** das Radikal 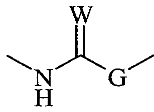 repräsentiert, wie im Anspruch 1 definiert,
**dadurch gekennzeichnet, dass** die folgenden Schritte durchgeführt werden:
1) Reaktion in einem sauren Milieu der Verbindung der Formel (V): 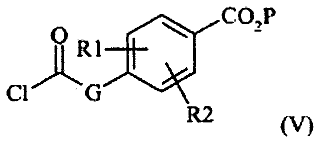 wobei R₁, R₂ und G wie in Anspruch 1 definiert sind und P eine Schutzgruppe der Säurefunktion repräsentiert, wie z.B. lineares oder verzweigtes (C₁-C₄)-Alkyl,
mit der Verbindung der Formel (XXIV): 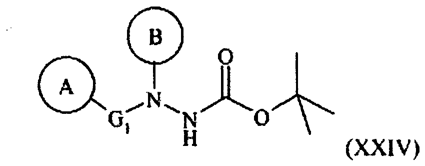 wobei A, B und G₁ wie in Anspruch 1 definiert sind,
um die Verbindung der Formel (IV) zu erhalten: 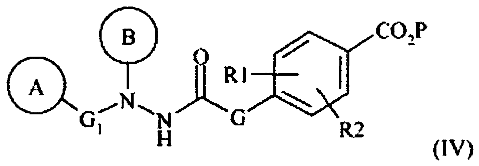
2) Entschützen der -CO₂P-Gruppe der Verbindung der Formel (IV) durch Hydrolyse, um die Verbindung der Formel (I) zu erhalten: 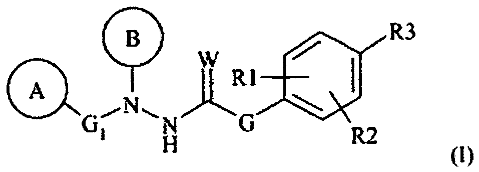 wobei W ein Sauerstoffatom repräsentiert und R₃ wie in Anspruch 1 definiert ist.

11. Pharmazeutische Zusammensetzung, umfassend mindestens eine Verbindung der Formel (I) nach einem der Ansprüche 1 bis 7, in Verbindung mit einem pharmazeutisch akzeptablen Hilfsstoff.

12. Verbindung der Formel (I) nach einem der Ansprüche 1 bis 7 für die Verwendung als Medikament.

13. Verwendung einer Verbindung der Formel (I) nach einem der Ansprüche 1 bis 7 für die Herstellung eines Medikaments, das zur Behandlung oder Prävention einer Infektion durch das Papillomvirus bestimmt ist.

14. Verwendung nach Anspruch 13 für die Behandlung oder Prävention von Läsionen und Krankheiten, die mit Infektionen durch das Papillomvirus assoziiert sind.

15. Verwendung nach Anspruch 13 oder 14 für die Behandlung oder Prävention von Ano-Genitalwarzen, wie z.B. Condylomata acuminata und Condylomata plana; laryngealen, konjunktivalen oder bukkalen Papillomen und anderen epithelialen Läsionen, wie z.B. rezidivierende respiratorische Papillomatosen sowie niedriggradige und hochgradige intra-epitheliale Neoplasein; bowenoiden Papulosen; Warzen (vulgäre Warzen, Plantarwarzen, Myrmecien, oberflächliche Warzen, Flachwarzen...); Epidermodysplasiae verruciformes; Karzinomen, insbesondere ano-genitalen Karzinomen; und allen Läsionen, die mit dem Papillomvirus assoziiert sind.

## Claims

1. Compounds of formula (I): 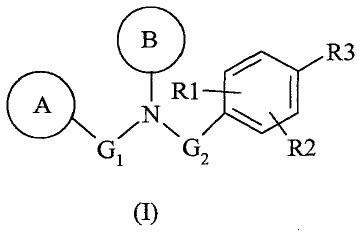 as well as their stereoisomers,
wherein
**G₁** represents a bond or a saturated or unsaturated, branched or linear hydrocarbon chain comprising 1-4 carbon atoms, optionally substituted with one or two alkyl groups, preferably identical,
**G₂** represents a 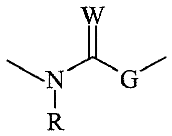 group, wherein:
• R represents a hydrogen atom, an alkyl, haloalkyl group, or a pro-drug radical such as a carbamate, acetyl, dialkylaminomethyl or -CH₂-O-CO-Alk,
• **G** represents a bond or a saturated or unsaturated, linear or branched hydrocarbon chain comprising 1-4 carbon atoms, optionally substituted with one or two alkyl groups, preferably identical,
• **W** represents an oxygen, sulfur atom or NH,
**R₁** and **R₂** either identical or different, each represent a group selected from a hydrogen atom, a halogen atom, a hydroxyl, thio, alkoxy, haloalkoxy, alkylthio, haloalkylthio, amino, monoalkylamino, dialkylamino, cycloalkyl, alkyl or haloalkyl group,
**R₃** represents an acid group or a pro-drug radical of the acid function such as an ester, or else a bioisoster of the acid function such as a tetrazole, phosphonate, phosphonamide, sulfonate or sulfonamide,
**A** represents an aryl, cycloalkyl, cycloalkenyl group or a heterocycle, each optionally substituted, and
**B** represents an aryl group or a 6-membered heterocycle, each optionally substituted,
as well as their pharmaceutically acceptable salts.

2. The compounds according to claim 1, **characterized in that A** is substituted with one or two groups, either identical or different, selected from:
• a hydrogen atom, a halogen atom,
• an alkoxy, alkylthio, haloalkoxy, haloalkylthio, hydroxyl, thio, cyano, amino, monoalkylamino, or dialkylamino group,
• an -SOₙR', -COR', -CO₂R', -OCOR', -CONR'R",-NR'COR" or -NR'SO₂R" group, wherein R' and R" each represent independently of each other a hydrogen atom, an alky, haloalkyl group, and n has the value 1 or 2,
• an alkyl or haloalkyl group, the alkyl group being optionally substituted with a cyano, amino, monoalkylamino, dialkylamino or acylamino group,
• an aryl, arylalkyl, -X-aryl, -X-arylalkyl or -Alk-X-aryl group wherein X represents -O-,-NH-, -N(Alk)-, -N(COCH₃)-, -S-, -SO-, -SO₂-, -CO- or -CONH-, each substituted on the aryl portion with one or two substituents, either identical or different, selected from:
✔ a hydrogen atom or an halogen atom,
✔ an alkyl, haloalkyl, alkoxy, haloalkoxy, thio, alkylthio, haloalkylthio, hydroxyl, cyano, acyl, amino, monoalkylamino or dialkylamino, acid, ester, amide, mono- or di-alkylamide group,
✔ an -SOₙR', -COR', -CO₂R', -OCOR',-CONR'R'', -NR'COR'' or -NR'SO₂R'' group, wherein R' and R'' each represent independently of each other a hydrogen atom, an alkyl, haloalkyl group and n has the value 1 or 2,
• a heterocycle, -Alk-heterocycle, -X-heterocycle, -X-Alk-heterocycle, or -Alk-X-heterocycle group, wherein X represents -O-, -NH-, -N(Alk)-, -N(COCH₃)-, -S-, -SO-, -SO₂-, -CO-, or -CONH-, each optionally substituted on the heterocycle portion with one or two substituents either identical or different, selected from:
✔ a hydrogen atom or a halogen atom,
✔ an alkyl, haloalkyl, alkoxy, haloalkoxy, thio, alkylthio, haloalkylthio, hydroxyl, cyano, acyl, amino, monoalkylamino or dialkylamino, acid, ester, amide, mono- or di-alkylamide group, or
✔ an -SOₙR', -COR', -CO₂R', -OCOR',-CONR'R'', -NR'COR'' or NR' SO₂R'' group, wherein R' and R'' each represent independently of each other a hydrogen atom, an alkyl, haloalkyl group and n has the value 1 or 2,
• a cycloalkyl, -Alk-cycloalkyl, cycloalkenyl, -Alk-cycloalkenyl, -X-cycloalkyl, -X-Alk-cycloalkyl, -X-cycloalkenyl, -X-Alk-cycloalkenyl, -Alk-X-cycloalkyl, -Alk-X-cycloalkenyl group, wherein X represents -O-, -NH-, -N(Alk)-, -N(COCH₃)-, -S-, -SO-, -SO₂-, -CO- or -CONH- each optionally substituted on the cyclic portion with one or two substituents, either identical or different, selected from:
✔ a hydrogen atom or a halogen atom,
✔ an alkyl, haloalkyl, alkoxy, haloalkoxy, thio, alkylthio, haloalkylthio, hydroxyl, cyano, acyl, amino, monoalkylamino or dialkylamino, acid, ester, amide, mono- or di-alkylamide, or oxo or
✔ an -SOₙR', -COR', -CO₂R', -OCOR',-CONR'R'', -NR'COR'' or NR'SO₂R'' group, wherein R' and R'' each represent independently of each other a hydrogen atom, an alkyl, haloalkyl group and n has the value 1 or 2,
and G₁, G₂, R₁, R₂, R₃ and B are as defined in claim 1.

3. Compounds according to claim 1 or 2, **characterized in that B** is an aryl or 6-membered heterocycle, substituted in the ortho position with a **R₄** group and optionally substituted with a **R₅** group, wherein:
• **R₄** represents:
✔ an alkyl, -NHAlk, -NAlkAlk', -NHcycloalkyl or -NAlkcycloalkyl group, Alk and Alk' being identical or different,
✔ a cycloalkyl, cycloalkenyl, N-cycloalkyl or N-cycloalkenyl group, each optionally substituted with one or two substituents, either identical or different, selected from a hydrogen atom, a halogen atom, an alkyl, haloalkyl, alkoxy, haloalkoxy, thio, alkylthio, haloalkylthio, hydroxyl, cyano, acyl, amino, monoalkylamino or dialkylamino, acid, ester, amide, mono- or dialkylamide, oxo or -X-aryl group and wherein X represents -O-, -NH-, -N(Alk)-, -N(COCH₃)-, -S-, -SO-,-SO₂-, -CO- or -CONH-, or
✔ an aryl group optionally substituted with one or two substituents, either identical or different, a hydrogen atom, a halogen atom, an alkyl, haloalkyl, alkoxy, haloalkoxy, thio, alkylthio, haloalkylthio, hydroxyl, cyano, acyl, amino, monoalkylamino or dialkylamino, acid, ester, amide, mono or dialkylamide or -X-aryl group, wherein X represents -O-, -NH-, -N(Alk)-, -N(COCH₃)-,-S-, -SO-, -SO₂-, -CO- or -CONH-,
• **R₅** represents:
✔ a hydrogen atom or a halogen atom,
✔ a hydroxyl, alkoxy, haloalkoxy, thio, alkylthio, haloalkylthio, amino, monoalkylamino, dialkylamino, -NHacyl, cyano, acyl, acid, ester, amide, monoalkylamide or dialkylamide group,
✔ an alkyl or haloalkyl group, the alkyl group may be substituted with a cyano, hydroxyl, alkoxy, acid or ester group,
✔ an -SOₙAlk, -SOₙNH₂, -SOₙNHAlk or - SOₙNAlkAlk' group, wherein n has the value of 1 or 2 and Alk and Alk' are either identical or different, or
✔ a piperidine, oxopiperidine, morpholine group, or else a piperazine group optionally substituted with an alkyl or acyl group,
and G₁, G₂, R₁, R₂, R₃ and A are as defined in claim 1 or 2.

4. Compounds according to any of claims 1 to 3, **characterized in that**:
**G₁** represents a bond or a saturated or unsaturated, linear or branched hydrocarbon chain comprising 1-4 carbon atoms, optionally substituted with one or two alkyl groups, preferably identical,
G₂ represents a 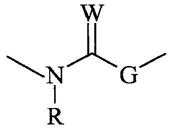 group, wherein:
• **R** represents a hydrogen atom, an alkyl, haloalkyl group, or a pro-drug radical such as carbamate, acetyl, dialkylaminomethyl or-CH₂-O-CO-Alk,
• **G** represents a bond or a saturated or unsaturated, linear or branched, hydrocarbon chain comprising 1-4 carbon atoms, optionally substituted with one or two alkyl groups, preferably identical, and
• **W** represents an oxygen, sulphur atom or NH,
**R₁** and **R₂** either identical or different, each represent a group selected from a hydrogen atom, a halogen atom, a hydroxyl, thio, alkoxy, haloalkoxy, alkylthio, haloalkylthio, amino, monoalkylamino, dialkylamino, cycloalkyl, alkyl or haloalkyl group,
**R₃** represents an acid group or a pro-drug radical of the acid function such as an ester, or else a bioisoster of the acid function such as tetrazole, phosphonate, phosphonamide, sulfonate or sulfonamide,
**A** represents an aryl or heterocycle group, each optionally substituted with one or two groups, either identical or different, selected from:
• a hydrogen atom, a halogen atom,
• an alkoxy, alkylthio, haloalkoxy, haloalkylthio, hydroxyl, thio, cyano, amino, monoalkylamino or dialkylamino group,
• a -SOₙR', -COR'-, -CO₂R', -OCOR', -CONR'R'', -NR'COR'' or -NR'SO₂R'' group, wherein R' and R" each represent independently of each other a hydrogen atom, an alkyl, haloalkyl group, and n has the value 1 or 2,
• an alkyl or haloalkyl group, the alkyl group being optionally substituted with a cyano, amino, monoalkylamino, dialkylamino or acylamino group,
• an aryl, arylalkyl, -X-aryl group, wherein X represents -O-, -NH-, -N(Alk)-, -N(COCH₃)-,-S-, -SO-, -SO₂-, -CO- or -CONH- group, each substituted on the aryl portion with one or two substituents, either identical or different, selected from:
✔ a hydrogen atom or a halogen atom,
✔ an alkyl, haloalkyl, alkoxy, haloalkoxy, thio, alkylthio, haloalkylthio, hydroxyl, cyano, acyl, amino, monoalkylamino or di-alkylamino, acid, ester, amide, mono- or dialkylamide group, or
✔ an -SOₙR', -COR', -CO₂R', -OCOR',-CONR'R'', -NR'COR'' or -NR'SO₂R'' group, wherein R' and R'' each represent independently of each other a hydrogen atom, an alkyl, haloalkyl group, and n has the value 1 or 2,
• a heterocycle, -X-heterocycle group wherein X represents -O-, -NH-, -N(Alk)-, -N(COCH₃)-, -S-, -SO-, -SO₂-, -CO- or -CONH-, each optionally substituted on the heterocycle portion with one or two substituents, either identical or different, selected from:
✔ a hydrogen atom or a halogen atom,
✔ an alkyl, haloalkyl, alkoxy, haloalkoxy, thio, alkylthio, haloalkylthio, hydroxyl, cyano, acyl, amino, monoalkylamino or dialkylamino, acid, ester, amide, mono- or di-alkylamide group, or
✔ an -SOₙR', -COR', -CO₂R', -OCOR',-CONR'R'', -NR'COR'' or -NR'SO₂R'' group, wherein R' and R'' each represent independently of each other a hydrogen atom, an alkyl, haloalkyl group, and n has the value 1 or 2, or
• a cycloalkyl, cycloalkenyl, -X-cycloalkyl, -X-cycloalkenyl group wherein X represents -O-,-NH-, -N(Alk)-, -N(COCH₃)-, -S-, -SO-, -SO₂-,-CO- or -CONH- group, each optionally substituted on the cyclic portion with one or two substituents, either identical or different, selected from:
✔ a hydrogen atom or a halogen atom,
✔ an alkyl, haloalkyl, alkoxy, haloalkoxy, thio, alkylthio, haloalkylthio, hydroxyl, cyano, acyl, amino, monoalkylamino or dialkylamino, acid, ester, amide, mono- or di-alkylamide, or oxo group, or
✔ an -SOₙR', -COR', -CO₂R', -OCOR',-CONR'R'', -NR'COR'' or -NR'SO₂R'' group, wherein R' and R'' each represent independently of each other a hydrogen atom, an alkyl, haloalkyl group, and n has the value 1 or 2, and
**B** represents a phenyl or pyridine group:
- substituted in the ortho position with an N-cycloalkyl group such as piperidine or with a cyclohexyl, each optionally substituted with one or two substituents, either identical or different, selected from a hydrogen atom, an alkyl, haloalkyl, alkoxy, haloalkoxy, -X-aryl group, where X represents -O-, -NH-, -N(Alk)-, - N(COCH₃)-, -S-, -SO-, -SO₂-, -CO- or -CONH-, and/or
- optionally substituted with a halogen atom or with an alkyl or haloalkyl group.

5. Compounds according to any of claims 1 to 4, **characterized in that**:
**G₁** represents a bond or a saturated or unsaturated, linear or branched hydrocarbon chain comprising 1-4 carbon atoms, optionally substituted with one or two alkyl groups, preferably identical,
preferably a bond or a hydrocarbon chain comprising 1 or 2 carbon atoms,
**G₂** represents a 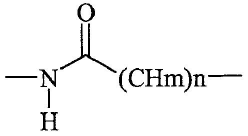 group,
wherein n is an integer comprised between 1 and 4 and m is an integer having the value 1 or 2, preferably n has the value 1 or 2,
**R₁** represents an alkoxy group, such as methoxy, preferably in the ortho position relatively to R₃,
**R₂** represents a hydrogen or halogen atom, such as chlorine or bromine, or an alkyl group, such as methyl, preferably in the meta position relatively to R₃,
**R₃** represents an acid or ester group,
**A** represents an aryl group such as a phenyl, preferably substituted:
- in the meta or para position with:
• a halogen atom or an alkyl, haloalkyl, cyano, alkoxy, haloalkoxy, acylaminoalkyl group or an -XR group, wherein X represents -O-, -NH-, -N(Alk)-, -N(COCH₃)-, -S-, -SO-, -SO₂-, -CO-or -CONH-, and R represents an arylalkyl, cycloalkyl or aryl group, each optionally substituted with one or two substituents, either identical or different, such as a halogen atom, an alkoxy, alkyl, haloalkyl, cyano, acyl, amino, monoalkylamino or dialkylamino, acid, ester, amide, mono- or di-alkylamide group, or an -SOₙR', -OCOR', - NR'COR'' or -NR'SO₂R'' group, wherein R' and R" each represent independently of each other a hydrogen atom, an alkyl, haloalkyl group, and n has the value 1 or 2,
• a cycloalkyl, aryl, arylalkyl group or heterocycle, preferably N-cycloalkyl, each being optionally substituted with one or two substituents, either identical or different, such as a halogen atom, an alkoxy, alkyl, haloalkyl, cyano, acyl, amino, monoalkylamino or dialkylamino, acid, ester, amide, mono- or di-alkylamide group, or an -SOₙR', -OCOR',-NR'COR'', or -NR'SO₂R'' group, wherein R' and R" each represent independently of each other a hydrogen atom, an alkyl, haloalkyl group and n has the value 1 or 2,
- and/or in the ortho or meta position with an alkyl group, and
**B** represents an aryl group, preferably a phenyl,
• substituted in the ortho position with a heterocycle, preferably a N-cycloalkyl, such as a piperidine group, and/or
• substituted in the ortho' position with an alkyl group, such as a methyl.

6. Compounds according to any of claims 1 to 5, **characterized in that**:
**G₁** represents a bond or a saturated or unsaturated, linear or branched hydrocarbon chain comprising 1-4 carbon atoms, optionally substituted with one or two alkyl groups, preferably identical,
preferably a bond or a hydrocarbon chain comprising 1 or 2 carbon atoms,
**G₂** represents a 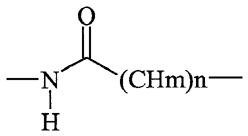 group,
wherein n is an integer comprised between 1 and 4 and m is an integer having the value 1 or 2, preferably n has the value 1 or 2,
**R₁** represents an alkoxy group, such as methoxy, preferably in the ortho position relatively to R₃,
**R₂** represents a hydrogen or halogen atom, such as chlorine or bromine, or an alkyl group, such as methyl, preferably in the meta position relatively to R₃,
**R₃** represents an acid or ester group,
**A** represents an aryl group such as a phenyl, preferably substituted:
- in the meta or para position with:
• a halogen atom or a cyano, alkoxy, haloalkoxy, acylaminoalkyl or -XR group, wherein X represents -O-, -S-, -SO-, -SO₂-, or -CO- and R represents an arylalkyl, cycloalkyl or aryl group, each optionally substituted with one or two substituents, either identical or different, such as a halogen atom, an alkoxy or acyl group, or
• a cycloalkyl, aryl or arlyalkyl group, each optionally substituted with one or two substituents, either identical or different, such as an acyl or alkoxy group, and
- and/or in the ortho or meta position with an alkyl group, and
B represents an aryl group, preferably a phenyl,
• substituted in the ortho position with a heterocycle, preferably a N-cycloalkyl, such as a piperidine group, and/or
• substituted in the ortho' position with an alkyl group, such as a methyl.

7. Compounds according to any of claims 1 to 6, selected from the following group:
1) 5-bromo-2-methoxy-4-[*N*'-(4-methoxy-phenyl)-*N*'-(2-piperidin-1-yl-phenyl)-hydrazinocarbonylmethyl]-benzoic acid hydrochloride
2) 5-bromo-2-methoxy-4-[N'-(2-piperidin-1-yl-phenyl)-N'-(4-trifluoromethoxy-phenyl)-hydrazinocarbonylmethyl]-benzoic acid hydrochloride
3) 5-bromo-2-methoxy-4-[N'-(3-methoxy-benzyl)-N'-(2-piperidin-1-yl-phenyl)-hydrazinocarbonylmethyl]-benzoic acid hydrochloride
4) 4-[N'-(4-benzyloxy-phenyl)-N'-(2-piperidin-1-yl-phenyl)-hydrazinocarbonylmethyl]-5-bromo-2-methoxy-benzoic acid hydrochloride
5) 5-bromo-4-{N'-[4-(4-fluoro-phenoxy)-phenyl]-N'-(2-piperidin-1-yl-phenyl)-hydrazinocarbonylmethyl}-2-methoxy-benzoic acid hydrochloride
6) 5-bromo-2-methoxy-4-{N'-[2-(4-methoxy-phenyl)-ethyl]-N'-2-piperidin-1-yl-phenyl)-hydrazinocarbonylmethyl}-benzoic acid hydrochloride 7) 5-bromo-2-methoxy-4-[N'-(4-methoxy-phenyl)-N'-(2-methyl-6-piperidin-1-yl-phenyl)-hydrazinocarbonyl-methyl]-benzoic acid hydrochloride
7a) Methyl 5-bromo-2-methoxy-4-[N'-(4-methoxy-phenyl)-N'-(2-methyl-6-piperidin-1-yl-phenyl)-hydrazinocarbonyl-methyl]-benzoate
8) 5-bromo-2-methoxy-4-[N'-(4-methoxy-benzyl)-N'-(2-piperidin-1-yl-phenyl)-hydrazinocarbonylmethyl]-benzoic acid hydrochloride
9) 5-bromo-4-[N'-(4-cyclohexyl-phenyl)-N'-(2-piperidin-1-yl-phenyl)-hydrazinocarbonylmethyl]-2-methoxy-benzoic acid hydrochloride
10) 5-bromo-2-methoxy-4-[N'-(2-methyl-6-piperidin-1-yl-phenyl)-N'-(4-trifluoromethoxy-phenyl)-hydrazino-carbonylmethyl]-benzoic acid hydrochloride
11) 5-bromo-2-methoxy-4-[N'-(4'-methoxy-biphenyl-4-yl)-N'-(2-piperidin-1-yl-phenyl)-hydrazinocarbonylmethyl]-benzoic acid hydrochloride
12) 5-bromo-4-[N'-(4-cyclohexyloxy-phenyl)-N'-(2-piperidin-1-yl-phenyl)-hydrazinocarbonylmethyl]-2-methoxy-benzoic acid hydrochloride
13) 5-bromo-2-methoxy-4-[N'-(4-phenoxy-phenyl)-N'-(2-piperidin-1-yl-phenyl)-hydrazinocarbonylmethyl]-benzoic acid hydrochloride
14) 5-bromo-4-{N'-[4-(4-chloro-phenoxy)-phenyl]-N'-(2-piperidin-1-yl-phenyl)-hydrazinocarbonylmethyl}-2-methoxy-benzoic acid hydrochloride
15) 4-{N'-[4-(4-fluoro-phenoxy)-phenyl]-N'-(2-methyl-6-piperidin-1-yl-phenyl)-hydrazinocarbonylmethyl}-2-methoxy-benzoic acid hydrochloride
16) 5-bromo-4-{N'-[4-(4-fluoro-phenoxy)-phenyl]-N'-(2-methyl-6-piperidin-1-yl-phenyl)-hydrazino-carbonyl-methyl}-2-methoxy-benzoic acid hydrochloride
16a) Methyl 5-bromo-4-{N'-[4-(4-fluoro-phenoxy)-phenyl]-N'-(2-methyl-6-piperidin-1-yl-phenyl)-hydrazino-carbonyl-methyl}-2-methoxy-benzoate
17) 4-[N'-(4-benzyl-phenyl)-N'-(2-piperidin-1-yl-phenyl)hydrazinocarbonylmethyl]-5-bromo-2-methoxy-benzoic acid hydrochloride
18) 4-[N'-(4-bromo-phenyl)-N'-(2-piperidin-1-yl-phenyl)hydrazinocarbonylmethyl]-5-chloro-2-methoxy-benzoic acid hydrochloride
19) 4-[N'-(3'-acetyl-biphenyl-4-yl)-N'-(2-piperidin-1-yl-phenyl)-hydrazinocarbonylmethyl]-5-chloro-2-methoxy-benzoic acid hydrochloride
20) 4-[N'-(4'-acetyl-biphenyl-4-yl)-N'-(2-piperidin-1-yl-phenyl-)-hydrazinocarbonylmethyl]-5-chloro-2-methoxy-benzoic acid hydrochloride
21) 5-bromo-2-methoxy-4-[N'-(3-phenoxy)-phenyl]-N'-(2-piperidin-1-yl-phenyl)-hydrazinocarbonylmethyl]-benzoic acid hydrochloride
22) 5-bromo-2-methoxy-4-[N'-(4-phenylsulfanyl)-phenyl]-N'-(2-piperidin-1-yl-phenyl)-hydrazinocarbonylmethyl]-benzoic acid hydrochloride
23) 4-[N'-(4-benzenesulfonyl-phenyl)-N'-(2-piperidin-1-yl-phenyl)-hydrazinocarbonylmethyl]-5-bromo-2-methoxy-benzoic acid hydrochloride
24) 4-[N'-(4-benzenesulfinyl-phenyl)-N'-(2-piperidin-1-yl-phenyl)-hydrazinocarbonylmethyl]-5-bromo-2-methoxy-benzoic acid hydrochloride
25) 2-methoxy-4-{(E)-2-[N'-(4-methoxy-phenyl)-N'-(2-methyl-6-piperidin-1-yl-phenyl)-hydrazinocarbonyl]-vinyl}-benzoic acid hydrochloride
26) 5-bromo-2-methoxy-4-{(E)-2-[N'-(4-methoxy-phenyl)-N'-(2-methyl-6-piperidin-1-yl-phenyl)-hydrazinocarbonyl]-vinyl}-benzoic acid hydrochloride
27) 4-[N'-(4-benzyl-phenyl]-N'-(2-methyl-6-piperidin-1-yl-phenyl)-hydrazinocarbonylmethyl]-5-bromo-2-methoxy-benzoic acid hydrochloride
28) 5-bromo-2-methoxy-4-[N'-(4'-methoxy-biphenyl-4-yl)-N'-(2-methyl-6-piperidin-1-yl-phenyl)-hydrazino-carbonyl-methyl]-benzoic acid hydrochloride
29) 4-[N'-(acetylamino-methyl)-phenyl]-N'-(2-methyl-6-piperidin-1-yl-phenyl)-hydrazinocarbonylmethyl]-5-bromo-2-methoxy-benzoic acid hydrochloride
30) 4-[N'-(4-benzoyl)-phenyl]-N'-(2-piperidin-1-yl-phenyl)-hydrazinocarbonylmethyl]-5-bromo-2-methoxy-benzoic acid hydrochloride
31) 5-bromo-4-[N'-(4-cyano-phenyl)-N'-(2-methyl-6-piperidin-1-yl-phenyl)-hydrazinocarbonylmethyl]-2-methoxy-benzoic acid hydrochloride
32) 4-[N'-(4'-acetyl-biphenyl-4-yl)-N'-(2-methyl-6-piperidin-1-yl-phenyl)-hydrazinocarbonylmethyl]-5-bromo-2-methoxy-benzoic acid hydrochloride
33) 5-bromo-2-methoxy-4-[N'-(4'-methoxy-2-methyl-biphenyl-4-yl)-N'-(2-methyl-6-piperidin-1-yl-phenyl)-hydrazinocarbonylmethyl]-benzoic acid hydrochloride.

8. Intermediates for the synthesis of compounds according to claim 7 selected from the following group:
a) N-(4-methoxy-phenyl)-N-(2-piperidin-1-yl-phenyl)-hydrazine
b) N-(2-piperidin-1-yl-phenyl)-N-(4-trifluoromethoxyphenyl)-hydrazine
c) N-(3-methoxy-benzyl)-N-(2-piperidin-1-yl-phenyl)-hydrazine
d) N-(4-benzyloxy-phenyl)-N-(2-piperidin-1-yl-phenyl)-hydrazine
e) N-[4-(4-fluoro-phenoxy)-phenyl]-N-(2-piperidin-1-yl-phenyl)-hydrazine
f) N-[2-(4-methoxy-phenyl)-ethy1]-N-(2-piperidin-1-yl-phenyl)-hydrazine
g) N-(4-methoxy-phenyl)-N-(2-methy1-6-piperidin-1-yl-phenyl)-hydrazine
h) N-(4-methoxy-benzyl)-N-(2-piperidin-1-yl-phenyl)-hydrazine
i) N-(4-cyclohexyl-phenyl)-N-(2-piperidin-1-yl-phenyl)-hydrazine
j) N-(2-methyl-6-piperidin-1-yl-phenyl)-N-(4-trifluoro-methoxy-phenyl)-hydrazine
k) N-(4'-methoxy-biphenyl-4-yl)-N-(2-piperidin-1-yl-phenyl)-hydrazine
l) N-(4-cyclohexyloxy-phenyl)-N-(2-piperidin-1-yl-phenyl)-hydrazine
m) N-(4-phenoxy-phenyl)-N-(2-piperidin-1-yl-phenyl)-hydrazine
n) N-[4-(4-chloro-phenoxy)-phenyl]-N-(2-piperidin-1-yl-phenyl)-hydrazine
o) N-[4-(4-fluoro-phenoxy)-phenyl]-N-(2-methyl-6-piperidin-1-yl-phenyl)-hydrazine
p) N-(4-benzyl-phenyl]-N-(2-piperidin-1-yl-phenyl)-hydrazine
q) N-(4-bromo-phenyl)-N-(2-piperidin-1-yl-phenyl)-hydrazine
r) N-(3-phenoxy-phenyl)-N-(2-piperidin-1-yl-phenyl)-hydrazine
s) N-(4-phenylsulfanyl-phenyl)-N-(2-piperidin-1-yl-phenyl)-hydrazine
t) N-(4-benzyl-phenyl)-N-(2-methy1-6-piperidin-1-yl-phenyl)-hydrazine
u) N-(4'-methoxy-bipheny1-4-yl)-N-(2-methy1-6-piperidin-1-yl-phenyl)-hydrazine
v) N-(4-benzoyl-phenyl)-N-(2-piperidin-l-yl-phenyl)-hydrazine
w) N-[4'-(2-methy1-[1,3]dithian-2-yl)-biphenyl-4-yl]-N-(2-methy1-6-piperidin-1-yl-phenyl)-hydrazine
x) N-(4'-methoxy-2-methyl-bipheny1-4-yl)-N-(2-methy1-6-piperidin-1-yl-phenyl)hydrazine.

9. A method for preparing compounds of formula (I) according to any of claims 1 to 7, wherein
**G₂** represents the claim 1, 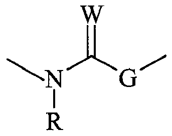 radical, as defined in
**characterized in that** the following steps are carried out:
1) Reaction of the hydrazine function of a compound of formula (II) 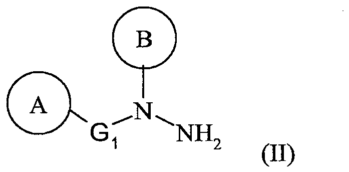 wherein A, B and G₁ are as defined in claim 1,
with the acid function of a compound of formula (III) 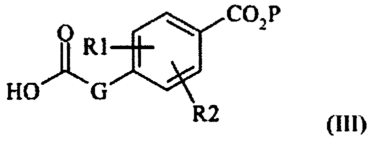 wherein R₁, R₂ and G are as defined earlier, and P represents a group protecting the acid function, in order to obtain the compound of formula (IV) 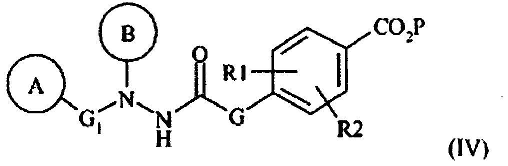
2) Deprotection of the -CO₂P group of the compound of formula (IV) by hydrolysis in order to obtain the compound of formula (I) 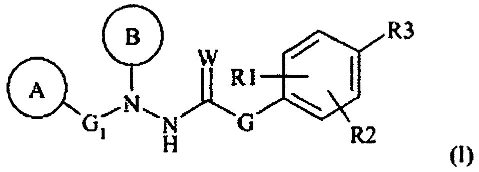 wherein W represents an oxygen atom and R₃ is as defined earlier,
3) If necessary, reaction of the compound of formula (I) obtained in step 2) or else of the compound of formula (IV) obtained in step 1) with a Lawesson reagent, so that a compound of formula (I) may be obtained, wherein W represents a sulphur atom.

10. A method for preparing compounds of formula (I) according to any of claims 1 to 7, wherein
**G₂** represents the 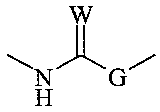 radical, as defined in claim 1,
**characterized in that** the following steps are carried out:
1) Reaction in an acid medium of the compound of formula (V): 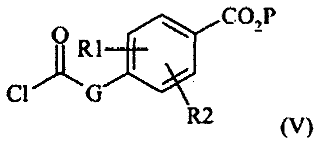 wherein R₁, R₂ and G are as defined in claim 1, and P represents a group protecting the acid function, such as a linear or branched (C₁-C₄) alkyl,
on a compound of formula (XXIV) 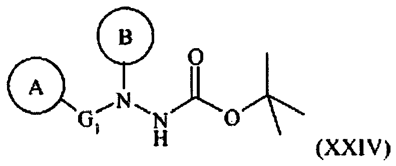 wherein A, B and G₁ are as defined in claim 1, in order to obtain the compound of formula (IV): 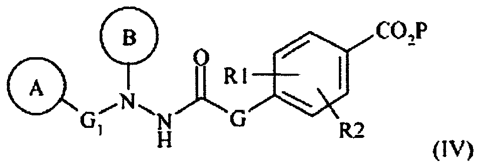
2) Deprotection of the -CO₂P group of the compound of formula (IV) by hydrolysis in order to obtain the compound of formula (I) 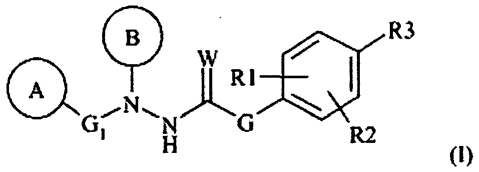 wherein W represents an oxygen atom and R₃ is as defined in Claim 1.

11. A pharmaceutical composition comprising at least one compound of formula (I) according to any of claims 1 to 7, in association with a pharmaceutically acceptable excipient.

12. A compound of formula (I) according to any of claims 1 to 7, for its use as a drug.

13. Use of a compound of formula (I) according to any of claims 1 to 7, for preparing a drug intended for treating or preventing an infection by the papilloma virus.

14. The use according to claim 13, for treating or preventing lesions and diseases associated with infections by the papilloma virus.

15. The use according to claim 13 or 14, for treating or preventing ano-genital warts, such as acuminated condylomas and plane condylomas, laryngeal, conjunctive or buccal papillomas or other epithelial lesions such as recurrent respiratory papillomatoses and low grade and high grade intra-epithelial neoplasias, bowenoid papuloses, warts (verruca vulgaris, verruca plantaris, myrmecia wart, surface wart, verruca plana...), epidermodysplasia verruciformis, carcinomas, in particular ano-genital carcinomas, and all lesions associated with the papilloma virus.
